# EUROPEAN PATENT APPLICATION

(11) **EP 0 677 522 A1**
(43) Date of publication of application: **18.10.1995**
(21) Application number: 94903097.7
(22) Date of filing: 28.12.1993
(51) Int. Cl.: C07D 401/04, A61K 31/47

(54) **8-METHOXYQUINOLONECARBOXYLIC ACID DERIVATIVE**

(30) Priority: 28.12.1992 JP 358515/92; 16.03.1993 JP 82721/93; 30.06.1993 JP 188904/93
(71) Applicant: YOSHITOMI PHARMACEUTICAL INDUSTRIES, LTD., Osaka-shi Osaka 541 (JP)
(72) Inventor: SAITO, Akira, Central Pharm. Res. Inst. of Japan, Takatsuki-sji, Osaka 569 (JP); UESATO, Shin-ichi, Central Pharm. Res. Inst. of, Takatsuki-shi, Osaka 569 (JP); IWATA, Hiromitsu, Central Pharm. Res. Inst. of, Takatsuki-shi, Osaka 569 (JP); AO, Hideki, Technology Development Department, Chikujo-gun, Fukuoka 871 (JP); KURODA, Tsuyoshi, Discovery Research Lab. II, Chikujo-gun, Fukuoka 871 (JP); KAWASAKI, Kazuyuki, Chemical Process Development, Chikujo-gun, Fukuoka 871 (JP); MORIGUCHI, Akihiko. Chemical Process Development, Chikujo-gun, Fukuoka 871 (JP); IKEDA, Yoshifumi, Discovery Research Lab II, Chikujo-gun, Fukuoka 871 (JP)
(74) Representative: von Kreisler, Alek, Dipl.-Chem.
(86) International application number: JP9301925
(87) International publication number: WO9414794

(57) **Abstract**

An 8-methoxyquinolonecarboxylic acid derivative represented by general formula (I), and optical isomers, pharmaceutically acceptable salts and hydrates thereof, wherein R₁ represents hydrogen, lower alkyl, phenylalkyl or an *in vivo* hydrolyzable ester residue; R₂ represents hydrogen or methyl; and n represents an integer of 0 or 1. This derivative has a wide antimicrobial spectrum based on the activity potentiated *in vitro* and *in vivo* against gram-positive bacteria while retaining the potent antibacterial activity of the conventional quinolonecarboxylate bactericides against gram-negative bacteria. Since it scarcely has problematic side effects and is reduced in toxicity, it is promising as a bactericide having more excellent clinical effects.

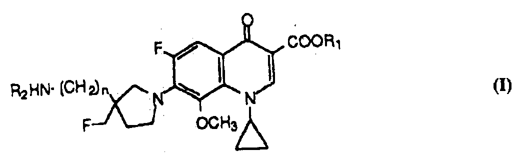

## Description

### Technical Field

The present invention relates to novel quinolonecarboxylic acid derivatives. More particularly, the present invention relates to novel 8-methoxy-quinolonecarboxylic acid derivatives having various properties to sufficiently satisfy various criteria that an antibacterial agent is requested to meet, such as extremely superior antibacterial activity, safety and so on, optical isomers thereof, pharmaceutically acceptable salts thereof and hydrates thereof.

### Background Art

There have been synthesized, developed and marketed quinolonecarboxylic acid derivatives having various chemical structures. These quinolone antibacterial agents generally show a wide range of antibacterial effects against Gram-positive bacteria and Gram-negative bacteria.

A number of compounds having cyclic amino as a substituent at the 7-position of these quinolonecarboxylic acids have been already known. In addition, many attempts have been made to modify the 7-position cyclic amino with various substituents to produce more superior compounds, and, for example, a cyclic amino such as (3-aminomethyl-3-hydroxy-1-piperidinyl) group wherein the same carbon atom constituting the cyclic amino is gem-substituted by two same or different substituents (aminomethyl and hydroxy in the above example) is known.

For example, Japanese Patent Unexamined Publication No. 19583/1987 discloses a compound having a cyclic amino substituent of the formula 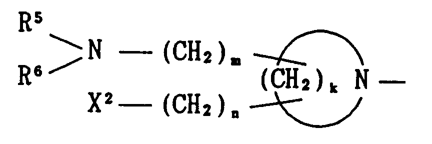
wherein each symbol is as defined in the specification of the above-mentioned Publication, at the 7-position of quinolonecarboxylic acid, and a compound having, at the 7-position, a pyrrolidinyl group gem-substituted by aminomethyl and fluoro at the 3-position, i.e. (3-aminomethyl-3-fluoro-1-pyrrolidinyl) group, is included in Examples therein. However, specific examples of the compounds having a substituent as the 7-position cyclic amino of the present invention are not included. What is more, the substituent at the 8-position is limited to a hydrogen atom or a halogen atom, and there is no disclosure or suggestion as to a lower alkoxy to be introduced as the 8-position substituent.

Japanese Patent Unexamined Publication No. 226883/1989 discloses a compound having a cyclic amino substituent of the formula 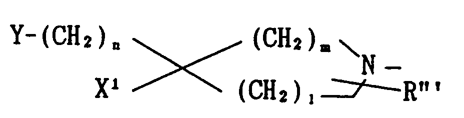
wherein each symbol is as defined in the specification of the above-mentioned Publication, at the 7-position of quinolonecarboxylic acid, and a compound having, for example, (3-hydroxy-3-methylaminomethyl-1-pyrrolidinyl) group at the 7-position is disclosed in Examples therein. Although the substituent name of (3-amino-3-fluoromethyl-1-pyrrolidinyl) group is found in examples, this is merely among the recitation of cyclic amino substituents, and the specifically exemplified compounds have a specific pyrrolidino group at the 7-position, which has hydroxy as a substituent at the 3-position thereof. What is more, the 8-position substituent is hydrogen atom, halogen atom, cyano, nitro and the like, and there is no disclosure or suggestion as to a lower alkoxy to be introduced as the 8-position substituent. Japanese Patent Unexamined Publication No. 198664/1988 discloses a compound having a cyclic amino of the formula 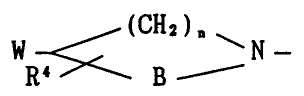
wherein each symbol is as defined in the specification of the above-mentioned Publication, at the 7-position of quinolonecarboxylic acid, and a compound having, for example, (3-aminomethyl-3-hydroxy-1-pyrrolidinyl) group is disclosed in Examples therein. The 7-position cyclic amino substituent is exemplified by (3-amino-3-methyl-1-pyrrolidinyl) group. However, this Publication does not suggest a 7-position cyclic amino substituent wherein amino or aminomethyl directly gem-substitutes the ring-constituting carbon atom, together with other substituents such as fluoromethyl. Journal of Medicinal Chemistry, vol. 35 (25), pp 4745-4750 (1992) discloses 7-(3-aminomethyl-3-methylpyrrolidin-1-yl)-1-cyclopropyl-6-fluoro-1,4- dihydro-8-methoxy-4-oxo-3-quinolinecarboxylic acid and 7-(3-amino-3-methylpyrrolidin-1-yl)-1-cyclopropyl-6-fuoro-1,4-dihydro-8-methoxy-4-oxo-3-quinolinecarboxylic acid. However, this literature does not suggest a quinolonecarboxylic acid wherein the 7-position pyrrolidino group is di-substituted by amino or aminomethyl, and fluoromethyl in a geminal form. Japanese Patent Unexamined Publication No. 124873/1990 discloses a compound having, at the 7-position of quinolonecarboxylic acid, a cyclic amino similar to the one disclosed in the aforementioned Japanese Patent Unexamined Publication No. 198664/1988 and methoxy substituted by fluorine at the 8-position. However, specific examples are 1-cyclopropyl-8-difluoromethoxy-6-fluoro-7-(3-amino-4-methoxymethylpyrrolidin-1-yl)-1,4-dihydro-4-oxoquinoline-3-carboxylic acid monohydrochloride 3/2 hydrate and so on, and the compounds having a 7-position cyclic amino which is gem-substituted are among mere recitation. In addition, there is no description that the 7-position cyclic amino is gem-substituted by fluoromethyl along with another substituent, or a description suggesting such structure. Japanese Patent Unexamined Publication No. 252772/1987 discloses a compound having methoxy at the 8-position and cyclic amino substituent at the 7-position. However, specific examples are 3-amino-4-methylpyrrolidinyl and the like, and this Publication does not suggest di-substitution of a cyclic amino such as pyrrolidino in a geminal form, with amino or aminomethyl along with fluoromethyl.

As described above, there have been heretofore synthesized, developed and used in clinical situations, various quinolone antibacterial agents. However, recent emergence of resistant bacteria showing resistance to these quinolone antibacterial agents has impaired their effectiveness. In particular, infections with methicillin-resistant *Staphylococcus aureus* (MRSA) has presently become a serious problem for clinical treatment. Along with the frequent use of the quinolone antibacterial agents for the treatment of such infections, quinolone resistant bacteria have appeared, thus posing new problems in that most of such bacteria are multiple drug resistant quinolone resistant MRSAs.

In the field of urinary tract infection, moreover, *Staphylococcus epidermidis* and *Enterococcus*, on which oral cephem preparations cannot show effects, have emerged. They have recently become resistant even to quinolone antibacterial agents, thus causing problems as causative bacteria for complicated urinary tract infection.

β-Lactam agents are scarcely effective against MRSA. Although the quinolone antibacterial agents recently developed can show effects against certain MRSA, the effects are not satisfactory. Quinolone antibacterial agents hardly show effects against quinolone resistant MRSA, *Staphylococcus epidermidis* and *Enterococcus*.

At present, Vancomycin and Arbekacin are clinically used as injections against such multiple drug resistant MRSA, and there is no oral preparation which can take the place of these drugs.

Accordingly, the creation of a highly safe pharmaceutical preparation showing, while retaining the antibacterial effect against Gram-negative bacteria that the conventional quinolone antibacterial agents have, a strong antibacterial effect against Gram-positive bacteria such as MRSA, quinolone resistant MRSA, *Staphylococcus epidermidis* and *Enterococcus*, has been a strong demand.

### Disclosure of the Invention

With the aim of solving the above-mentioned problems, the present inventors have substituted a cyclic amino at the 7-position of quinolonecarboxylic acid with various substituents, and found that a compound having a cyclic amino of the structure, wherein the same carbon atom on the cyclic amino is gem-substituted, shows a strong antibacterial effect, that the compound particularly shows a strong antibacterial effect when the cyclic amino is pyrrolidino, and that the compound shows particularly strong and a wide range of antibacterial effects, as well as superior safety, when the pyrrolidino is gem-substituted by fluoromethyl and amino, aminomethyl, methylamino or methylaminomethyl. Moreover, the present inventors have found that substitution of the 8-position of quinolonecarboxylic acid with methoxy markedly reduces therapeutically unfavorable effects such as phototoxicity, and increases stability of the compound structure. On the other hand, the present inventors have newly synthesized compounds having methoxy at the 8-position of quinolonecarboxylic acid and 3-amino-3-hydroxymethylpyrrolidino, 3-aminomethyl-3-hydroxymethylpyrrolidino or 3-aminomethyl-3-aminopyrrolidino at the 7-position, and confirmed that these compounds did not show the expected antibacterial effect. The present inventors have found that a specific and novel quinolonecarboxylic acid derivative wherein the same carbon atom of a pyrrolidino group constituting the 7-position substituent of quinolonecarboxyic acid is gem-substituted by two specific, different substituents (one being fluoromethyl and the other being amino, aminomethyl, methylamino or methylaminomethyl) and the 8-position is substituted by methoxy, and optical isomers thereof retain strong antibacterial effects against Gram-negative bacteria, show stronger antibacterial effects against Gram-positive bacteria, particularly strong antibacterial effects against quinolone resistant MRSA, *Staphylococcus epidermidis* and *Enterococcus*, than do the conventional quinolone antibacterial agents, and markedly reduce side-effects such as phototoxicity, which resulted in the completion of the invention.

That is, the present invention is detailedly as follows.
(1) 8-Methoxy-quinolonecarboxylic acid derivatives of the formula 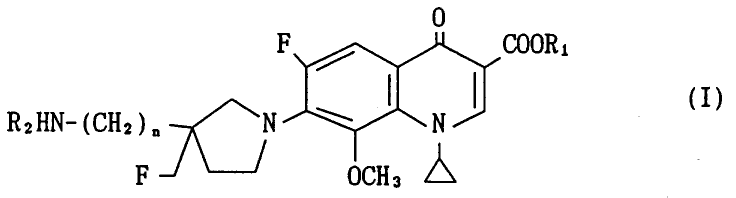 wherein R₁ is a hydrogen atom, a lower alkyl, a phenylalkyl or an ester residue hydrolyzable in the living body, R₂ is a hydrogen atom or methyl and n is an integer of 0 or 1, optical isomers thereof, pharmaceutically acceptable salts thereof and hydrates thereof [hereinafter also referred to as Compound (I)].
(2) 8-Methoxy-quinolonecarboxylic acid derivatives of the above-mentioned (1) wherein R₁ is a hydrogen atom and n is 0, optical isomers thereof, pharmaceutically acceptable salts thereof and hydrates thereof.
(3) 8-Methoxy-quinolonecarboxylic acid derivatives of the above-mentioned (1) wherein R₁ is a hydrogen atom and n is 1, optical isomers thereof, pharmaceutically acceptable salts thereof and hydrates thereof.
(4) 8-Methoxy-quinolonecarboxylic acid derivatives of the above-mentioned (1) which are selected from 7-(3-amino-3-fluoromethylpyrrolidin-1-yl)-1-cyclopropyl-6-fluoro-1,4-dihydro-8-methoxy-4-oxo-3-quinolinecarboxylic acid, 7-(3-aminomethyl-3-fluoromethylpyrrolidin-1-yl)-1-cyclopropyl-6-fluoro-1,4-dihydro-8-methoxy-4-oxo-3-quinolinecarboxylic acid, 1-cyclopropyl-6-fluoro-7-(3-fluoromethyl-3-methylaminopyrrolidin-1-yl)-1,4-dihydro-8-methoxy-4-oxo-3-quinolinecarboxylic acid and 1-cyclopropyl-6-fluoro-7-(3-fluoromethyl-3-methylaminomethylpyrrolidin-1-yl)-1,4-dihydro-8-methoxy-4-oxo-3-quinolinecarboxylic acid, optical isomers thereof, pharmaceutically acceptable salts thereof and hydrates thereof.
(5) 8-Methoxy-quinolonecarboxylic acid derivatives of the above-mentioned (1), (2) or (4), which are selected from 7-(3-amino-3-fluoromethylpyrrolidin-1-yl)-1-cyclopropyl-6-fluoro-1,4-dihydro-8- methoxy-4-oxo-3-quinolinecarboxylic acid, (R)-7-(3-amino-3-fluoromethylpyrrolidin-1-yl)-1-cyclopropyl-6-fluoro-1,4-dihydro-8-methoxy-4-oxo-3-quinolinecarboxylic acid and (S)-7-(3-amino-3-fluoromethylpyrrolidin-1-yl)-1-cyclopropyl-6-fluoro-1,4-dihydro-8-methoxy-4-oxo-3-quinolinecarboxylic acid, pharmaceutically acceptable salts thereof and hydrates thereof.
(6) (S)-7-(3-Amino-3-fluoromethylpyrrolidin-1-yl)-1-cyclopropyl-6-fluoro-1,4-dihydro-8-methoxy-4-oxo-3-quinolinecarboxylic acid and pharmaceutically acceptable salts thereof.
(7) 8-Methoxy-quinolonecarboxylic acid derivatives of the above-mentioned (1), (3) or (4), which are selected from 7-(3-aminomethyl-3-fluoromethylpyrrolidin-1-yl)-1-cyclopropyl-6-fluoro-1,4-dihydro-8-methoxy-4-oxo-3-quinolinecarboxylic acid, (R)-7-(3-aminomethyl-3-fluoromethylpyrrolidin-1-yl)-1-cyclopropyl-6-fluoro-1,4-dihydro-8-methoxy-4-oxo-3-quinolinecarboxylic acid and (S)-7-(3-aminomethyl-3-fluoromethylpyrrolidin-1-yl)-1-cyclopropyl-6-fluoro-1,4-dihydro-8-methoxy-4-oxo-3-quinolinecarboxylic acid, optical isomers thereof, pharmaceutically acceptable salts thereof and hydrates thereof.
(8) (S)-7-(3-Aminomethyl-3-fluoromethylpyrrolidin-1-yl)-1-cyclopropyl-6-fluoro-1,4-dihydro-8-methoxy-4-oxo-3-quinolinecarboxylic acid and pharmaceutically acceptable salts thereof.
(9) Pharmaceutical compositions comprising a 8-methoxy-quinolonecarboxylic acid derivative of the above-mentioned (1) to (8), an optical isomer thereof, a pharmaceutically acceptable salt thereof or a hydrate thereof, and a pharmaceutically acceptable carrier.

In the present invention, the terms used for various definitions mean the following.

Lower alkyl means straight or branched alkyl having 1 to 6, preferably 1 to 4, carbon atoms, and is exemplified by methyl, ethyl, propyl, isopropyl, butyl, sec-butyl, tert-butyl, pentyl, isopentyl, neopentyl and hexyl.

Preferred lower alkyl for R₁ is an alkyl having 1 to 4 carbon atoms such as methyl and ethyl.

Phenylalkyl means the above-mentioned lower alkyl substituted by a phenyl. The phenyl may be substituted by a substituent such as methyl, ethyl, methoxy, ethoxy, and the like. Examples of phenylalkyl include benzyl, phenylethyl and p-methylbenzyl, with preference given to benzyl.

Ester residue hydrolyzable in the living body is exemplified by alkanoyloxyalkyl ester such as acetoxymethyl, pivaloyloxymethyl, 1-acetoxyethyl and 1-pivaloyloxyethyl; alkoxycarbonyloxyalkyl ester such as ethoxycarbonyloxymethyl and 1-ethoxycarbonyloxyethyl; phthalidyl ester such as phthalidyl and dimethoxyphthalidyl; carbamoylalkyl ester such as carbamoylmethyl, carbamoylethyl, N-methylcarbamoylmethyl, N,N-dimethylcarbamoylmethyl and N,N-diethylcarbamoylmethyl; alkoxyalkyl ester such as methoxymethyl and methoxyethyl; aminoalkyl ester such as aminomethyl, aminoethyl and aminopropyl; alkylaminoalkyl ester such as methylaminomethyl, methylaminoethyl, methylaminopropyl, ethylaminomethyl, ethylaminoethyl, ethylaminopropyl, dimethylaminomethyl, dimethylaminoethyl, dimethylaminopropyl, diethylaminomethyl, diethylaminoethyl and diethylaminopropyl; morpholinoalkyl ester such as morpholinoethyl; piperidinoalkyl ester such as piperidinoethyl; alkylphenylamino ester such as methylphenylamino; cycloalkyloxycarbonylalkyl ester such as 1-cyclohexyloxycarbonylethyl; and 5-methyl-1,3-dioxolen-2-one-4-ylmethyl ester.

The salts of the compound (I) are exemplified by acid addition salts, metal salts and heavy metal salts. Examples of the acid addition salt include salts with inorganic acid such as hydrochloric acid, hydrobromic acid, sulfuric acid, phosphoric acid and nitric acid; salts with organic acid such as p-toluenesulfonic acid, propionic acid, succinic acid, glycolic acid, malic acid, ascorbic acid, methanesulfonic acid, citric acid, lactic acid, maleic acid, fumaric acid, acetic acid and tartaric acid. Examples of the metal salts include alkali metal salts, alkaline earth metal salts such as sodium, potassium, calcium, magnesium and aluminum salts, heavy metal salts such as copper, zinc, iron, gold, silver, platinum and manganese salts. Further, the salts are exemplified by salts with amino acid such as lysine and ornithine.

The Compounds (I) of the present invention have asymmetric carbon, and the present invention encompasses optical isomers and enantiomers derived therefrom, and racemates thereof. In addition, hydrates are also encompassed.

The Compound (I) of the present invention can be produced, for example, by the methods shown in the following.

### Production of Compound (I)

### Synthesis 1

A quinolonecarboxylic acid of the formula (II) 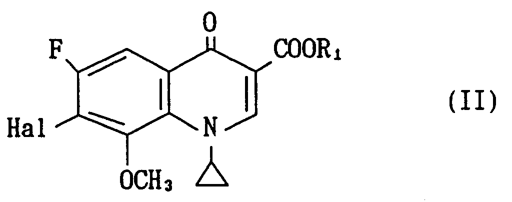
wherein R₁ is as defined above and Hal is a halogen atom, with particular preference given to fluorine atom, which is synthesized according to the method described in, for example, Japanese Patent Unexamined Publication No. 252772/1987, is condensed with a pyrrolidine compound of the formula (III) 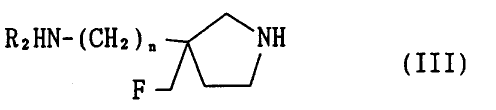
wherein each symbol is as defined above.

This condensation *per se* is well known and the compound (III) is used in 1 to 4-fold molar amount relative to the quinolonecarboxylic acid compound (II). The reaction proceeds without solvent or in a suitable solvent at 0-200°C, preferably 30-150°C, particularly preferably 30-100°C for 1 to 48 hours. Examples of the suitable solvent include water, alcohols such as methanol, ethanol, propanol and isopropanol, acetonitrile, pyridine, dimethylformamide, dimethyl sulfoxide, hexamethylphosphoric triamide and 1-methyl-2-pyrrolidone. In this case, an organic base such as 1,8-diazabicyclo[5.4.0]undeca-7-ene and triethylamine, or an inorganic base such as potassium carbonate, sodium carbonate, potassium hydrogencarbonate and sodium hydrogencarbonate may be used as a dehydrohalogenating agent.

### Synthesis 2

A chelate compound of the formula (IV) 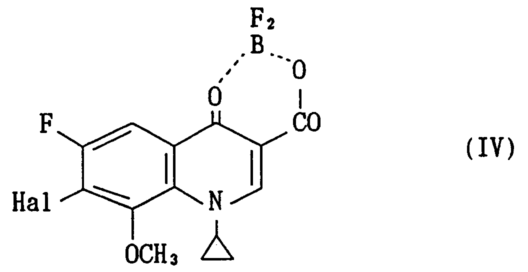
wherein each symbol is as defined above, which is obtained by reacting the above-mentioned compound (II) with boron trifluoride, boron trifluoride complex or fluoroboric acid according to the method described in Japanese Patent Unexamined Publication No. 67290/1984, is condensed with a pyrrolidine compound of the formula (III) to give a compound of the formula 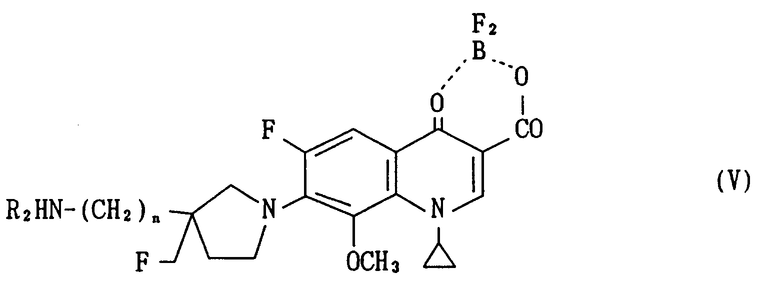
wherein each symbol is as defined above, which is further treated with a base. The carboxylic acid may be converted to a salt or an ester where necessary.

More specifically, the method described in Japanese Patent Unexamined Publication No. 67290/1984 for producing a compound (IV) from the compound (II) is carried out using an equimolar amount or more of boron trifluoride, boron trifluoride complex or fluoroboric acid relative to the compound (II), without solvent or in a suitable solvent at room temperature to 150°C, preferably 30-100°C, particularly preferably 50-100°C, for 1 to 48 hours.

The condensation of the compound (III) with the compound (IV) is carried out using 1 to 4-fold molar amount of the compound (III) relative to the compound (IV), without solvent or in a suitable solvent at 0-150°C, preferably 30-100°C for 1 to 48 hours. Examples of the suitable solvent include water, alcohols such as methanol, ethanol, propanol and isopropanol, acetonitrile, pyridine, dimethylformamide, dimethyl sulfoxide, hexamethylphosphoric triamide and 1-methyl-2-piperidone. In this case, an organic base such as 1,8-diazabicyclo[5.4.0]undeca-7-ene and triethylamine, or an inorganic base such as potassium carbonate, sodium carbonate, potassium hydrogencarbonate and sodium hydrogencarbonate may be used as a dehydrohalogenating agent.

Examples of the base to be reacted with the compound (V) include an organic base such as 1,8-diazabicyclo[5.4.0]undeca-7-ene and triethylamine, and an inorganic base such as potassium carbonate, sodium carbonate, potassium hydrogencarbonate, sodium hydrogencarbonate, sodium hydoxide and potassium hydoxide. Examples of the suitable solvent include water, alcohols such as methanol, ethanol, propanol and isopropanol, halogenated hydrocarbon such as dichloromethane, methylene chloride and chloroform, and mixtures thereof. The reaction is carried out at room temperature to the refluxing temperature of the solvent used, for 1 to 24 hours.

It is also possible to use a boron compound of the formula 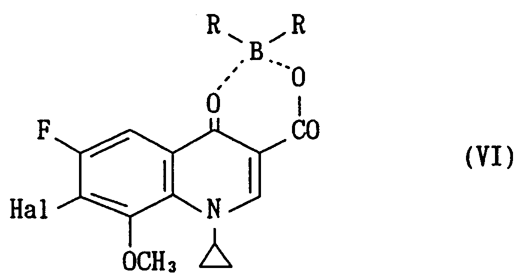
wherein Hal is as defined above and R is aliphatic acyloxy having 2 to 6 carbon atoms, aliphatic acyloxy having 2 to 6 carbon atoms optionally substituted by halogen atom or aromatic acyloxy having 7 to 11 carbon atoms, which is obtained according to the method described in Japanese Patent Unexamined Publication No. 69388/1992, in place of the chelate compound (IV) to be used in the present synthesis.

### Synthesis 3

A quinolonecarboxylic acid compound (VII) of the formula 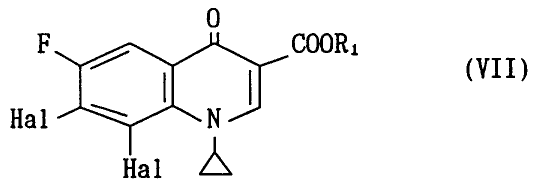
wherein each symbol is as defined above, which is synthesized according to the method described in, for example, Japanese Patent Unexamined Publication No. 212474/1984, or a chelate compound of the formula 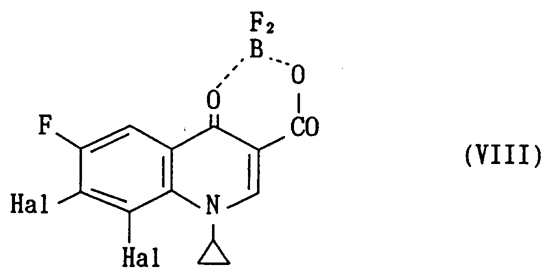
wherein each symbol is as defined above, which is obtained from the compound (VII) by the above-mentioned method described in Japanese Patent Unexamined Publication No. 67290/1984, is reacted with a pyrrolidine compound of the formula (III) according to the method of Synthesis 1 or Synthesis 2 to give a quinolone compound (IX) of the formula 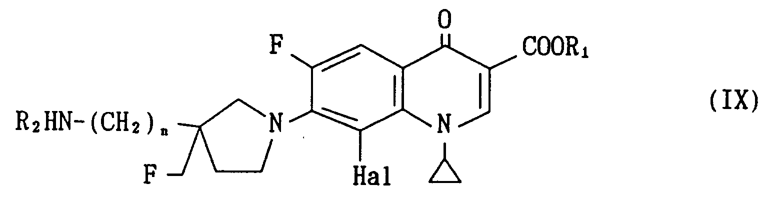
wherein each symbol is as defined above, which is further reacted with a metal alkoxide such as sodium methoxide and potassium methoxide, whereby a compound (I) can be synthesized.

This reaction is carried out using 1 to 8-fold molar amount of the metal alkoxide such as sodium methoxide and potassium methoxide relative to the compound (IX), in a suitable solvent at 0-200°C, preferably 30-150°C, particularly preferably 30-100°C, for 1 to 48 hours. Examples of the suitable solvent include pyridine, dimethylformamide, dimethylacetamide, dimethyl sulfoxide, hexamethylphosphoric triamide and 1-methyl-2-piperidone.

### Synthesis 4

A compound of the formula 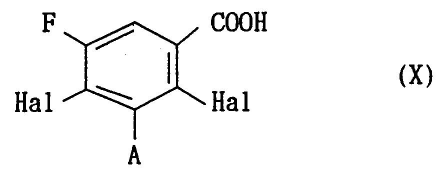
wherein Hal is as defined above and A is a halogen atom such as chlorine, fluorine and bromine or methoxy, is reacted with a cyclic amine compound (III) according to the method of Synthesis 1 to give a compound of the formula 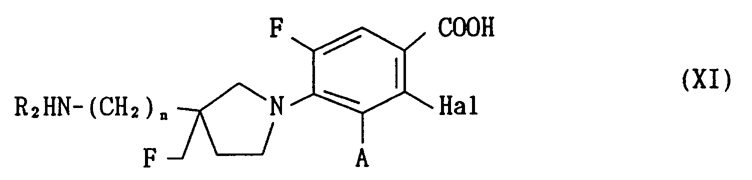
wherein each symbol is as defined above, and the obtained compound of the formula (XI) or a reactive derivative thereof such as acid halide, thioester, acid anhydride, mixed acid anhydride, ester, acid azide and acid amide is then reacted with an aminoacrylic acid derivative of the formula

R₃R₄NCH=CHCOOR₁ (XII)

wherein R₁ is as defined above and R₃ and R₄ are the same or different and each is a lower alkyl such as methyl and ethyl. The obtained compound is reacted with a compound of the formula 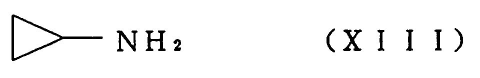
to give a compound of the formula 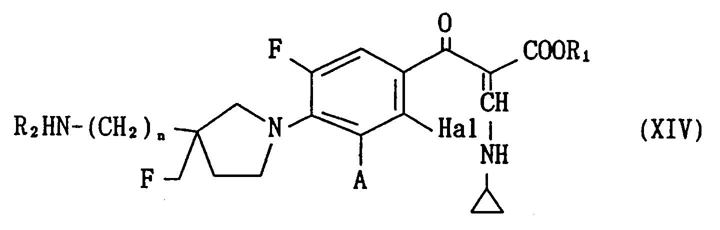
wherein each symbol is as defined above, which is then cyclized to give a compound of the formula 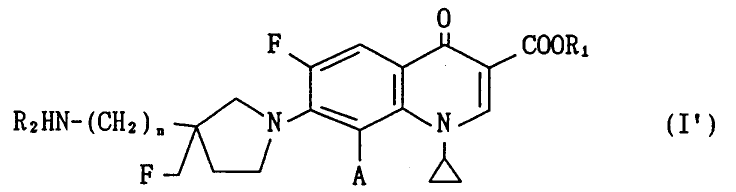
wherein each symbol is as defined above.

The reaction for obtaining a compound (XIV) from the compound (XI) is carried out using 0.8 to 3-fold molar amount of the compound (XII) and 1 to 3-fold molar amount of the compound (XIII) relative to the compound (XI) without solvent or in a suitable solvent at 0-50°C for the reaction with the compound (XII) and at 30-200°C, preferably 50-120°C, for the reaction with the compound (XIII), for 1 to 24 hours. Examples of the suitable solvent include toluene, xylene, cyclohexane, dimethylformamide and dimethyl sulfoxide. In this case, an organic base such as triethylamine, dimethylaniline and 1,8-diazabicyclo[5.4.0]undeca-7-ene or an inorganic base such as potassium carbonate, sodium carbonate, potassium hydrogencarbonate and sodium hydrogencarbonate may be used as a dehydrohalogenating agent.

The cyclization reaction is carried out by heating at 0-200°C, preferably 50-150°C, for 1 to several hours in an appropriate solvent such as polar solvents (e.g. dioxane, alcohol such as methanol, ethanol, propanol and butanol, dimethylformamide, dimethyl sulfoxide and sulfolane) in the presence of an appropriate basic catalyst such as potassium carbonate, sodium carbonate, 1,8-diazabicyclo[5.4.0]undeca-7-ene and potassium fluoride.

Of the compounds (I') thus obtained, a compound wherein A is a halogen atom can be converted to a compound wherein A is methoxy (the object compound of the present invention) by reacting the compound (I') with a metal alkoxide as in the aforementioned Synthesis 3.

### Synthesis 5

A compound of the formula 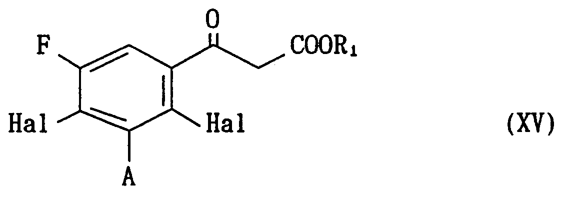
wherein each symbol is as defined above, is reacted with a cyclic amine compound (III) according to the method of Synthesis 1 to give a compound of the formula 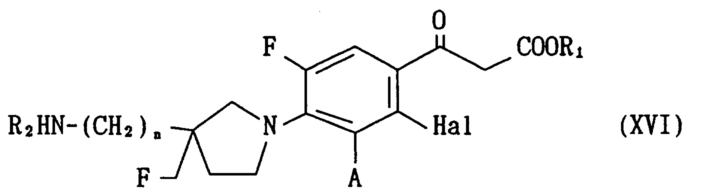
wherein each symbol is as defined above, which is then reacted with orthoformate-acetic anhydride or dimethylformamide dialkyl acetal (e.g. dimethylformamide dimethyl acetal and dimethylformamide diethyl acetal) and with the compound (XIII) in the same manner as in Synthesis 4 to give a compound (XIV). This compound is cyclized in the same manner as in Synthesis 4, whereby a compound (I') is obtained.

The reaction of the compound (XVI) and orthoformate or dimethylformamide dialkyl acetal is carried out using 1 to 3-fold molar amount of orthoformate or dimethylformamide dialkyl acetal relative to the compound (XVI), without solvent or in a suitable solvent at 30-200°C, preferably 50-150°C, for 1 to 24 hours. Examples of the suitable solvent include acetic anhydride, toluene, xylene, cyclohexane, dimethylformamide and dimethyl sulfoxide.

Of the compounds thus obtained, a compound wherein A is a halogen atom can be converted to the object compound wherein A is methoxy by reacting the compound with a metal alkoxide as in the aforementioned Synthesis 3.

### Synthesis 6

When an optically active compound of the formula 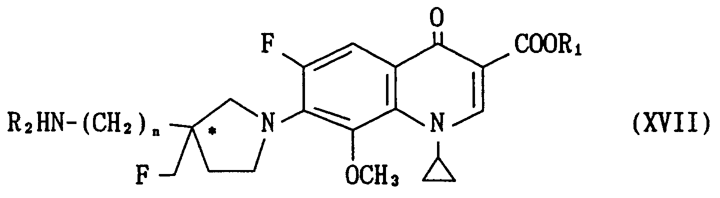
wherein each symbol is as defined above, is desired, a racemate of the compound of the formula (I) wherein R₁ is a lower alkyl or a phenylalkyl is optically resolved by a conventional method.

For optical resolution, an ester compound of the formula (I) wherein R₁ is a lower alkyl or a phenylalkyl, having asymmetric carbon atom, is converted to a salt with an optical active acid such as (R)-(-)-10-camphorsulfonic acid, (S)-(+)-10-camphorsulfonic acid, (R)-(-)-mandelic acid, (S)-(+)-mandelic acid, dibenzoyl-L-tartaric acid, dibenzoyl-D-tartaric acid, D-malic acid, L-malic acid, N-acetyl-L-glutamic acid, N-acetyl-D-glutamic acid, N-acetyl-L-tryptophan, N-acetyl-D-tryptophan, (R)-O-methylmandelic acid, (S)-O-methylmandelic acid, N-ortho-nitrobensenesulfonyl-L-proline, N-ortho-nitrobenzenesulfonyl-D-proline, L-tartaric acid and D-tartaric acid, preferably with (R)-O-methylmandelic acid or (S)-O-methylmandelic acid, and the obtained salt is separated by fractional crystallization, followed by elimination of ester residue, whereby respective optical isomers are obtained.

Alternatively, an optical isomer can be obtained from a synthetic intermediate of the formula (III) which is optically resolved by a conventional method or synthesized by asymmetric synthesis, by following the above-mentioned Syntheses 1 to 5. For example, a synthetic intermediate of the formula (III) is treated with an optically active organic acid compound to form a diastereomer salt as mentioned above. The obtained diastereomer salt is subjected to fractional recrystallization and treatment with an alkali for desalting.

When the amino group of the Compounds (I) obtained in the above-mentioned Syntheses 1-6 has been protected by an amino-protecting group, wherein amino-protecting group is a protecting group conventionally used for organic synthesis and is exemplified by benzyl, substituted benzyl (e.g. 2-, 3-, or 4-chlorobenzyl, 2-, 3-, or 4-methylbenzyl, 2-, 3-, or 4-methoxybenzyl, 2-, 3-, or 4-nitrobenzyl, and 3-, 4-dimethoxybenzyl), α-methylbenzyl, (S)-α-methylbenzyl, (R)-α-methylbenzyl, diphenylmethyl, bis(4-methoxyphenyl)methyl, triphenylmethyl, phenacyl, acetyl, trifluoroacetyl, methoxycarbonyl, ethoxycarbonyl, benzyloxycarbonyl and tert-butoxycarbonyl, the protecting group can be removed by hydrogenation in an alcohol such as methanol, ethanol and propanol, water or a mixed solvent thereof in the presence of a catalyst (e.g. palladium-carbon), or by refluxing in the presence of hydrazine monohydrate and a catalyst (e.g. palladium-carbon), or by hydrolysis with an acid or alkali.

The Compound (I) of the present invention wherein R₁ is a lower alkyl, a phenylalkyl, or an ester residue which is hydrolyzable in the living body, is obtained by reacting a compound wherein R₁ is a hydrogen, with a compound of the formula

R₁'-OH

wherein R₁' is a group other than hydrogen atom for R₁, according to an esterification widely used in organic chemistry. In addition, an ester exchange reaction may be applied.

The substituent for R₁ can be easily removed by hydrolysis with a conventional acid or alkali, hydrogenation in an alcohol such as methanol, ethanol and propanol, water or a mixed solvent thereof in the presence of palladium-carbon, or by refluxing using hydrazine monohydrate in the presence of palladium-carbon.

The production of the compounds of the formula (III) which are the starting materials used in the present invention is described in the following. These compounds can be produced, for example, by the following production methods.

### Production of intermediate (III)

### Production 1 : Compounds of the formula (III) wherein n is 0

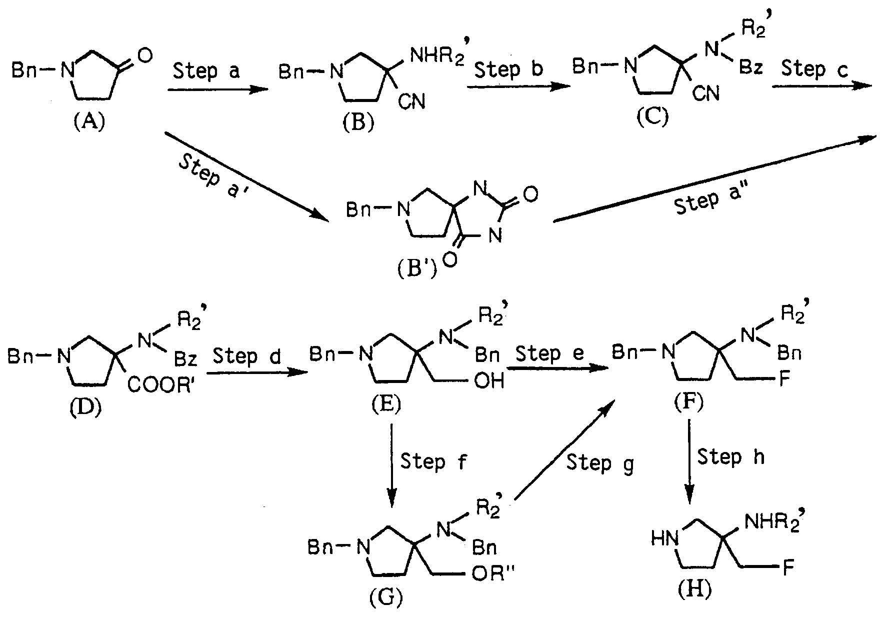

In the above reaction steps, Bn is benzyl or substituted benzyl as an amino-protecting group, Bz is benzoyl or substituted benzoyl such as p-methoxybenzoyl, R₂' is a hydrogen atom, a lower alkyl, benzyl or substituted benzyl, R' is a lower alkyl and R'' is methanesulfonyl, trifluoromethanesulfonyl or p-toluenesulfonyl.

### Step a

In Step a, a compound (A) is reacted with an alkali metal cyanate such as sodium cyanate and potassium cyanate, or alkaline earth metal cyanate such as magnesium cyanate and calcium cyanate, in an alcohol such as methanol, ethanol, propanol and isopropanol, a mixed solvent of such alcohol and water or water, in the presence of ammonium chloride, alkylamine hydrochloride, benzylamine hydrochloride, aqueous ammonia or a mixture thereof at room temperature or under heating, preferably at 10-80°C, whereby a compound (B) is obtained.

### Step b

In Step b, a compound (B) is reacted with benzoyl chloride or a substituted benzoyl halide such as p-methoxybenzoyl chloride, without solvent or in a solvent such as hydrocarbon halide (e.g. chloroform and dichloroethane), toluene, dimethylformamide, dimethylacetamide, tetrahydrofuran and diethyl ether, or a mixed solvent thereof with water, in the presence of an inorganic base such as sodium hydroxide, potassium hydroxide, magnesium hydroxide, calcium hydroxide, potassium carbonate, potassium hydrogencarbonate, sodium carbonate and sodium hydrogencarbonate or an organic base such as triethylamine, dicyclohexylamine, N-methylmorpholine and pyridine, under cooling to under heating, whereby a compound (C) is obtained.

### Step c

In Step c, the compound (C) is treated as in the following i)-iii) to give a compound (D).
i) The compound (D) is obtained by refluxing the compound (C) under heating in an alcohol of the formula R'OH such as methanol, ethanol, propanol and isopropanol, in the presence of hydrochloric acid, sulfuric acid or p-toluenesulfonic acid, and extracting with an organic solvent such as chloroform and toluene.
ii) According to Step a', the compound (A) is reacted with an alkali metal cyanate such as sodium cyanate and potassium cyanate, or alkaline earth metal cyanate such as magnesium cyanate and calcium cyanate, in an alcohol such as methanol, ethanol, propanol and isopropanol, a mixed solvent of such alcohol and water, in the presence of ammonium carbonate under heating, preferably at 50-200°C, whereby an intermediate hydantoin compound (B') is obtained.
   According to Step a'', the compound (B') is refluxed under heating in an aqueous solution of alkali such as sodium hydroxide, potassium hydroxide, magnesium hydroxide and calcium hydroxide, added with an acid such as hydrochloric acid, sulfuric acid and acetic acid under ice-cooling to under heating, preferably under ice-cooling. The mixture is concentrated to dryness under reduced pressure, and an alcohol of the formula R'OH such as methanol, ethanol, propanol and isopropanol is added to the residue. Oxalyl chloride or thionyl chloride is added and the mixture is reacted preferably at room temperature. The mixture is reacted with benzoyl halide such as benzoyl chloride in the presence of an alkali such as potassium carbonate and sodium carbonate to give a compound (D).
iii) The compound (D) can be obtained by adding conc. hydrochloric acid to a compound (C) in an alcohol such as methanol, ethanol, propanol and isopropanol, leaving the mixture at not less than 0°C, preferably at room temperature and neutralizing the mixture to give an acid amide intermediate, which is then added with thionyl chloride or sulfuric acid in an alcohol of the formula R'OH such as methanol, ethanol, propanol and isopropanol.

### Step d

In Step d, the compound (D) is reduced with lithium aluminum hydride, sodium bis(2-methoxy)aluminum hydride, diborane or sodium borohydride in a solvent such as tetrahydrofuran, diethyl ether, diisopropyl ether and toluene to give a compound (E).

### Step e

In Step e, the compound (E) obtained in Step d is reacted with a fluorinating agent, such as diethylaminosulfur trifluoride and hexafluoropropene diethylamine, in a solvent such as tetrahydrofuran, diethyl ether, diisopropyl ether, toluene, dioxane, benzene, dimethylformamide, dimethylacetamide, dimethyl sulfoxide, hexane, chloroform, dichloromethane and dichloroethane to give a compound (F).

### Step f

In Step f, the compound (E) obtained in Step d is reacted with p-toluenesulfonyl chloride, methanesulfonyl chloride or trifluoromethanesulfonic anhydride in a solvent such as tetrahydrofuran, diethyl ether, diisopropyl ether, toluene, dioxane, benzene, dimethylformamide, dimethylacetamide, dimethyl sulfoxide, hexane, dichloromethane and dichloroethane to give a compound (G).

### Step g

In Step g, the compound (G) is reacted with sodium fluoride, potassium fluoride, magnesium fluoride, calcium fluoride or tetrabutylammonium fluoride, in a solvent such as alcohol (e.g. methanol, ethanol and isopropanol), tetrahydrofuran, diethyl ether, diisopropyl ether, toluene, acetone, acetonitrile, dioxane, benzene, dimethylacetamide, dimethylformamide, dimethyl sulfoxide, hexane, dichloromethane, dichloroethane, water, or a mixed solvent thereof to give a compound (F).

### Step h

In Step h, the compound (F) is subjected to hydrogenolysis in a solvent such as alcohol (e.g. methanol, ethanol, propanol and isopropanol) and acetic acid, in the presence of a catalyst such as palladium-carbon, palladium hydroxide-carbon and palladium black, under heating under pressurization where necessary; or refluxed under heating with hydrazine hydrate, ammonium formate or the like, in a solvent such as alcohol (e.g. methanol, ethanol, propanol and isopropanol) and acetic acid in the presence of a catalyst such as palladium-carbon, palladium hydroxide-carbon and palladium black for deprotection to give a compound (H).

### Production 2 : Compounds of the formula (III) wherein n is 0

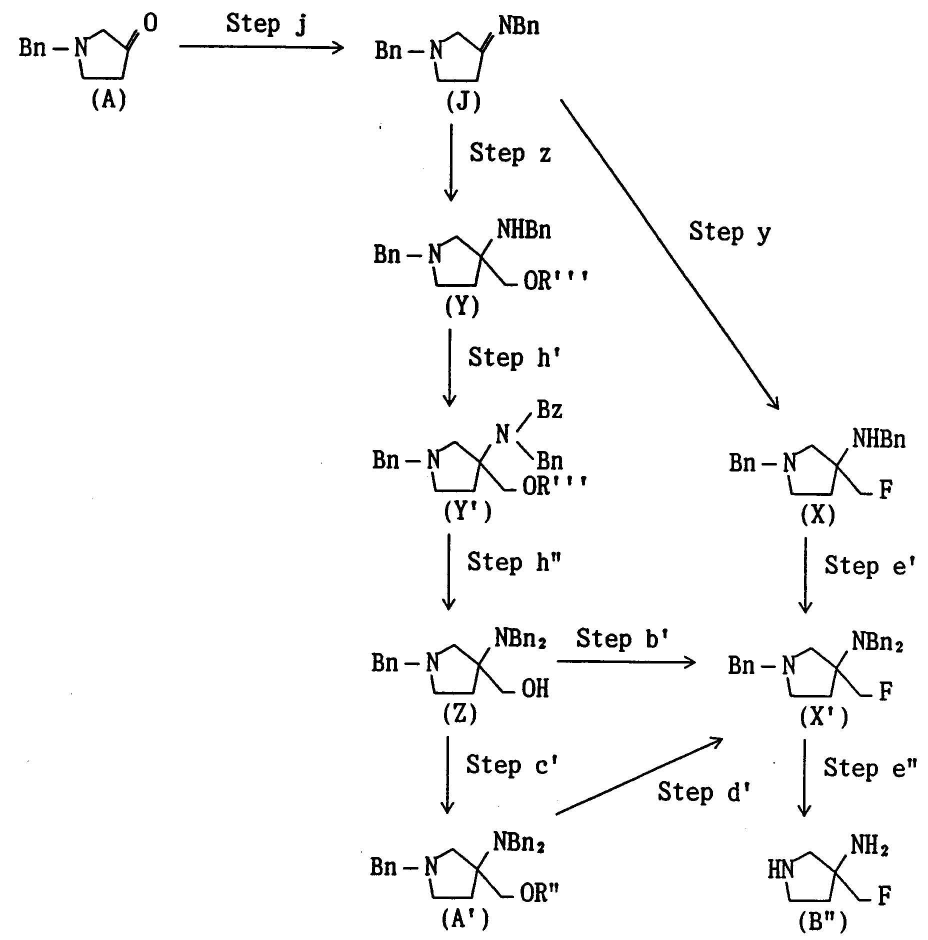

In the above reaction steps, Bn, Bz, R' and R'' are as defined above, and R''' is an alcohol-protecting group.

### Step j

In Step j, the compound (A) is reacted with benzylamine or substituted benzylamine in a solvent such as dry benzene and dry toluene to give a compound (J).

### Step y

In Step y, the compound (J) is reacted with a fluoromethyl metal compound such as fluoromethylmagnesium bromide in a solvent such as tetrahydrofuran, diethyl ether, diisopropyl ether, toluene, dioxane, benzene and hexane to give a compound (X).

### Step e'

In Step e', the compound (X) is reacted with benzyl chloride or a substituted benzyl halide such as p-methoxybenzyl chloride, without solvent or in a solvent such as halogenated hydrocarbon (e.g. chloroform and dichloroethane), toluene, dimethylformamide, dimethylacetamide, tetrahydrofuran, diethyl ether or a mixed solvent thereof with water, in the presence of an inorganic base such as sodium hydroxide, potassium hydroxide, magnesium hydroxide, calcium hydroxide, potassium carbonate, potassium hydrogencarbonate, sodium carbonate and sodium hydrogencarbonate or an organic base such as triethylamine, dicyclohexylamine, N-methylmorpholine and pyridine under cooling to under heating, whereby a compound (X') is obtained.

### Step z

In Step z, the compound (J) is reacted with a protected hydroxyalkylmetallic compound such as tetrahydropyranyloxymethylmagnesium bromide and tert-butyldimethylsilyloxymethylmagnesium bromide, in a solvent such as tetrahydrofuran, diethyl ether, diisopropyl ether, toluene, dioxane, benzene and hexane to give a compound (Y).

### Step h'

In Step h', the compound (Y) is reacted in the same manner as in Production 1, Step b, to give a compound (Y').

### Step h''

In Step h'', the compound (Y') is reacted with hydrochloric acid, trifluoroacetic acid, potassium fluoride or tetrabutylammonium fluoride in a solvent such as acetic acid, tetrahydrofuran, diethyl ether, diisopropyl ether, toluene, acetone, dioxane, benzene, dimethylformamide, dimethylacetamide, dimethyl sulfoxide, hexane and dichloromethane, and reacted in the same manner as in Production 1, Step d, to give a compound (Z).

### Step b'

In Step b', the compound (Z) is reacted in the same manner as in Production 1, Step e, to give a compound (X').

### Step c'

In Step c', the compound (Z) is reacted in the same manner as in Production 1, Step f, to give a compound (A').

### Step d'

In Step d', the compound (A') is reacted in the same manner as in Production 1, Step g, to give a compound (X').

### Step e''

In Step e'', the compound (X') is reacted in the same manner as in Production 1, Step h, to give a compound (B'').

When the compounds of the present invention have a chiral carbon atom as mentioned above, they are generally obtained as racemates. The racemates can be resolved into optically active compounds by a conventional method. Such optically active compounds can be also produced by using an optically active starting compound.

Alternatively, when an optically active starting compound of the formula 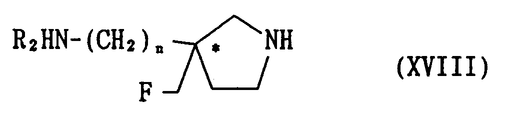
wherein each symbol is as defined above, is used, it can be synthesized by using an optically active α-methylbenzylamine by the following production methods.

### Production 3 : Compounds of the formula (III) wherein n is 0

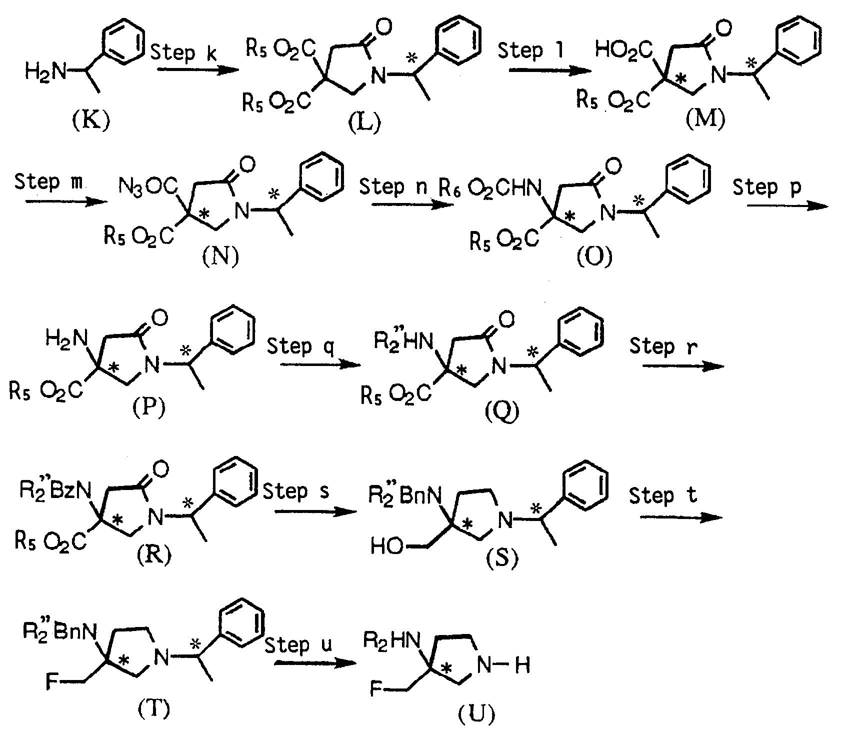

In the above reaction steps, Bn, Bz and R₂ are as defined above, R₂'' is methyl or benzyl, and R₅ and R₆ are each independently lower alkyl or phenylalkyl.

### Step k

In Step k, an optically active α-methylbenzylamine (K) is reacted with formaldehyde in a solvent such as benzene, toluene and xylene in the presence of sodium hydroxide or potassium hydroxide. The obtained compound is reacted with a triester compound of the formula 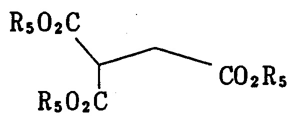
wherein each symbol is as defined above, in the presence of trifluoroacetic acid, whereby an oxo-substituted cyclic amine compound (L) is obtained.

### Step l

In Step l, the compound (L) is hydrolyzed in an alcohol such as methanol, ethanol and isopropyl alcohol, water or a mixed solvent thereof, using an inorganic base such as lithium hydroxide, sodium hydroxide and potassium hydroxide or under the acidic conditions using hydrochloric acid or sulfuric acid, and the obtained monocarboxylic acid compound which is a mixture of diastereomers is subjected to fractional recrystallization for optical resolution by a conventional method to give an optically active monocarboxylic acid compound (M).

Alternatively, the compound (L) is subjected to asymmetric enzyme hydrolysis using esterase derived from animal or plant tissues, or lipase derived from microorganism, to give a compound (M).

### Step m

In Step m, the compound (M) is treated with a chlorinating agent such as thionyl chloride, phosphorous oxychloride and oxalyl chloride, in a solvent such as chloroform, 1,2-dichroloethane, benzene and toluene by a conventional method and the obtained acid chloride compound is treated with an agent for converting into azide, such as sodium azide and potassium azide, in a solvent such as acetone, benzene, toluene and dimethylforamide, or a mixed solvent thereof with water where necessary, to give an optically active carbonyl aside compound (N).

Alternatively, the compound (M) is treated with diethylphospholyl triazide in a solvent such as benzene and toluene by a conventional method to give a compound (N).

### Step n

In Step n, the compound (N) is subjected to Crutius rearrangement by heating the compound (N) in a solvent such as benzene and toluene by a conventional method to give an isocyanate compound. The isocyanate compound is heated with an alcohol compound such as t-butyl alcohol and benzyl alcohol to give an optically active carbamate compound (O).

The compound (O) can be also produced by heating the compound (N) in an alcohol solvent such as t-butyl alcohol and benzyl alcohol.

### Step p

In Step p, the compound (O) wherein R₆ is a lower alkyl is hydrolyzed with an enzyme by a conventional method to give an optically active amine compound (P). When R₆ is, for example, aryl, said compound is reacted with palladium-carbon by a conventional method to give a compound (P). For example, when R₆ is t-butyl, the compound (O) is treated with trifluoroacetic acid by a conventional method to give an optically active amine compound (P). When R₆ is benzyl, the compound (O) is subjected to catalytic hydrogenolysis to give a compound (P).

### Step q

In Step q, the compound (P) is treated with methyl halide such as methyl bromide and methyl iodide, benzyl chloride, benzyl bromide or benzyl iodide, in a solvent such as acetone, acetonitrile, benzene, toluene and dimethylformamide to give an optically active amine compound (Q).

### Step r

In Step r, the compound (Q) is treated with benzoyl chloride compound optionally substituted by lower alkyl, in pyridine or triethylamine by a conventional method to give an optically active amide compound (R).

### Step s

In Step s, the compound (R) is reduced to give an optically active alcohol compound. Examples of the reducing agent include lithium aluminum hydride, diisobutylaluminum hydride and sodium borohydride. When sodium borohydride is used, aluminum chloride, sulfuric acid and the like is added as necessary.

### Step t

In Step t, the compound (S) is terated with a fluorinating agent such as diethylaminosulfur trifluoride and hexafluoropropene diethylamine to give an optically active fluoride compound (T).

### Step u

In Step u, the compound (T) is deprotected to give an optically active amine compound (U). For deprotection, the compound (T) is hydrogenolyzed in a solvent such as methanol, ethanol, isopropanol, acetic acid and ethyl acetate, in the presence of a catalyst such as palladium-carbon, palladium black and palladium hydroxide and, where necessary, an acid such as hydrochloric acid and perchloric acid, under atmospheric pressure or under pressurization, or refluxing in a solvent such as ethanol, isopropanol and acetic acid in the presence of a catalyst such as palladium-carbon, palladium black and palladium hydroxide and using hydrazine hydrate or ammonium formate.

The production of compounds of the formula (III) wherein n is 1, which are synthetic starting materials to be used in the present invention is described in the following. These compounds can be produced, for example, by the following production methods.

### Production of intermediate (III) : Compounds of the formula (III) wherein n is 1

### Production 1

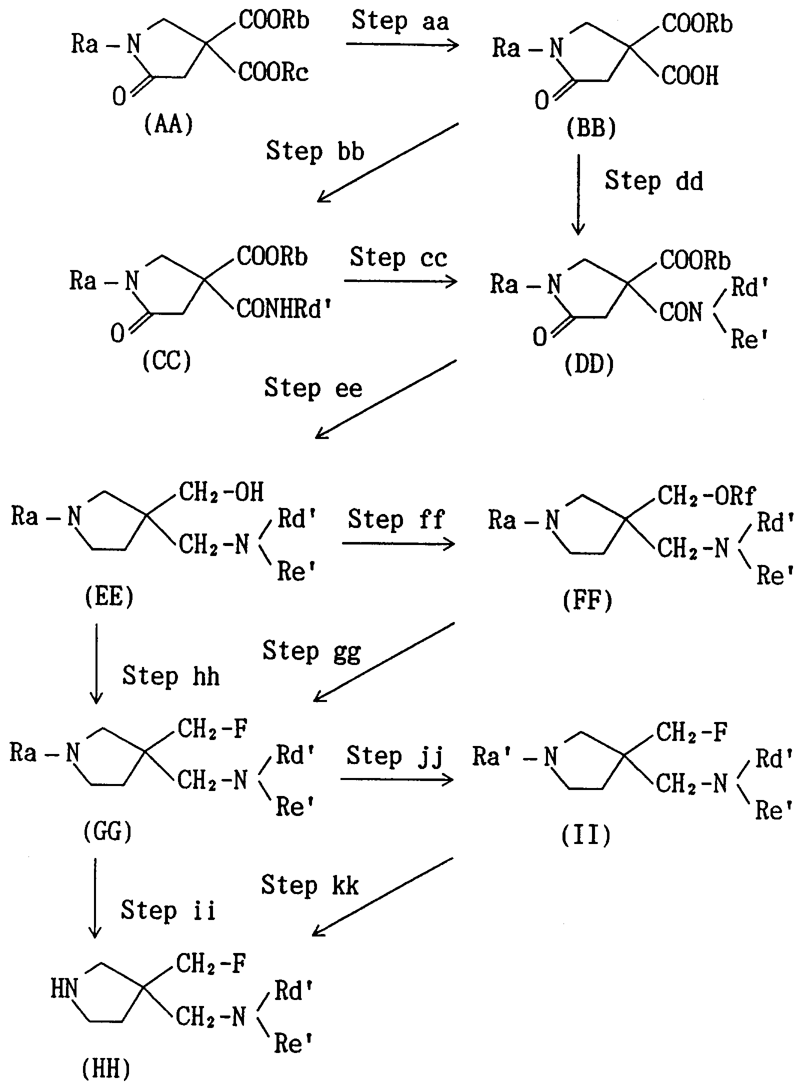

In the above reaction steps, Ra is lower alkyl, acyl or amino-protecting group, Ra' is alkoxycarbonyl such as methoxycarbonyl and ethoxycarbonyl, Rb and Rc are each lower alkyl or phenylalkyl, Rd and Re are each lower akyl or amino-protecting group, Rd' and Re' are each hydrogen atom, lower alkyl or amino-protecting group and Rf is methanesulfonyl, trifluoromethanesulfonyl or p-toluenesulfonyl.

As used herein, acyl is an alkanoyl having 1 to 6 carbon atoms, such as formyl, acetyl, propionyl, butyryl, valeryl and pivaloyl, or arolyl such as benzoyl, toluoyl and naphthoyl.

The amino-protecting group is conventionally used for organic synthesis, and is exemplified by benzyl, substituted benzyl (e.g. 2-, 3- or 4-chlorobenzyl, 2-, 3- or 4-methylbenzyl, 2-, 3- or 4-methoxybenzyl, 2-, 3- or 4-nitrobenzyl and 3,4-dimethoxybenzyl), α-methylbenzyl, (S)-α-methylbenzyl, (R)-α-methylbenzyl, diphenylmethyl, bis(4-methoxyphenyl)methyl, triphenylmethyl, phenacyl, acetyl, trifluoroacetyl, methoxycarbonyl, ethoxycarbonyl, benzyloxycarbonyl and tert-butoxycarbonyl.

The compound (AA) which is the starting material in Production 1 can be synthesized by the method described in, for example, Japanese Patent Unexamined Publication No. 209382/1991.

### Step aa

In Step aa, the compound (AA) is hydrolyzed in an alcohol such as methanol, ethanol and isopropyl alcohol, water or a mixed solvent thereof, in the presence of an inorganic acid such as lithium hydroxide, sodium hydroxide and potassium hydroxide or an acid such as hydrochloric acid and sulfuric acid to give a compound (BB).

### Step bb

In Step bb, the compound (BB) is reacted with amine (Rd'NH₂) in a solvent such as alcohol (e.g. methanol, ethanol and isopropyl alcohol), tetrahydrofuran, diethyl ether, diisopropyl ether, dioxane, dimethylformamide, dimethyl sulfoxide and water, in the presence of a dehydrating agent such as dicyclohexylcarbodiimide triphenylphosphine-diethylazodicarboxylate, or the compound (BB) is converted to a halide such as acid halide or an acid anhydride and amine of Rd'NH₂ is reacted to give a compound (CC).

### Step cc

In Step cc, the compound (CC) is reacted with a lower alkyl halide or phenylalkyl halide in a solvent such as alcohol (e.g. methanol,ethanol and isopropyl alcohol), tetrahydrofuran, diethyl ether, diisopropyl ether, dioxane, dimethylformamide, dimethyl sulfoxide, toluene, xylene and benzene in the presence of an inorganic base such as potassium carbonate, sodium carbonate, potassium hydrogencarbonate, sodium hydrogencarbonate, sodium methoxide, sodium ethoxide, potassium methoxide, potassium ethoxide and sodium hydride to give a compound (DD).

### Step dd

In Step dd, the compound (BB) or the compound (BB) after introducing said compound into an halide such as acid chloride or an acid anhydride, is reacted with an amine (Rd'Re'NH) in the same manner as in Step bb to give a compound (DD).

### Step ee

In Step ee, the compound (DD) is treated as in i) or ii) to give a compound (EE).
i) The compound (DD) is reduced with lithium aluminium hydride or sodium bis(2-methoxyethoxy)aluminum hydride in a solvent such as tetrahydrofuran, diethyl ether, diisopropyl ether, dioxane, toluene, xylene, benzene and diethylene glycol dimethyl ether to give a compound (EE).
ii) The compound (DD) is hydrolyzed under the alkaline or acidic conditions to give a carboxylic acid and reduced with diborane, sodium borohydride-iodine or sodium borohydride-sulfuric acid in a solvent such as tetrahydrofuran, diethyl ether, diisopropyl ether, dioxane, toluene, xylene, benzene and diethylene glycol dimethyl ether to give a compound (EE).

### Step ff

In Step ff, the compound (EE) is reacted with p-toluenesulfonyl chloride, methanesulfonyl chloride or trifluoromethanesulfonic anhydride in a solvent such as tetrahydrofuran, diethyl ether, diisopropyl ether, toluene, xylene, dioxane, benzene, dimethylformamide, dimethylacetamide, dimethyl sulfoxide, hexane, dichloromethane, dichloroethane and chloroform in the presence of a base (e.g. pyridine and triethylamine) to give a compound (FF).

### Step gg

In Step gg, the compound (FF) is reacted with a fluorinating agent such as sodium fluoride, potassium fluoride, magnesium fluoride, calcium fluoride and tetrabutylammonium fluoride in a solvent such as alcohol (e.g. methanol, ethanol and isopropyl alcohol), tetrahydrofuran, diethyl ether, diisopropyl ether, toluene, acetone, acetonitrile, dioxane, dimethylformamide, dimethylacetamide, dimethyl sulfoxide, hexane, dichloromethane, dichloroethane, chloroform, water or a mixed solvent thereof to give a compound (GG).

### Step hh

In Step hh, the compound (EE) is reacted with a fluorinating agent such as diethylaminosulfur trifluoride or hexafluoropropene diethylamine, in a solvent such as tetrahydrofuran, diethyl ether, diisopropyl ether, toluene, xylene, dioxane, benzene, dimethylformamide, dimethylacetamide, dimethyl sulfoxide, hexane, chloroform, dichloromethane and dichloroethane to give a compound (GG).

### Step ii

In Step ii, the compound (GG) is hydrogenolyzed in an alcohol such as methanol, ethanol and isopropyl alcohol or acetic acid, in the presence of a catalyst such as palladium-carbon, palladium hydroxide-carbon and palladium black under heating and under pressurization where necessary, or refluxing under heating for deprotection in an alcohol such as methanol, ethanol, and isopropyl alcohol or acetic acid, in the presence of a catalyst such as palladium-carbon, palladium hydroxide-carbon and palladium black, using hydrazine hydrate or ammonium formate to give a compound (HH).

The compound (GG) wherein Ra is acyl can be converted to a compound (HH) under the alkaline or acidic conditions.

### Step jj

In Step jj, the compound (GG) is reacted with chlorocarbonate (e.g. methyl chlorocarbonate, ethyl chlorocarbonate, isopropyl chlorocarbonate and benzyl chlorocarbonate) in a solvent such as tetrahydrofuran, diethyl ether, diisopropyl ether, toluene, xylene, benzene, dioxane, dimethylformamide, dimethylacetamide, dimethyl sulfoxide, hexane, dichloromethane, dichloroethane and chloroform to give a compound (II).

### Step kk

In Step kk, the compound (II) is reacted with sodium hydroxide, potassium hydroxide and the like, or hydrochloric acid, sulfuric acid, hydrobromic acid-acetic acid and the like without solvent or in an alcohol such as methanol, ethanol, propanol and isopropanol, water or a mixed solvent thereof to give a compound (HH).

### Production 1'

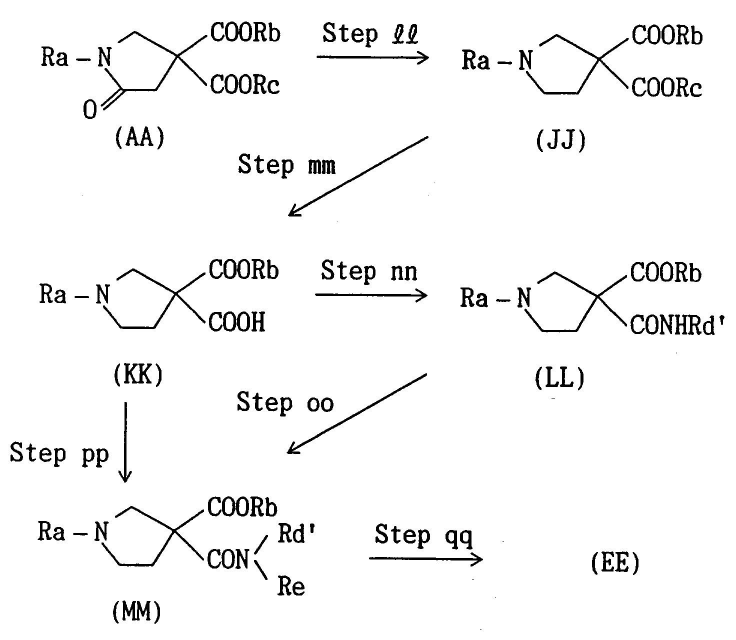

In the above reaction steps, Ra, Rb, Rc, Rd' and Re are as defined above.

### Step ℓℓ

In Step ℓℓ, the compound (AA) is reduced with diborane, sodium borohydride-iodine or sodium borohydride-sulfuric acid, in a solvent such as tetrahydrofuran, diethyl ether, diisopropyl ether, toluene, xylene, benzene and diethylene glycol dimethyl ether to give a compound (JJ).

### Step mm

In Step mm, the compound (JJ) is treated in the same manner as in Step aa to give a compound (KK).

### Step nn

In Step nn, the compound (KK) is treated in the same manner as in Step bb to give a compound (LL).

### Step oo

In Step oo, the compound (LL) is treated in the same manner as in Step cc to give a compound (MM).

### Step pp

In Step pp, the compound (KK) is treated in the same manner as in Step dd to give a compound (MM).

### Step qq

In Step qq, the compound (MM) is treated in the same manner as in Step ee to give a compound (EE).

### Production 2

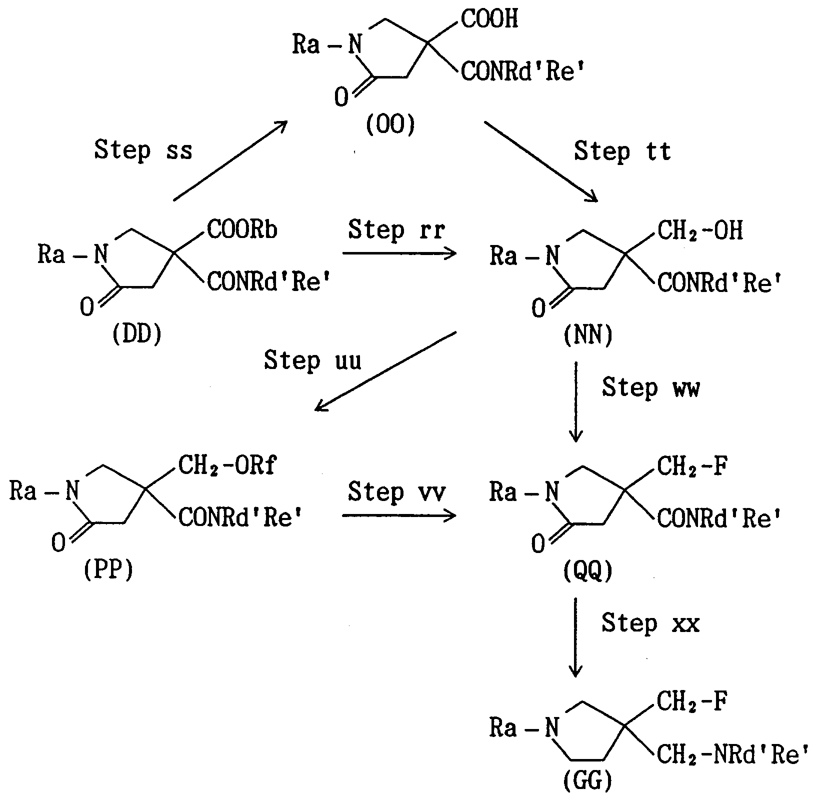

In the above reaction steps, Ra, Rb, Rd', Re' and Rf are as defined above.

### Step rr

In Step rr, the compound (DD) is reduced with sodium borohydride, lithium aluminum hydride and sodium bis(2-methoxyethoxy)aluminum hydride in a solvent such as alcohol (e.g. methanol, ethanol and isopropyl alcohol), tetrahydrofuran, diethyl ether, diisopropyl ether, toluene, xylene and benzene to give a compound (NN).

### Step ss

In Step ss, the compound (DD) is treated in the same manner as in Producation 1, Step aa to give a compound (OO).

### Step tt

In Step tt, the compound (OO) is reacted with thionyl chloride, phosphorus pentachloride, phosphorus oxychloride, chlorocarbonate (e.g. methyl chlorocarbonate, ethyl chlorocarbonate and isopropyl chlorocarbonate) or dimethylformamide-oxalyl chloride in a solvent such as tetrahydrofuran, diethyl ether, diisopropyl ether, toluene, xylene, benzene, dioxane, dimethylformamide, dimethylacetamide, dimethyl sulfoxide, hexane, dichloromethane, dichloroethane and chloroform to give a reactive derivative thereof and the derivative is reduced with sodium borohydride, lithium aluminium hydride or sodium bis(2-methoxyethoxy)aluminum hydride in a solvent such as tetrahydrofuran, diethyl ether, diisopropyl ether, toluene, xylene, benzene, diethylene glycol dimethyl ether, acetonitrile, dimethylformamide and dimethylacetamide to give a compound (NN).

### Step uu

In Step uu, the compound (NN) is treated in the same manner as in Producation 1, Step ff to give a compound (PP).

### Step vv

In Step vv, the compound (PP) is treated in the same manner as in Producation 1, Step gg to give a compound (QQ).

### Step ww

In Step ww, the compound (NN) is treated in the same manner as in Producation 1, Step hh to give a compound (QQ).

### Step xx

In Step xx, the compound (QQ) is treated in the same manner as in Producation 1, Step ee, i) or the compound (QQ) is reduced with diborane, sodium borohydride-iodine or sodium borohydride-sulfuric acid to give a compound (GG).

### Production 2'

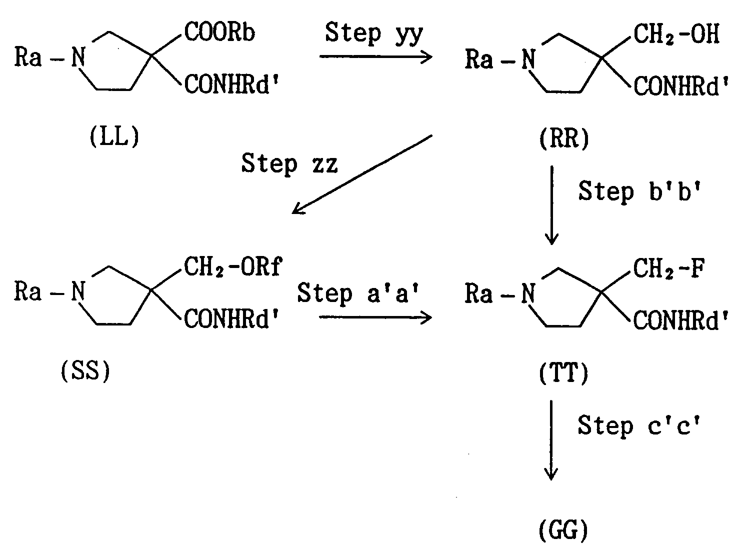

In the above reaction steps, Ra, Rb, Rd' and Rf are as defined above.

### Step yy

In Step yy, the compound (LL) is treated in the same manner as in Step rr to give a compound (RR).

### Step zz

In Step zz, the compound (RR) is treated in the same manner as in Production 1, Step ff to give a compound (SS).

### Step a'a'

In Step a'a', the compound (SS) is treated in the same manner as in Production 1, Step gg to give a compound (TT).

### Step b'b'

In Step b'b', the compound (RR) is treated in the same manner as in Production 1, Step hh to give a compound (TT).

### Step c'c'

In Step c'c', the compound (TT) is treated in the same manner as in Production 1, Step cc and Production 2, Step xx to give a compound (GG).

### Production 3

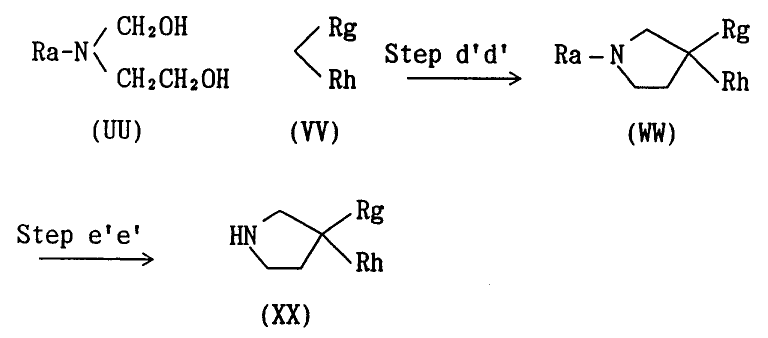

In the above reaction steps, Rg and Rh are each cyano, alkoxycarbonyl, phenylalkoxycarbonyl, carbamoyl or mono- or dialkylcarbamoyl, and Ra is as defined above.

### Step d'd'

In Step d'd', the compound (UU) is reacted with the compound (VV) in a solvent such as tetrahydrofuran,diethyl ether, diisopropyl ether, toluene, xylene, benzene, acetone, acetonitrile, dioxane, dimethylformamide, dimethylacetamide, dimethyl sulfoxide, hexane, dichloromethane, dichloroethane and chloroform in the presence of triphenylphosphine-diethyl azodicarboxylate to give a compound (WW).

### Step e'e'

In Step e'e', the compound (WW) is treated in the same manner as in Production 1, Step ii to give a compound (XX).

In the compound of the formula (WW) wherein Rg and Rh are each cyano, Rg and Rh can be converted to alkoxycarbonyl or phenylalkoxycarbonyl by reacting with an alcohol in the presence of an acid catalyst; to aminomethyl by reduction; to carbamoyl by hydrolysis. In the compound of the formula (WW) wherein Rg and Rh are each alkoxycarbonyl or phenylalkoxycarbonyl, Rg and Rh can be converted to hydroxymethyl by reduction; to carboxy by hydrolysis. In the compound of the formula (WW) wherein Rg and Rh are each carbamoyl, or mono- or dialkylcarbamoyl, Rg and Rh can be converted to aminomethyl and mono- or dialkylaminomethyl by reduction; to carboxy by hydrolysis. The thus-obtained compound wherein Rg and Rh are each hydroxymethyl can be introduced into a compound wherein Rg and Rh are each fluoromethyl according to Production 1, steps ff and gg or step hh and can be deprotected according to step ii.

When the compounds of the present invention have a chiral carbon atom as mentioned above, they are generally obtained as racemates. The racemates can be resolved into optically active compounds by a conventional method. Such optically active compounds can be also produced by using an optically active starting compound.

When an optically active starting compound is used, an oxo-substituted cyclic amine compound of the formula 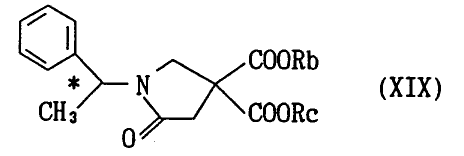
wherein each symbol is as defined above, is synthesized using an optically active α-methylbenzylamine. The obtained compound is reacted according to Production 1, Production 1', Production 2 and Production 2' for the above-mentioned synthetic starting compound. When respective synthetic intermediates have diastereomers, they can be resolved into respective diastereomers by fractional recrystallization or chromatography. The separated diastereomers are reacted according to the respective Production methods to give optically active synthetic intermediates.

In particular, when the optically active intermediate is a cyclic amine compound of the formula 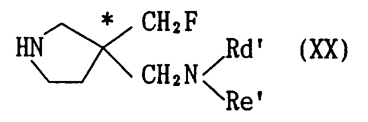
wherein Rd' and Re' are each hydrogen atom, lower alkyl or amino-protecting group, the following methods can be used for the synthesis.

### Production 4

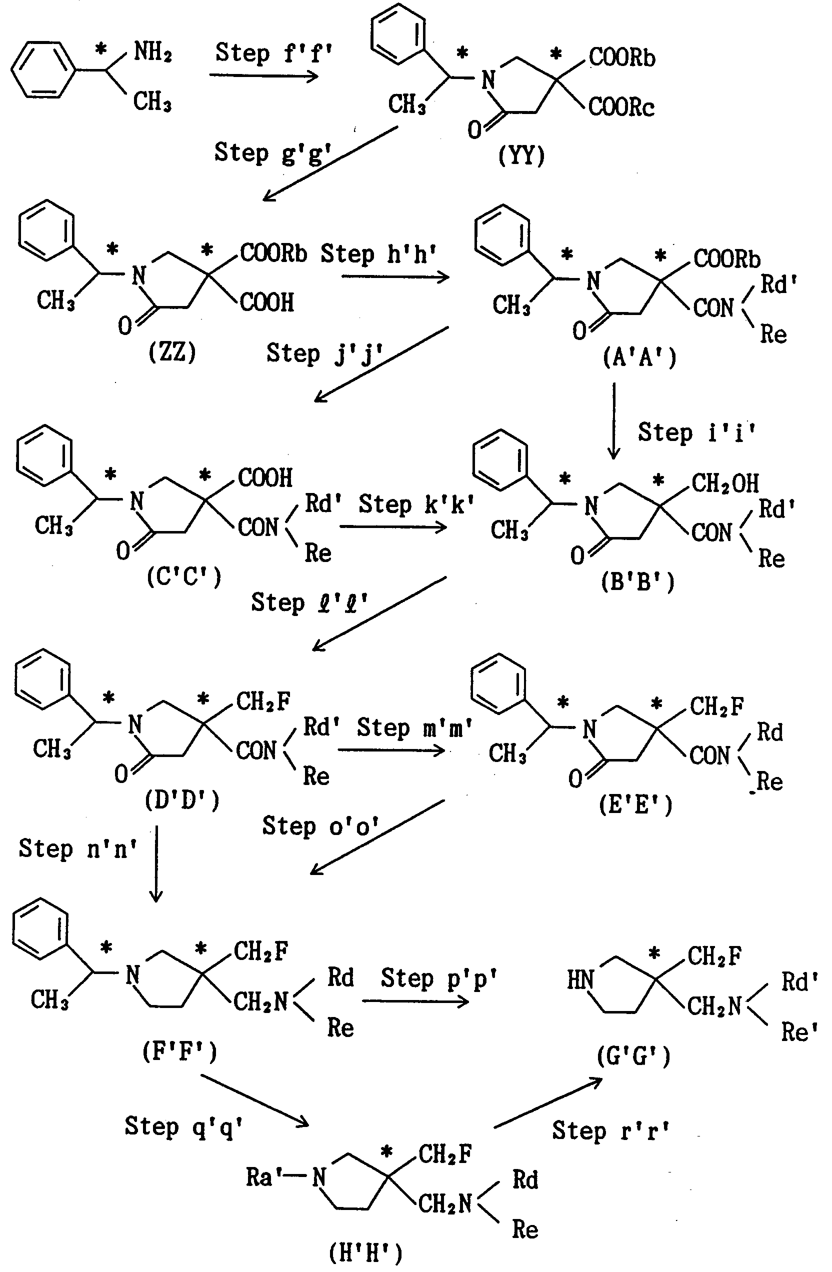

In the above reaction steps, Ra', Rb, Rc, Rd, Rd', Re and Re' are as defined above.

### Step f'f'

In Step f'f', an optically active α-methylbenzylamine is reacted with formaldehyde in a solvent such as toluene, xylene and benzene in the presence of sodium hydroxide or potassium hydroxide, and the obtained compound is reacted with a compound of the formula 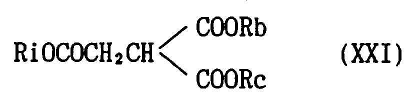
wherein Ri is a lower alkyl or a phenylalkyl and other symbols are as defined above, in the presence of trifluoroacetic acid to give a compound (YY).

### Step g'g'

In Step g'g', the compound (YY) is treated in the same manner as in Production 1, Step aa, to give a compound (ZZ).

### Step h'h'

In Step h'h', the compound (ZZ) is treated in the same manner as in Production 1, Step bb, to give a compound (A'A').

### Step i'i'

In Step i'i', the compound (A'A') is treated in the same manner as in Production 2, Step rr, to give a compound (B'B').

### Step j'j'

In Step j'j', the compound (A'A') is treated in the same manner as in Production 1, Step aa, to give a compound (C'C').

### Step k'k'

In Step k'k', the compound (C'C') is treated in the same manner as in Production 2, Step tt, to give a compound (B'B').

### Step ℓ'ℓ'

In Step ℓ'ℓ', the compound (B'B') is treated in the same manner as in Production 1, Step hh, to give a compound (D'D').

### Step m'm'

In Step m'm', the compound (D'D') is treated in the same manner as in Production 1, Step cc, to give a compound (E'E').

### Step n'n'

In Step n'n', the compound (D'D') is treated in the same manner as in Production 2, Step xx, to give a compound (F'F').

### Step o'o'

In Step o'o', the compound (E'E') is treated in the same manner as in Production 1, Step ee, to give a compound (F'F').

### Step p'p'

In Step p'p', the compound (F'F') is treated in the same manner as in Production 1, Step ii, to give a compound (G'G').

### Step q'q'

In Step q'q', the compound (F'F') is treated in the same manner as in Production 1, Step jj, to give a compound (H'H').

### Step r'r'

In Step r'r', the compound (H'H') is treated in the same manner as in Production 1, Step kk, to give a compound (G'G').

In these reaction steps, the compounds (ZZ), (A'A'), (B'B'), (C'C'), (D'D'), (E'E') and (F'F') have optical isomers. These respective diastereomers are optically resolved by fractional recrystallization or chromatography, and the optically active compounds resolved are treated according to respective reaction steps mentioned above to give an optically active intermediate (G'G').

In particular, the compound (B'B') is optically resolved by silica gel column chromatography and the optically active compound obtained is preferably reacted according to the above steps.

According to the production method of the present invention, the diester compounds of (AA), (JJ) and (YY) or the ester compounds of (DD) and (A'A') are asymmetrically hydrolyzed using lipase derived from microorganism, lipoprotein lipase derived from microorganism, esterase derived from animal tissues or esterase derived from plant tissues.

While the pH of hydrolysis by an enzyme is appropriately determined according to the enzyme to be used, it is generally in the range of from 4 to 9. While the reaction temperature is appropriately determined according to the enzyme to be used, it is generally in the range of from 10°C to 50°C.

The optically active compounds (BB), (KK), (ZZ), (OO) and (C'C') thus obtained can be reacted according to each step of respective Production methods to give an optically active compound (G'G').

The compounds (I) of the present invention thus obtained can be separated and purified from reaction mixture by recrystallization and chromatography as necessary.

The compound (I) of the present invention can be converted to pharmaceutically acceptable salts by treating with an inorganic acid (e.g. hydrochloric acid, hydrobromic acid, sulfuric acid, phosphoric acid and nitric acid), an organic acid (e.g. acetic acid, propionic acid, succinic acid, glycolic acid, lactic acid, malic acid, tartaric acid, citric acid, maleic acid, fumaric acid, methanesulfonic acid, p-toluenesulfonic acid and ascorbic acid), an amino acid (e.g. lysine and ornithine), alkali metal salts, alkaline earth metal salts (e.g. salts of sodium, potassium, magnesium and calcium) or oxide of heavy metal salts (e.g. salts of copper, zinc, iron, gold, silver, platinum and manganese), and further into hydrates or various solvates.

When the compounds of the present invention have a chiral carbon atom as mentioned above, they are generally obtained as racemates. The racemates can be resolved into optically active compounds by a conventional method. Such optically active compounds can be also produced by using an optically active starting compound. Respective enantiomers can be purified by fractional recrystallization.

When the compound of the present invention is used as an antibacterial agent, a therapeutically effective amount of the compound of the present invention is prepared using an organic or inorganic, solid or liquid pharmacologically acceptable carrier into a conventional preparation form, and administered orally, parenterally or externally.

Examples of the pharmaceutical preparation include solid preparations such as tablet, granule, powder and capsule, and emulsions such as suspension, syrup, emulsion and lemonade. Where necessary, auxiliary, lubricant and other conventional additives such as lactose, magnesium stearate, kaolin, sucrose, corn starch, talc, stearic acid, gelatin, agar, pectin, peanut oil and cacao butter can be added.

While the dose of the compound of the present invention varies depending on age, sex, symptom etc. of patients, the kind of disease, the kind of the compound to be administered and administration route, the compound of the present invention can be generally administered in 1 mg to about 4,000 mg or above daily. The average dose of the compound of the present invention per administration is about 50 mg, 100 mg, 250 mg, 500 mg, 1,000 mg or 2,000 mg for the treatment of the diseases caused by pathogenic microorganisms.

### Experimental Example

The antibacterial effect of the compound of the present invention is examined by the following test. The test compounds used are as follows.
Example 1: 7-(3-amino-3-fluoromethylpyrrolidin-1-yl)-1-cyclopropyl-6-fluoro-1,4-dihydro-8-methoxy-4-oxo-3-quinolinecarboxylic acid
Example 5: (R)-7-(3-amino-3-fluoromethylpyrrolidin-1-yl)-1-cyclopropyl-6-fluoro-1,4-dihydro-8-methoxy-4-oxo-3-quinolinecarboxylic acid
Example 6: (S)-7-(3-amino-3-fluoromethylpyrrolidin-1-yl)-1-cyclopropyl-6-fluoro-1,4-dihydro-8-methoxy-4-oxo-3-quinoline carboxylic acid
Example 17: (S)-7-(3-aminomethyl-3-fluoromethylpyrrolidin-1-yl)-1-cyclopropyl-6-fluoro-1,4-dihydro-8-methoxy-4-oxo-3-quinolinecarboxylic acid
Comparative Example 1: 7-(4-amino-4-aminomethylpiperidin-1-yl)-1-cyclopropyl-6-fluoro-1,4-dihydro-8-methoxy-4-oxo-3-quinolinecarboxylic acid
Comparative Example 2: 9-fluoro-2,3-dihydro-3-methyl-10-(4-methyl-1-piperazinyl)-7-oxo-7H-pyrido [1. 2. 3-de) [1.4] benzoxazine-6-carboxylic acid (ofloxacin)

### Experiment 1 : Antibacterial activity (in vitro)

The *in vitro* antibacterial activity (minimum inhibitory concentration, MIC, µg/ml) of the test compounds was determined according to Japan Society of Chemotherapy Standard [Chemotherapy, vol. 29, pp 76-79 (1981)]. The antibacterial spectrum is shown in Table 1-1 and Table 1-2.

**Table 1-1**

| Antibacterial activity (MIC : µg/ml) | | | | | |
|---|---|---|---|---|---|
| Strain | Example 6 | Example 5 | Example 1 | Comp. Ex. 1 | Comp. Ex. 2 |
| Gram-positive bacteria | | | | | |
| S.aureus FDA 209P | ≦ 0.006 | ≦ 0.006 | ≦ 0.006 | 0.10 | 0.20 |
| S.aureus No.88 ¹⁾ | 0.39 | 0.78 | 0.39 | - | 50 |
| S.epidermidis ATCC 12228 | ≦ 0.006 | 0.012 | 0.012 | 0.78 | 0.20 |
| S.pyogenes C-203 * | 0.05 | 0.05 | 0.05 | - | 1.56 |
| S.pneumoniae Type-III * | 0.025 | 0.012 | 0.012 | - | 0.78 |
| E.faecalis LS-101 | 0.012 | 0.012 | 0.012 | 1.56 | 0.39 |
| E.faecium EFMY-28 | 0.20 | 0.10 | 0.20 | - | 3.13 |
| E.avium EAY-30 | 0.10 | 0.10 | 0.10 | - | 1.56 |

| Gram-negative bacteria | | | | | |
|---|---|---|---|---|---|
| E.coli NIHJ JC-2 | 0.025 | 0.025 | 0.025 | 1.56 | 0.05 |
| S.flexneri EW-10 | 0.012 | 0.012 | ≦ 0.006 | 0.39 | 0.05 |
| C.freundii IFO 12681 | 0.10 | 0.05 | 0.05 | - | 0.10 |
| K.pneumoniae DT-S | 0.05 | 0.05 | 0.025 | 6.25 | 0.05 |
| E.cloacae IFO 12937 | 0.20 | 0.20 | 0.20 | 12.5 | 0.39 |
| S.marcescens IFO 12648 | 0.20 | 0.20 | 0.20 | 6.25 | 0.20 |
| P.vulgaris IFO 3988 | 0.39 | 0.39 | 0.20 | 25 | 0.10 |
| P.mirabilis IFO 3849 | 0.20 | 0.20 | 0.39 | 25 | 0.39 |
| A.calcoaceticus IFO 13006 | 0.025 | 0.025 | 0.05 | - | 0.20 |
| P.aeruginosa U-31 | 0.39 | 0.39 | 0.39 | 12.5 | 1.56 |
| L.monocytogenes VTU-206 * | 0.10 | 0.10 | 0.10 | - | 3.13 |
| M.catarrhalis ATCC 25238 * | 0.012 | 0.012 | 0.012 | - | 0.10 |

**Table 1-2**

| Antibacterial activity (MIC : µg/ml) | | |
|---|---|---|
| Strain | Example 17 | Comp. Ex. 2 |
| Staphylococcus aureus FDA 209P | 0.012 | 0.20 |
| S. aureus No.88¹⁾ | 0.78 | 50 |
| S. epidermidis ATCC 12228 | 0.025 | 0.20 |
| S. epidermidis SEY-223²⁾ | 0.78 | > 100 |
| Streptococcus pyogenes C-203* | 0.10 | 1.56 |
| S. viridans America * | 0.10 | 3.13 |
| S. pneumoniae Type-III* | 0.05 | 0.78 |
| Enterococcus faecalis LS-101 | 0.025 | 0.39 |
| E. faecalis EFY-204²⁾ | 1.56 | 50 |
| E. faecium EFMY-28 | 0.39 | 3.13 |
| E. faecium EFMY-207 ²⁾ | 3.13 | 100 |
| E. avium EAY-30 | 0.20 | 3.13 |
| Corynebacterium diphtheriae Tronto* | 0.025 | 0.39 |
| Escherichia coli NIHJ JC-2 | 0.10 | 0.05 |
| Shigella flexneri EW-10 | 0.025 | 0.05 |
| Klebsiella pneumoniae DT | 0.05 | 0.10 |
| Proteus vulgaris IFO 3988 | 0.78 | 0.10 |
| P. mirabilis IFO 3849 | 0.78 | 0.39 |
| Serratia marcescens IFO 12648 | 0.39 | 0.20 |
| Acinetobacter calcoaceticus ATCC 13006 | 0.10 | 0.20 |
| Citrobacter freundii IFO 12681 | 0.20 | 0.10 |
| Enterobacter cloacae IFO 12937 | 0.39 | 0.39 |
| Pseudomonas aeruginosa IFO 12582 | 1.56 | 1.56 |

Inoculated cells were 10⁶ cells/ml and the strain with * was incubated in a medium supplemented with 10% horse blood. The mark "1)" means quinolone resistant MRSA and "2)" means quinolone resistant bacteria.

### Experimental Example 2

In the same manner as in Experimental Example 1, the antibacterial effect against specific bacteria such as *Chlamydia*, *Mycoplasma* and acid-fast bacteria and anaerobic bacteria was determined. The results are shown in Table 2-1 and Table 2-2.

**Table 2-1**

| Antibacterial activity (MIC : µg/ml) | | |
|---|---|---|
| Strain | Example 6 | Comp. Ex. 2 |
| Mycobacterium smegmatis IFO 13167 | 0.10 | 0.39 |
| Mycoplasma pneumoniae ATCC 29343 | 0.025 | 1.56 |
| Ureaplasma urealyticum ATCC 27618 | 0.10 | 3.13 |
| Chlamydia trachomatis D/UW-3Cx | 0.012 | 0.39 |
| Bacteroides fragilis GAI 5560 | 0.05 | 1.56 |

**Table 2-2**

| Antibacterial activity (MIC : µg/ml) | | |
|---|---|---|
| Strain | Example 17 | Comp. Ex. 2 |
| Mycobacterium smegmatis IFO 3153 | 0.10 | 0.39 |
| Mycoplasma pneumoniae ATCC 29343 | 0.10 | 1.56 |
| Ureaplasma urealyticum ATCC 27618 | 0.39 | 3.13 |
| Chlamydia trachomatis D/UW-3Cx | 0.025 | 0.39 |
| B. fragilis BFY-18 | 0.20 | 1.56 |

### Experimental Example 3 : Protection of mice from experimental infection

Experimental infection was caused by intraperitoneal inoculation of respective bacteria to male mice. One hour after the inoculation, a test compound was orally administered to the mice. The therapeutic effect of each test compound was evaluated by reference to the 50% effective dose (ED₅₀) which was based on the survival ratio at 7 days after the administration according to the probit method [Proc. Soc. Exp. Med., Vol. 57, p 261-264 (1944)]. The minimum inhibitory concentration (MIC) of the infectious bacteria used was also determined. The results are shown in Table 3-1 and Table 3-2. The compounds of the present invention showed superior antibacterial activity *in vivo*.

**Table 3-1**

| Strain | Amount of inoculation (cells/mouse) | Test compound | MIC (µg/ml) | ED₅₀ (mg/mouse) (95% confidence limit) |
|---|---|---|---|---|
| S. aureus | 2.8×10 ⁶ | Ex. 6 | ≦ 0.006 | 0.036(0.025-0.052) |
| Smith | (123×LD₅₀) | Com. Ex.2 | 0.05 | 0.208(0.137-0.344) |
| S. aureus | 7.5×10 ⁶ | Ex. 6 | 0.39 | 1.54(1.103-2.151) |
| MRSA Y-238 | (185×LD₅₀) | Com. Ex.2 | > 100 | > 16 |
| E. coli | 5.0×10 ⁶ | Ex. 6 | 0.012 | 0.011(0.008-0.015) |
| KC-14 | (744×LD₅₀) | Com. Ex.2 | 0.05 | 0.012(0.009-0.018) |

**Table 3-2**

| Strain | Amount of inoculation (cells/mouse) | Test compound | MIC (µg/ml) | ED₅₀ (mg/mouse) (95% confidence limit) |
|---|---|---|---|---|
| S. aureus | 2.8×10⁶ | Ex. 17 | ≦ 0.006 | 0.031(0.021-0.044) |
| Smith | (123×LD₅₀) | Com. Ex.2 | 0.05 | 0.208(0.137-0.344) |
| S. aureus | 7.5×10⁵ | Ex. 17 | 0.39 | 1.540(1.103-2.151) |
| MRSA Y-238 | (185×LD₅₀) | Com. Ex.2 | > 100 | > 16 |
| E. coli | 5.0×10⁴ | Ex. 17 | 0.025 | 0.011(0.008-0.015) |
| KC-14 | (744×LD₅₀) | Com. Ex.2 | 0.05 | 0.012(0.009-0.018) |

### Experimental Example 4 : Acute toxicity

Male ddY mice (5 per group) were orally administered with the compound of Example (2,000 mg/kg). No death case or abnormality was found.

### Experimental Example 5 : Preliminary micronucleus test

A preliminary micronucleus test was performed by oral administration to male ICR mice. The results are shown in Table 4. The group administered with the compound of Example 6 did not show significant difference in emergence of micronucleus in polychromatic cells after 24 hours, from the control group, thus indicating that the compound had no influence on the chromosome of bone marrow cells. The group administered with the compound of Example 5 showed significant increase in emergence frequency of micronucleus, in comparison with the control group administered in the dose of 1,000 mg/kg or above.

**Table 4**

| Micronucleus test using mice | | |
|---|---|---|
| Drug | Dose (mg/kg) | Emergence of micronucleus (%) |
| 0.5%HPMC | - | 0.20±0.04 |
| Example 6 | 500 | 0.16±0.16 |
| " | 1000 | 0.16±0.11 |
| " | 2000 | 0.26±0.04 |
| Example 5 | 500 | 0.45±0.28* |
| " | 1000 | 1.45±1.45** |
| " | 2000 | 2.38±0.85** |
| MMC | 1 | 2.36±1.52** |
| HPMC is hydroxypropylmethylcellulose and MMC is mitomycin C. | | |

| | | |
|---|---|---|
| * means significant difference of 0.05<P and | | |
| ** means significant difference of 0.01<P when compared with 0.5% HPMC. | | |

As is evident from the Experimental Examples as mentioned above, the compound (I) of the present invention wherein n is 0 retains strong antibacterial activity against Gram-negative bacteria, that the conventional pyridonecarboxylic acid antibacterial agents have, and additionally shows enforced and a wide range of *in vitro* and *in vivo* antibacterial effects against Gram-positive bacteria such as *Staphylococcus* (e.g. methicillin- and cephem-resistant *Staphylococcus aureus* and *Staphylococcus epidermidis*), *Streptococcus pneumoniae* and *Enterococcus*, as well as strong antibacterial effects against anaerobic bacteria, *Chlamydia*, *Mycoplasma* and acid-fast bacteria. In addition, the compound scarcely shows problematic side-effects and is low toxic. Accordingly, the compound of the present invention is expected to show clinically superior effects as a therapeutic agent for various infectious diseases caused by these pathogenic bacteria. According to the tests performed by the present inventors, the compounds of the present invention showed not only fine antibacterial activity on the whole, but also fine antibacterial activity against *Pseudomonas aeruginosa* and superior antibacterial activity against MRSA which is considered to be difficult to treat with the presently-available drugs. In particular, the compounds showed remarkably strong antibacterial activity against quinolone resistant *Staphylococcus aureus*. The compounds scarcely caused problematic side-effects and were low toxic, so that clinically superior utility as new antibacterial agents is expected. In particular, the compound (I) of the present invention wherein n is 0 markedly reduces frequency of micronucleus emergence in comparison with conventional antibacterial agents, and an optically active compound thereof further enhances the effect, as demonstrated by the effect achieved by the (R) compound, which was 1,000 times or more greater than that achieved by the conventional antibacterial agents. The effect of the (S) compound was extremely higher than the conventional antibacterial agents and reached a 10,000 times or more greater level. The compounds of Comparative Example 1 etc. were also evaluated by the *in vitro* test of Experimental Example 1 and found to show somewhat weaker property as compared with the compounds of the present invention.

The compound (I) of the present invention wherein n is 1 retains a strong antibacterial effect against Gram-negative bacteria, as mentioned above, and additionally shows enforced effects and a wide range of antibacterial effects against Gram-positive bacteria, both *in vitro* and *in vivo*. In addition, the problematic side-effects on the central nervous system were scarcely found and the compound was low toxic. Accordingly, clinically superior utility as an antibacterial agent is expected. In particular, the compound showed a markedly enforced antibacterial effect against Gram-negative bacteria such as *Staphylococcus aureus*, *Staphylococcus epidermidis*, *Streptococcus pneumoniae* and *Enterococcus*, while retaining an antibacterial effect against Gram-negative bacteria, which was equal to or superior to that of the conventional quinolonecarboxylic acid antibacterial agents. In addition, the compound showed a strong antibacterial effect against MRSA, quinolone resistant MRSA, quinolone resistant *Staphylococcus epidermidis* and quinolone resistant *Enterococcus*, as well as against anaerobic bacteria, *Chlamydia*, *Mycoplasma* and acid-fast bacteria. Since the strong *in vitro* antibacterial effect against such wide range of bacteria has been proven to be consistent with the therapeutic effect in mice, the compound is expected to show extremely superior clinical effects as a therapeutic agent for various infectious diseases caused by these pathogenic bacteria. In the absence of a suitable treatment drug for the infectious diseases caused by multiple drug resistant MRSA which poses very serious clinical problems as a causative bacterium of nosocomial infections, or the infectious diseases caused by, from among the causative bacteria of complicated urinary tract infection, *Staphylococcus epidermidis* or *Enterococcus* having resistance to both oral cephem preparations and quinolone preparations, the clinical utility of the compound of the present invention is considered to be extremely high. As discussed earlier, the compounds of the present invention have antibacterial effects against an extremely broad range of pathogenic bacteria including resistant bacteria. Therefore, the compounds of the present invention are expected to scarcely cause superinfection even when administered as a therapeutic agent for bacterial infections on an extended term basis to patients who acquired immune deficiency as a result of administrations of anticancer drugs and other medicaments.

### Examples

The present invention is explained in the following by way of Preparative Examples and Examples. It is needless to say that the present invention is not limited to these Examples alone.

### Preparative Example 1

### (1) 4-Benzamido-1-benzyl-4-cyanopiperidine

(a) 1-Benzyl-4-piperidone (40 g) was dissolved in a mixed solvent of 25% aqueous ammonia (30 ml) and methanol (35 ml), and an aqueous solution (44 ml) of ammonium chloride (11.4 g) and sodium cyanide (9.5 g) was added. The mixture was stirred overnight at 50°C. Methanol was distilled away and the residue was extracted with chloroform. The chloroform layer was washed with water and dried over magnesium sulfate. The solvent was distilled away to give an oil.
(b) This oil was dissolved in chloroform (250 ml) and triethylamine (32.2 ml) was added. Benzoyl chloride (29.8 g) was dropwise added with stirring under ice-cooling and the mixture was stirred at room temperature for 3 hours. The reaction mixture was washed with water and dried over magnesium sulfate. The solvent was distilled away and the residue was purified by silica gel column chromatography to give 48.7 g of the object compound as colorless powdery crystals, melting point 166-167°C.

### (2) 4-Benzamido-1-benzyl-4-ethoxycarbonylpiperidine

4-Benzamido-1-benzyl-4-cyanopiperidine (21 g) obtained in Preparative Example 1 (1) was dissolved in ethanol (350 ml) and conc. sulfuric acid (15 ml) was added. The mixture was refluxed under heating for 20 hours. Ethanol was distilled away and water (200 ml) was added. The mixture was neutralized with potassium carbonate. Chloroform was added and the mixture was partitioned. The chloroform layer was washed with water and dried over magnesium sulfate, and the solvent was distilled away. The residue was purified by silica gel column chromatography to give 16.1 g of the object compound as colorless powdery crystals, melting point 104-105°C.

### (3) 1-Benzyl-4-benzylamino-4-hydroxymethylpiperidine

Lithium aluminum hydride (3.8 g) was suspended in tetrahydrofuran (300 ml) and a solution of 4-benzamido-1-benzyl-4-ethoxycarbonylpiperidine (16.1 g) obtained in Preparative Example 1 (2), in tetrahydrofuran (150 ml), was dropwise added under ice-cooling. The reaction mixture was refluxed under heating overnight. A small amount of water and an aqueous solution of 15% sodium hydroxide was added under ice-cooling. The insoluble matter was filtered off and the filtrate was dried over magnesium sulfate. The solvent was distilled away to give 12.8 g of the object compound as colorless prism crystals, melting point 83-84°C.

### Preparative Example 2

### (1) 7-Benzyl-1,3,7-triazaspiro[4,4]nonane-2,4-dione

1-Benzyl-3-pyrrolidone (20.9 g), sodium cyanide (8.7 g) and ammonium carbonate (34.4 g) were dissolved in a 50% aqueous solution (200 ml) of ethanol and the mixture was heated overnight at 150°C. Ethanol was distilled away under reduced pressure and the residue was extracted with chloroform. The extract was washed with saturated brine and the organic layer was dried over magnesium sulfate. The solvent was distilled away and the residue was purified by silica gel column chromatography (eluent; chloroform:methanol=10:1) to give 18 g of the object compound as colorless crystals.
NMR(CDCl₃)δ : 2.0-2.1(1H,m), 2.4-2.6(3H,m), 2.7(1H,d,J=10Hz), 2.9(1H,d,J=10Hz), 3.1(1H,m), 3.6(1H,d,J=13Hz), 3.75(1H,d,J=13Hz), 7.2-7.4(5H,m)

### (2) 1-Benzyl-3-benzamido-3-methoxycarbonylpyrrolidine

7-Benzyl-1,3,7-triazaspiro[4,4]nonane-2,4-dione (18 g) obtained in Preparative Example 2 (1) was suspended in a 4N sodium hydroxide solution (183 ml) and the suspension was refluxed under heating for 36 hours. Conc. hydrochloric acid was added to the reaction mixture under ice-cooling to make the mixture acidic, and the mixture was concentrated to dryness under reduced pressure. Dry methanol (100 ml) was added to the residue, and thionyl chloride (13 ml) was dropwise added under ice-cooling. The mixture was stirred at room temperature overnight. Methanol was distilled away under reduced pressure and the residue was dissolved in tetrahydrofuran-water (2:3, 500 ml). Potassium carbonate (150 g) was added to make the solution alkaline, and benzoyl chloride (28 ml) was dropwise added under ice-cooling. The mixture was stirred at room temperature for 2 hours, and the reaction mixture was extracted with chloroform. The organic layer was washed with saturated brine and dried over magnesium sulfate. The solvent was distilled away and the residue was purified by silica gel column chromatography (eluent; chloroform : methanol=95:5) to give 14.6 g of the object compound as a pale-yellow oil.
NMR(CDCl₃)δ : 2.4-2.5(1H,m), 2.7-2.85(2H,m), 3.15(1H,d,J=10Hz), 3.25-3.35(1H,d,J=10Hz), 3.45(1H,d,J=10Hz), 3.75(3H,s), 3.85(1H,d,J=12Hz), 3.95(1H,d,J=12Hz), 7.3-7.6(6H,m), 7.8-7.9(2H,m), 8.0-8.1(2H,m)

### (3) 1-Benzyl-3-benzylamino-3-hydroxymethylpyrrolidine

Lithium aluminum hydride (4.1 g) was suspended in tetrahydrofuran (150 ml) and a solution of 1-benzyl-3-benzamido-3-methoxycarbonylpyrrolidine (14.6 g) obtained in Preparative Example 2 (2), in tetrahydrofuran (50 ml), was dropwise added under ice-cooling. The mixture was refluxed under heating for 12 hours, and water (4 ml), a 15% aqueous solution (4 ml) of sodium hydroxide and water (13 ml) were sequentially added under ice-cooling. The resulting precipitate was filtered off and the filtrate was concentrated to give 12.5 g of the object compound as a colorless oil.
NMR(CDCl₃)δ : 1.8-1.9(2H,m), 2.5-2.75(4H,m), 3.5-3.75(6H,m), 7.2-7.4(10H,m)

### Preparative Example 3

### (1) 3-Amino-3-cyano-1-diphenylmethylazetidine

A solution of 1-diphenylmethylazetidin-3-one [H. Baumann and R.O. Duthaler, Helvetica Chimica Acta Vol.71, 1038 (1988)] (57.4 g) in methanol (172 ml) was added to an aqueous solution (28.5 ml) of ammonium chloride (14.2 g). An aqueous solution (26 ml) of sodium cyanide (13.0 g) was added and the mixture was stirred at room temperature for 12 hours. Water and chloroform were added for partitioning and the organic layer was dried over magnesium sulfate and concentrated to give 63.7 g of the object compound as a brown oil.

### (2) 3-Benzamido-3-cyano-1-diphenylmethylazetidine

3-Amino-3-cyano-1-diphenylmethylazetidine (63.7 g) obtained in Preparative Example 3 (1) and triethylamine (40.5 ml) were dissolved in dichloromethane (637 ml) and benzoyl chloride (33.7 ml) was dropwise added with stirring under ice-cooling. The mixture was allowed to react at room temperature for 12 hours and water (500 ml) and ether (200 ml) were added. The mixture was stirred and the resultant precipitate was collected by filtration and dried to give 51.8 g of the object compound as pale-yellow amorphous.
NMR(CDCl₃)δ : 3.41(2H,d,J=10.0Hz), 4.02(2H,d,J=10.0Hz), 4.43(1H,s), 7.2-7.31(6H,m), 7.41-7.49(6H,m), 7.60-7.70(1H,m), 8.00-8.03(2H,m)

### (3) 3-Benzamido-3-ethoxycarbonyl-1-diphenylmethylazetidine

3-Benzamido-3-cyano-1-diphenylmethylazetidine (17 g) obtained in Preparative Example 3 (2) was dissolved in tetrahydrofuran (85 ml) and conc. hydrochloric acid (425 ml) was added. The mixture was allowed to react at room temperature for 12 hours. The reaction mixture was poured into water (1600 ml), extracted with dichloromethane and concentrated to give 17 g of crude carboxylic acid. This was dissolved in ethanol (432 ml), and thionyl chloride (34 ml) was dropwise added with stirring under ice-cooling. The reaction mixture was refluxed under heating overnight and the insoluble matter was filtered off. The filtrate was concentrated to give the object compound as pale-yellow amorphous.
NMR(CDCl₃)δ : 1.25(3H,t,J=7.1Hz), 3.52(2H,d,J=10.3Hz), 3.87(2H,d,J=10.3Hz), 4.25(2H,q,J=7.1Hz), 4.57(1H,s), 7.18-7.29(6H,m), 7.43-7.48(6H,m), 7.55-7.65(1H,m), 8.01-8.10(2H,m)

### (4) 3-Amino-3-hydroxymethyl-1-diphenylmethylazetidine

Lithium aluminum hydride (4.7 g) was suspended in tetrahydrofuran (47 ml). Thereto was dropwise added a solution of 3-benzamido-3-ethoxycarbonyl-1-diphenylmethylazetidine (23.2 g) obtained in Preparative Example 3 (3), in tetrahydrofuran (230 ml), with stirring under ice-cooling. The mixture was stirred at room temperature for 1 hour and ice-cooled again. Water (4.7 ml), a 15% aqueous solution of sodium hydroxide (4.7 ml) and water (24 ml) were sequentially added and the resulting precipitate was filtered off. The filtrate was concentrated and the residue was purified by silica gel column chromatography to give 5.23 g of the object compound as pale-yellow amorphous which was a debenzoylated reduced product.
NMR(CD₃OD)δ : 2.92(2H,d,J=9.0Hz), 3.30(2H,d,J=9.0Hz), 3.70 (2H,s), 4.46(1H,s), 7.14-7.28(6H,m), 7.39-7.41(4H,m)

### Preparative Example 4

### (1) 1-Benzyl-4-cyano-4-(N-methyl)benzamidopiperidine

Methylamine hydrochloride (9.2 g) and sodium cyanide (6.7 g) were dissolved in water (40 ml). Thereto was dropwise added a solution of 1-benzyl-4-piperidone (25.5 g) in methanol (40 ml) and the mixture was stirred at room temperature for 4 hours. Water and chloroform were added for partitioning and the organic layer was dried over magnesium sulfate and concentrated to give crude aminonitrile compound as a residue. The residue was dissolved in dichloromethane (300 ml) together with triethylamine (20.4 g), and benzoyl chloride (28.4 g) was dropwise added with stirring under ice-cooling. The mixture was stirred at room temperature overnight and the reaction mixture was treated with an aqueous solution of sodium hydrogencarbonate. The organic layer was dried over magnesium sulfate and concentrated. The residue obtained was purified by silica gel column chromatography to give 30.8 g of the object compound as a pale-yellow oil.
NMR(CDCl₃)δ : 2.05-2.20(2H,m), 2.48-2.53(4H,m), 2.85-3.00(5H,m), 3.57(2H,s), 7.20-7.60(10H,m)

### (2) 1-Benzyl-4-ethoxycarbonyl-4-(N-methyl)benzamidopiperidine

1-Benzyl-4-cyano-4-(N-methyl)benzamidopiperidine (10.0 g) obtained in Preparative Example 4 (1) was treated in the same manner as in Preparative Example 1 (2) to give 5.2 g of the object compound as a pale-yellow oil.
NMR(CDCl₃)δ : 1.26(3H,t), 2.17-2.20(2H,m), 2.32-2.35(2H,m), 2.59-2.63(4H,m), 3.03(3H,s), 3.54(2H,s), 4.20(2H,q), 7.26-7.42(10H,m)

### (3) 1-Benzyl-4-hydroxymethyl-4-(N-benzyl-N-methyl)aminopiperidine

1-Benzyl-4-ethoxycarbonyl-4-(N-methyl)benzamidopiperidine (5.2 g) obtained in Preparative Example 4 (2) was treated in the same manner as in Preparative Example 1 (3) to give 3.7 g of the object compound as a pale-yellow oil.
NMR(CDCl₃)δ : 1.61-1.65(2H,m), 1.90-2.05(2H,m), 2.16(3H,s), 2.19-2.30(2H,m), 2.76-2.80(2H,m), 3.55(2H,s), 3.63(2H,s), 3.68(2H,s), 7.20-7.34(10H,m)

### Preparative Example 5

### (1) 1-Benzoyl-4-piperidone

4-Piperidone hydrochloride (50 g) was dissolved in water (600 ml), and potassium carbonate(135 g) was added. Benzoyl chloride (50 ml) was dropwise added with stirring under ice-cooling. The mixture was stirred at room temperature for 2 hours and the crystals precipitated during the reaction were collected by filtration. After washing with methanol, the crystals were dried to give 53 g of the object compound as colorless crystals.
NMR(CDCl₃)δ : 2.3-2.8(4H,m), 3.6-4.2(4H,m), 7.4-7.6(5H,m)

### (2) 4-Benzamido-1-benzoyl-4-cyanopiperidine

1-Benzoyl-4-piperidone (55 g) obtained in Preparative Example 5 (1) was treated in the same manner as in Preparative Example 1 (1) to give 71 g of the object compound as colorless crystals.
NMR(DMSO) δ : 2.0-2.2(2H,m), 2.3-2.45(2H,m), 3.3-3.5(2H,m), 3.5-3.7(1H,m), 4.0-4.2(1H,m), 7.4-7.65(8H,m), 7.85-7.95(2H,m)

### (3) 4-Benzamido-1-benzoyl-4-ethoxycarbonylpiperidine

4-Benzamido-1-benzoyl-4-cyanopiperidine (71 g) obtained in Preparative Example 5 (2) was treated in the same manner as in Preparative Example 1 (2) to give 44 g of the object compound as a pale-yellow oil.
NMR(CDCl₃)δ : 1.25(3H,t,J=7.5Hz), 2.1-2.35(4H,m), 3.3-3.8(4H,m), 4.25(2H,q,J=7.5Hz), 6.48(1H,s), 7.35-7.6(6H,m), 7.75-7.8(2H,m), 8.0-8.1(2H,m)

### (4) 4-Benzamido-1-benzoyl-4-hydroxymethylpiperidine

4-Benzamido-1-benzoyl-4-ethoxycarbonylpiperidine (42 g) obtained in Preparative Example 5 (3) was dissolved in tetrahydrofuran (450 ml) and sodium borohydride (21 g) was added. The mixture was refluxed under heating overnight. Water (200 ml) was added and the mixture was extracted with chloroform. The extract was washed with water and brine in order and dried over magnesium sulfate. The solvent was distilled away and the residue was purified by silica gel column chromatography to give 30.5 g of the object compound as a pale-yellow oil.
NMR(CDCl₃)δ : 1.8-1.95(2H,m), 2.05-2.2(2H,m), 3.3-3.7(4H,m), 3.85(2H,s), 6.15(1H,s), 7.4-7.6(8H,m), 7.7-7.8(2H,m)

### (5) 4-Benzamido-1-benzoyl-4-methoxymethylpiperidine

4-Benzamido-1-benzoyl-4-hydroxymethylpiperidine (16 g) obtained in Preparative Example 5 (4) was dissolved in tetrahydrofuran (80 ml) and sodium hydride (2.8 g) was added under ice-cooling. The mixture was stirred at room temperature for 30 minutes. The mixture was ice-cooled again and methyl iodide (3.5 ml) was dropwise added. The mixuture was stirred at room temperature for 2 hours. Water was added, and the mixture was extracted with chloroform. The extract was washed with brine and dried over magnesium sulfate. The solvent was distilled away and the residue was purified by silica gel column chromatography to give 5.7 g of the object compound as a pale-yellow oil.
NMR(CDCl₃)δ : 1.6-1.9(2H,m), 2.35-2.5(2H,m), 3.25-3.4(5H,m), 3.4-3.7(3H,m), 4.3-4.4(1H,m), 6.3(1H,s), 7.35-7.55(8H,m), 7.7-7.8(2H,m)

### (6) 1-Benzyl-4-benzylamino-4-methoxymethylpiperidine

4-Benzamido-1-benzoyl-4-methoxymethylpiperidine (2 g) obtained according to Preparative Example 5 (5) was dissolved in tetrahydrofuran (120 ml), and lithium aluminum hydride (540 mg) was added under ice-cooling. The mixture was refluxed under heating for 6 hours. Water (540 µl), a 15% aqueous solution (540 µl) of sodium hydroxide and water (1.6 ml) were sequentially added under ice-cooling and the resulting precipitate was filtered off. The filtrate was dried over magnesium sulfate. The solvent was distilled away and the residue was purified by silica gel column chromatography to give 1.3 g of the object compound as a colorless oil.
NMR(CDCl₃)δ : 1.6-1.8(2H,m), 1.85-1.95(2H,m), 2.4-2.55(2H,m), 2.55-2.7(2H,m), 3.35(3H,s), 3.4(2H,s), 3.6(2H,s), 3.75(2H,s), 7.2-7.5(10H,m)

### Preparative Example 6

### (1) 1-Benzoyl-4-methoxymethyl-4-(N-methyl)benzamidopiperidine

4-Benzamido-1-benzoyl-4-hydroxymethylpiperidine (6.2 g) obtained in Preparative Example 5 (4) was dissolved in tetrahydrofuran (300 ml), and sodium hydride (2.2 g) was added under ice-cooling. The mixture was stirred at room temperature for 50 minutes. The mixture was ice-cooled again and methyl iodide (2.7 ml) was dropwise added. The mixture was stirred at room temperature overnight. Water was added and the mixture was extracted with chloroform. The extract was washed with water and brine in order and dried over magnesium sulfate. The solvent was distilled away and the residue was purified by silica gel column chromatography to give 3.2 g of the object compound as a pale-yellow colorless oil.
NMR(CDCl₃)δ : 1.7-2.1(2H,m), 2.6-2.7(2H,m), 3.0(3H,s), 3.25-3.4(5H,m), 3.55-3.7(1H,m), 3.75-3.9(2H,m), 4.3-4.45(1H,m), 7.35-7.55(10H,m)

### (2) 1-Benzyl-4-methoxymethyl-4-(N-benzyl-N-methyl)aminopiperidine

1-Benzoyl-4-methoxymethyl-4-(N-methyl)benzamidopiperidine (4.2 g) obtained in Preparative Example 6 (1) was treated in the same manner as in Preparative Example 5 (6) to give 1.3 g of the object compound as a colorless oil.
NMR(CDCl₃)δ : 1.6-1.8(2H,m), 1.8-2.0(2H,m), 2.15(3H,m), 2.4-2.55(2H,m), 2.55-2.7(2H,m), 3.35(3H,s), 3.45(2H,s), 3.6(2H,s), 3.75(2H,s), 7.2-7.5(10H,m)

### Preparative Example 7

### (1) 1-Benzyl-4-benzyliminopiperidine

1-Benzyl-4-piperidone (2.0 g) was dissolved in dry benzene (11.5 ml), and benzylamine (1.15 ml) was dropwise added at room temperature. One hour later, the mixture was dried over anhydrous sodium sulfate and concentrated. Dry benzene was added to the residue and the mixture was concentrated. These steps were repeated once to give 2.94 g of the object compound as a brown oil.
NMR(CDCl₃)δ : 2.44-2.73(8H,m), 3.57(2H,s), 4.53(2H,s), 7.22-7.35(10H,m)

### (2) 1-Benzyl-4-benzylamino-4-ethoxycarbonylmethylpiperidine

A mixture of ethyl acetate (1.24 ml) and tetrahydrofuran (24.8 ml) was stirred at -78°C, during which lithium diisopropylamide (1.5 M cyclohexane solution, 9.84 ml) was dropwise added. 1-Benzyl-4-benzyliminopiperidine (2.94 g) obtained in Preparative Example 7 (1) was dissolved in tetrahydrofuran (14.7 ml) and the mixture was stirred at -78°C, during which boron trifluoride ether complex (1.3 ml) was dropwise added. The solution was dropwise added to the previously prepared anion solution at -78°C and the mixture was slowly heated to -30°C. A saturated aqueous solution (50 ml) of ammonium chloride was added and ethyl acetate was added for extraction. The organic layer was dried and the residue obtained by concentration was purified by silica gel column chromatography to give 1.53 g of the object compound as a brown oil.
NMR(CDCl₃)δ : 1.23(3H,t,J=7.1Hz), 1.60-1.78(2H,m), 2.51-2.52(6H,m), 3.53(2H,s), 3.67(2H,s), 4.12(2H,q,J=7.1Hz), 7.24-7.40(10H,m)

### (3) 1-Benzyl-4-benzylamino-4-(2-hydroxyethyl)piperidine

1-Benzyl-4-benzylamino-4-ethoxycarbonylmethylpiperidine (1.5 g) obtained in Preparative Example 7 (2) was treated in the same manner as in Preparative Example 1 (3) to give 1.23 g of the object compound as a brown oil.
NMR(CDCl₃)δ : 1.67-1.89(6H,m), 2.34-2.4(2H,m), 2.50-2.60(2H,m), 3.51(2H,s), 3.72(2H,s), 3.88(2H,m), 7.28-7.32(10H,m)

### Preparative Example 8

### (1) 1-Benzyl-4-benzylamino-4-cyanopiperidine

1-Benzylamine (173 ml) and conc. hydrochloric acid (88 ml) were mixed under ice-cooling to give a solution of corresponding hydrochloride and a solution of 1-benzyl-4-piperidone (100 g) in methanol (300 ml) was dropwise added. Then, an aqueous solution (142.5 ml) of sodium cyanide (28.5 g) was added and the mixture was stirred with heating at 80°C. Twelve hours later, the resultant precipitate was collected by filtration to give 157 g of the object compound as pale-yellow amorphous.
NMR(CDCl₃)δ : 1.78-1.88(2H,m), 2.02-2.07(2H,m), 2.35-2.44(2H,m), 2.80-2.90(2H,m), 3.54(2H,s), 3.90(2H,s), 7.25-7.35(10H,m)

### (2) 1-Benzyl-4-(N-benzyl)benzamido-4-cyanopiperidine

1-Benzyl-4-benzylamino-4-cyanopiperidine (30 g) obtained in Preparative Example 8 (1) was treated in the same manner as in Preparative Example 1 (1), (b) to give 13.68 g of the object compound as white amorphous.
NMR(CDCl₃)δ : 1.98-2.08(2H,m), 2.42-2.54(4H,m), 2.85-2.90(2H,m), 3.51(2H,s), 4.70(2H,s), 7.20-7.36(13H,m), 7.45-7.47(2H,m)

### (3) 1-Benzyl-4-(N-benzyl)benzamido-4-ethoxycarbonylpiperidine

1-Benzyl-4-(N-benzyl)benzamido-4-cyanopiperidine (23.8 g) obtained in Preparative Example 8 (2) was treated in the same manner as in Preparative Example 1 (2) to give 15.1 g of the object compound as a pale-yellow oil.
NMR(CDCl₃)δ : 1.34(3H,t,J=7.0Hz), 1.78-1.90(2H,m), 2.30-2.37(2H,m), 2.42-2.52(2H,m), 2.69-2.76(2H,m), 3.50(2H,s), 4.27(2H,q,J=7.0Hz), 4.65(2H,s), 7.25-7.37(15H,m)

### (4) 1-Benzyl-4-dibenzylamino-4-hydroxymethylpiperidine

1-Benzyl-4-(N-benzyl)benzamido-4-ethoxycarbonylpiperidine (15.1 g) obtained in Preparative Example 8 (3) was treated in the same manner as in Preparative Example 1 (3) to give 13.1 g of the object compound as a pale-yellow oil.
NMR(CDCl₃)δ : 1.47-1.51(2H,m), 1.85-1.96(2H,m), 2.03-2.12(2H,m), 2.71-2.75(2H,m), 3.48(2H,s), 3.71(2H,s), 3.76(2H,s), 7.10-7.30(15H,m)

### (5) 1-Benzyl-4-dibenzylamino-4-fluoromethylpiperidine

1-Benzyl-4-dibenzylamino-4-hydroxymethylpiperidine (7.8 g) obtained in Preparative Example 8 (4) was dissolved in dichloromethane (156 ml) and stirred at -78°C, during which diethylaminosulfur trifluoride (2.83 ml) was dropwise added. The mixture was stirred at room temperature for 3 hours and water (100 ml) was added for partition. The organic layer was dried over magnesium sulfate and concentrated. The residue obtained was purified by silica gel column chromatography to give 2.7 g of the object compound as white amorphous.
NMR(CDCl₃)δ : 1.39-1.60(2H,m), 1.90-2.00(2H,m), 2.23-2.30(2H,m), 2.47-2.52(2H,m), 2.56(2H,d,J=23.6Hz), 3.44(2H,s), 3.63(2H,s), 7.24-7.35(15H,m)

### Preparative Example 9

### 1-Benzyl-4-fluoromethyl-4-(N-methyl)benzylaminopiperidine

1-Benzyl-4-hydroxymethyl-4-(N-methyl)benzylaminopiperidine (962 mg) obtained in Preparative Example 4 (3) was treated in the same manner as in Preparative Example 8 (5) to give 360 mg of the object compound as a pale-yellow oil.
NMR(CDCl₃)δ : 1.66-1.69(2H,m), 1.89-1.95(2H,t), 2.27-3.35(5H,m), 2.45(1H,s), 2.52(1H,s), 2.52-2.59(2H,m), 3.48(2H,s), 3.54(2H,s), 7.22-7.30(10H,m)

### Preparative Example 10

### (1) 3-Benzamido-1-benzyl-3-cyanopiperidine

1-Benzyl-3-piperidone was treated in the same manner as in Preparative Example 1 (1) to give the object compound.

### (2) 3-Benzamido-1-benzyl-3-ethoxycarbonylpiperidine

3-Benzamido-1-benzyl-3-cyanopiperidine obtained in Preparative Example 10 (1) was treated in the same manner as in Preparative Example 1 (2) to give the object compound.

### (3) 1-Benzyl-3-benzylamino-3-hydroxymethylpiperidine

3-Benzamido-1-benzyl-3-ethoxycarbonylpiperidine obtained in Preparative Example 10 (2) was treated in the same manner as in Preparative Example 1 (3) to give the object compound.

### Preparative Example 11

### (1) 1-Benzyl-3-(N-benzyl)benzamido-3-cyanopyrrolidine

1-Benzyl-3-pyrrolidone (33.7 g) was treated in the same manner as in Preparative Example 8 (1) and Preparative Example 8 (2) to give 53.4 g of the object compound as colorless crystals, yield 70%.
NMR(CDCl₃)δ : 2.4-2.5(1H,m), 2.55-2.7(1H,m), 2.8-3.0(3H,m), 3.15(1H,d,J=10Hz), 3.5(1H,d,J=12Hz), 3.75(1H,d,J=12Hz), 4.65-4.75(2H,m), 7.0-7.1(2H,m), 7.1-7.6(13H,m)

### (2) 1-Benzyl-3-(N-benzyl)benzamido-3-ethoxycarbonylpyrrolidine

1-Benzyl-3-(N-benzyl)benzamido-3-cyanopyrrolidine (12 g) obtained in Preparative Example 11 (1) was treated in the same manner as in Preparative Example 1 (2) to give 7.4 g of the object compound as a pale-yellow oil, yield 55%.
NMR(CDCl₃)δ : 1.33(3H,t,J=9Hz), 2.05-2.2(1H,m), 2.3-2.45(1H,m), 2.6(1H,d,J=11Hz), 2.9-3.1(2H,m), 3.15(1H,d,J=11Hz), 3.45(1H,d, J=13Hz), 3.65(1H,d,J=13Hz), 4.25(2H,q,J=9Hz), 4.7(1H,d,J=20Hz), 4.9(1H,d,J=20Hz), 7.2-7.5(15H,m)

### (3) 1-Benzyl-3-dibenzylamino-3-hydroxymethylpyrrolidine

1-Benzyl-3-(N-benzyl)benzamido-3-ethoxycarbonylpyrrolidine (147.8 g) obtained in Preparative Example 11 (2) was treated in the same manner as in Preparative Example 1 (3) to give 106 g of the object compound as a colorless oil, yield 82.1%.
NMR(CDCl₃)δ : 1.90-2.0(1H,m), 2.05-2.2(1H,m), 2.5-2.6(1H,m), 2.55(1H,d,J=10Hz), 2.7(1H,d,J=10Hz), 2.75-2.85(1H,m), 3.5-3.6 (2H,m), 3.6-3.9(6H,m), 7.1-7.4(15H,m)

### (4) 1-Benzyl-3-dibenzylamino-3-fluoromethylpyrrolidine

1-Benzyl-3-dibenzylamino-3-hydroxymethylpyrrolidine (51.2 g) obtained in Preparative Example 11 (3) was treated in the same manner as in Preparative Example 8 (5) to give 42.2 g of the object compound as colorless crystals, yield 82%.
NMR(CDCl₃)δ : 1.8-2.2(2H,m), 2.35-2.45(1H,m), 2.5-2.9(5H,m), 3.55(2H,s), 3.65(4H,q,J=13Hz), 7.2-7.4(15H,m)

### (5) 3-Amino-3-fluoromethylpyrrolidine

Hydrazine hydrate (5 ml) was added to a mixture of 1-benzyl-3-dibenzylamino-3-fluoromethylpyrrolidine (10 g) obtained in Preparative Example 11 (4), 10% palladium-carbon (3 g) and ethanol (200 ml), and the mixture was stirred at room temperature for 0.5 hour. Then, the mixture was refluxed for 3.5 hours and an insoluble matter was filtered off. The filtrate was concentrated and the residue was diluted with chloroform, dried over magnesium sulfate and concentrated under reduced pressure to give 2.634 g of the object compound as a pale-yellow oil.
NMR(CDCl₃)δ : 1.6-1.9(1H,m), 1.95-2.2(1H,m), 2.75-3.00(4H,m), 3.17-3.25(2H,m)

### Preparative Example 12

### (1) 1-Benzyl-3-(N-methyl)benzamido-3-cyanopyrrolidine

1-Benzyl-3-pyrrolidone (10.471 g) was treated in the same manner as in Preparative Example 4 (1) to give 14.364 g of the object compound as colorless scale-like crystals.
NMR(CDCl₃)δ : 2.26-2.38(1H,m), 2.62-2.75(1H,m), 2.80-2.95(3H,m), 3.05(3H,s), 3.48(1H,d,J=7.8Hz), 3.71(2H,s), 7.22-7.57(10H,m)

### (2) 1-Benzyl-3-(N-methyl)benzamido-3-ethoxycarbonylpyrrolidine

1-Benzyl-3-(N-methyl)benzamido-3-cyanopyrrolidine (12.776 g) obtained in Preparative Example 12 (1) was treated in the same manner as in Preparative Example 1 (2) to give 6.793 g of the object compound as a red-yellow oil.
NMR(CDCl₃)δ : 1.24(3H,t,J=7.7Hz), 2.09-2.20(1H,m), 2.48(1H,q,J=7.7Hz), 2.70(1H,d,J=8.4Hz), 2.89-3.06(2H,m), 3.09(3H,s), 3.20(1H,d,J=8.4Hz), 3.58(1H,d,J=10Hz), 3.74(1H,d,J=10.0Hz), 4.18(2H,q,J=7.7Hz), 7.19-7.48(10H,m)

### (3) 1-Benzyl-3-(N-benzyl-N-methyl)amino-3-hydroxymethylpyrrolidine

1-Benzyl-3-(N-methyl)benzamido-3-ethoxycarbonylpyrrolidine (8.429 g) obtained in Preparative Example 12 (2) was treated in the same manner as in Preparative Example 1 (3) to give 6.162 g of the object compound as a pale-yellow oil.
NMR(CDCl₃)δ : 1.88-1.97(1H,m), 2.00-2.12(1H,m), 2.15(3H,s), 2.53-2.69(2H,m), 2.82-2.86(2H,m), 3.48-3.73(5H,m), 3.86(1H,d,J=8.5Hz), 7.22-7.31(10H,m)

### (4) 1-Benzyl-3-(N-benzyl-N-methyl)amino-3-fluoromethylpyrrolidine

1-Benzyl-3-(N-benzyl-N-methyl)amino-3-hydroxymethylpyrrolidine (4.726 g) obtained in Preparative Example 12 (3) was treated in the same manner as in Preparative Example 8 (5) to give 3.559 g of the object compound as a pale-yellow oil.
NMR(CDCl₃)δ : 1.83-2.24(2H,m), 2.29(3H,s), 2.47-2.84(6H,m), 3.56(2H,d,J=3.9Hz), 3.61(2H,s), 7.16-7.33(10H,m)

### Preparative Example 13

### (1) 1-Benzyl-3-cyano-3-methylaminopiperidine

1-Benzyl-3-piperidone (23.4 g) and methylamine hydrochloride (12.5 g) were treated in the same manner as in Preparative Example 8 (1) to give 23 g of the object compound as an oil, which was used in the next step without purification.

### (2) 1-Benzyl-3-cyano-3-(N-methyl)benzamidopiperidine

1-Benzyl-3-cyano-3-methylaminopiperidine (23 g) obtained in (1) was treated in the same manner as in Preparative Example 1 (1), (b) to give 32 g of the object compound as an oil.
NMR(CDCl₃)δ:1.50-3.60(8H,m), 3.00(3H,s), 3.64(2H,s), 7.00-7.60(10H,m)

### (3) 1-Benzyl-3-ethoxycarbonyl-3-(N-methyl)benzamidopiperidine

1-Benzyl-3-cyano-3-(N-methyl)benzamidopiperidine (32 g) obtained in (2) was treated in the same manner as in Preparative Example 1 (2) to give 15.9 g of the object compound as an oil.
NMR(CDCl₃)δ :1.28(8H,t), 1.50-2.90(8H,m), 3.16(3H,s), 3.50 (2H,s), 4.18(2H,q), 7.28(5H,s), 7.40(5H,s)

### (4) 1-Benzyl-3-(N-benzyl-N-methyl)amino-3-hydroxymethylpiperidine

1-Benzyl-3-ethoxycarbonyl-3-(N-methyl)benzamidopiperidine (15.8 g) obtained in (3) was treated in the same manner as in Preparative Example 1 (3) to give 13.3 g of the object compound as an oil.
NMR(CDCl₃)δ :1.46-2.36(9H,m), 2.50-3.00(2H,m), 3.50(2H,s), 3.62(2H,d), 3.82(2H,d), 7.28(10H,s)

### (5) 1-Benzyl-3-(N-benzyl-N-methyl)amino-3-fluoromethylpiperidine

1-Benzyl-3-(N-benzyl-N-methyl)amino-3-hydroxymethylpiperidine (10 g) obtained in (4) and hexafluoropropene diethylamine (13.4 g) were dissolved in chloroform (100 ml), and the mixture was refluxed under heating for 2 hours. After cooling, the reaction mixture was washed with a 10% aqueous solution of potassium carbonate and water in order and dried over magnesium sulfate. Chloroform was distilled away and the residue was purified by silica gel column chromatography to give 7.3 g of the object compound as an oil.
NMR(CDCl₃)δ : 1.51-1.82(4H,m), 2.28(3H,s), 2.30-3.00(6H,m), 3.54(2H,s), 3.56(2H,s), 7.28(10H,s)

### Preparative Example 14

### (1) 1-Benzyl-4-cyano-4-cyclopropylaminopiperidine

1-Benzyl-4-piperidone (75 g) and cyclopropylamine hydrochloride (46 g) were treated in the same manner as in Preparative Example 8 (1) to give 98 g of the object compound as crystals, melting point 64-67°C.

### (2) 1-Benzyl-4-cyano-4-(N-cyclopropyl)benzamidopiperidine

1-Benzyl-4-cyano-4-cyclopropylaminopiperidine (48 g) obtained in (1) was treated in the same manner as in Preparative Example 1 (1), (b) to give 47.5 g of the object compound as crystals, melting point 250-255°C.

### (3) 1-Benzyl-4-(N-cyclopropyl)benzamido-4-ethoxycarbonylpiperidine

1-Benzyl-4-cyano-4-(N-cyclopropyl)benzamidopiperidine (47.5 g) obtained in (2) was treated in the same manner as in Preparative Example 1 (2) to give 22 g of the object compound as a viscous oil.
NMR(CDCl₃)δ :0.50-0.78(4H,m), 1.27(3H,t,J=8.0Hz), 2.30-2.52 (4H,m), 2.52-2.80(4H,m), 3.58(2H,s), 4.19(2H,q,J=8.0Hz), 4.20-4.50(1H,m), 7.20-7.60(10H,m)

### (4) 1-Benzyl-4-(N-benzyl-N-cyclopropyl)amino-4-hydroxymethylpiperidine

1-Benzyl-4-(N-cyclopropyl)benzamido-4-ethoxycarbonylpiperidine (16 g) obtained in (3) was treated in the same manner as in Preparative Example 1 (3) to give 9.4 g of the object compound as crystals, melting point 75-77°C.

### (5) 1-Benzyl-4-(N-benzyl-N-cyclopropyl)amino-4-fluoromethylpiperidine

1-Benzyl-4-(N-benzyl-N-cyclopropyl)amino-4-hydroxymethylpiperidine (10.6 g) obtained in (4) and hexafluoropropene diethylamine (13.2 g) were dissolved in chloroform (100 ml) and the mixture was refluxed under heating for 2 hours. After cooling, the reaction mixture was washed with a 10% aqueous solution of potassium carbonate and water in order and dried over anhydrous magnesium sulfate. Chloroform was distilled away and the residue was purified by silica gel column chromatography to give 8.0 g of the object compound as an oil.
NMR(CDCl₃)δ:0.25-0.35(4H,m), 1.10-1.60(2H,m), 1.70-2.00(4H,m), 2.10-2.70(3H,m), 2.74(2H,d,J=24Hz), 3.52(2H,s), 3.85(2H,s), 7.28(10H,s)

### Preparative Example 15

### 1-Benzyl-3-dibenzylamino-3-chloromethylpyrrolidine

1-Dibenzyl-3-dibenzylamino-3-hydroxymethylpyrrolidine (5.0 g) obtained in Preparative Example 11 (3) was dissolved in pyridine (25.0 ml) and thionyl chloride (1.23 ml) was dropwise added under ice-cooling. The reaction mixture was heated to room temperature and stirred for 1.5 hours. Water (25 ml) was added and the mixture was neutralized with potassium carbonate. Ethyl acetate was added for partitioning and the organic layer was dried over sodium sulfate. The solvent was distilled away and the residue was purified by silica gel column chromatography to give 2.3 g of the object compound as pale-yellow amorphous.
NMR(CDCl₃)δ :2.10(2H,t,J=7.5Hz), 2.46-2.54(1H,m), 2.69-2.82 (3H,m), 2.96(2H,s), 3.50(1H,d,J=12Hz), 3.60(1H,d,J=12Hz), 3.74(4H,s), 7.23-7.32(15H,m)

### Preparative Example 16

### 1-Benzyl-3-(N-benzyl-N-methyl)amino-3-chloromethylbenzylaminopyrrolidine

1-Benzyl-3-(N-benzyl-N-methyl)amino-3-hydroxymethylpyrrolidine (3.104 g) obtained in Preparative Example 12 (3) was dissolved in pyridine (30 ml), and phosphorus oxychloride (1.2 ml) was slowly added at 0°C. The mixture was stirred at room temperature for 4 hours, and the reaction mixture was poured into ice water (50 ml) and extracted with ethyl acetate. The ethyl acetate layer was washed with saturated brine, dried over magnesium sulfate and concentrated under reduced pressure. The residue was purified by silica gel column chromatography to give 2.075 g of the object compound as a pale-yellow oil.
NMR(CDCl₃)δ:1.85-2.22(2H,m), 2.31(3H,s), 2.35-2.79(6H,m), 3.55(2H,d,J=4.2Hz), 3.78(2H,s), 7.18-7.33(10H,m)

### Preparative Example 17

### (1) 1-Benzyl-3-benzylamino-3-cyanopiperidine

1-Benzyl-3-piperidone is treated in the same manner as in Preparative Example 8 (1) to give the object compound.

### (2) 1-Benzyl-3-(N-benzyl)benzamido-3-cyanopiperidine

1-Benzyl-3-benzylamino-3-cyanopiperidine obtained in Preparative Example 17 (1) is treated in the same manner as in Preparative Example 1 (1), (b) to give the object compound.

### (3) 1-Benzyl-3-(N-benzyl)benzamido-3-ethoxycarbonylpiperidine

1-Benzyl-3-(N-benzyl)benzamido-3-cyanopiperidine obtained in Preparative Example 17 (2) is treated in the same manner as in Preparative Example 1 (2) to give the object compound.

### (4) 1-Benzyl-3-dibenzylamino-3-hydroxymethylpiperidine

1-Benzyl-3-(N-benzyl)benzamido-3-ethoxycarbonylpiperidine obtained in Preparative Example 17 (3) is treated in the same manner as in Preparative Example 1 (3) to give the object compound.

### (5) 1-Benzyl-3-dibenzylamino-3-fluoromethylpiperidine

1-Benzyl-3-dibenzylamino-3-hydroxymethylpiperidine obtained in Preparative Example 17 (4) is treated in the same manner as in Preparative Example 8 (5) to give the object compound.

### Preparative Example 18

### (1) 1-Benzyl-3-benzyliminopyrrolidine

1-Benzyl-3-pyrrolidone is treated in the same manner as in Preparative Example 7 (1) to give the object compound.

### (2) 1-Benzyl-3-benzylamino-3-ethoxycarbonylmethylpyrrolidine

1-Benzyl-3-benzyliminopyrrolidine obtained in Preparative Example 18 (1) is treated in the same manner as in Preparative Example 7 (2) to give the object compound.

### (3) 1-Benzyl-3-(N-benzyl)benzamido-3-ethoxycarbonylmethylpyrrolidine

1-Benzyl-3-benzylamino-3-ethoxycarbonylmethylpyrrolidine obtained in Preparative Example 18 (2) is benzoylated to give the object compound.

### (4) 1-Benzyl-3-dibenzylamino-3-hydroxyethylpyrrolidine

1-Benzyl-3-(N-benzyl)benzamido-3-ethoxycarbonylmethylpyrrolidine obtained in Preparative Example 18 (3) is treated in the same manner as in Preparative Example 1 (3) to give the object compound.

### (5) 1-Benzyl-3-dibenzylamino-3-fluoroethylpyrrolidine

1-Benzyl-3-dibenzylamino-3-hydroxyethylpyrrolidine obtained in Preparative Example 18 (4) is treated in the same manner as in Preparative Example 8 (5) to give the object compound.

### (6) 3-amino-3-fluoroethylpyrrolidine

1-Benzyl-3-dibenzylamino-3-fluoroethylpyrrolidine obtained in Preparative Example 18 (5) is treated in the same manner as in Preparative Example 11 (5) to give the object compound.

### Preparative Example 19

### (1) 4-Aminomethyl-1-benzyl-4-benzylaminopiperidine

Lithium aluminum hydride (15.1 g) was suspended in tetrahydrofuran (1000 ml) and a solution of 4-benzamido-1-benzyl-4-cyanopiperidine (57.7 g) obtained in Preparative Example 1 (1), in tetrahydrofuran (500 ml), was dropwise added with stirring under ice-cooling. The mixture was refluxed under heating overnight, and a small amount of water and a 15% aqueous solution of sodium hydroxide were added. The resulting insoluble matter was filtered off and the filtrate was dried over magnesium sulfate. The solvent was distilled away to give 34.4 g of the object compound as a pale-yellow oil.
NMR(CDCl₃)δ:1.45-1.75(4H,m), 2.35-2.45(2H,m), 2.55-2.65(2H,m), 2.69(2H,s), 3.50(2H,s), 3.60(2H, s), 7.23-7.34(10H,m)

### (2) 1-Benzyl-4-benzylamino-4-(t-butoxycarbonylaminomethyl)piperidine

4-Aminomethyl-1-benzyl-4-benzylaminopiperidine (7.0 g) obtained in Preparative Example 19 (1) and triethylamine (3.66 g) were dissolved in dichloromethane (200 ml) and di-t-butyl dicarbonate (7.42 g) was added. The mixture was stirred at room temperature for 15 hours and water was added for partitioning. The organic layer was dried over magnesium sulfate. The solvent was distilled away and the residue was purified by silica gel column chromatography to give 4.63 g of the object compound as a pale-yellow oil.
NMR(CDCl₃)δ:1.43(9H,s), 1.56-1.58(4H,m), 2.30-2.50(4H,m), 3.10-3.20(2H,m), 3.46(2H,s), 3.57(2H,s), 5.00-5.10(1H,t), 7.10-7.30(10H,m)

### Preparative Example 20

### (1) 3,3-diethoxycarbonyl-1-((R)-α-methylbenzyl)-5-pyrrolidone

(R)-(+)-α-Methylbenzylamine (363.5 g) was dissolved in toluene (300 ml) and 37% formaldehyde (267.9 g) was dropwise added slowly with stirring under ice-cooling. The mixture was stirred at 35-40°C for 2 hours and a 25% aqueous solution of sodium hydroxide (9 ml) was added at said temperature. The mixture was further stirred for one hour. The reaction mixture was partitioned, and the organic layer was washed with saturated brine, dried over magnesium sulfate and concentrated under reduced pressure to give 442.0 g of an oil.

Trifluoroacetic acid (12.3 ml) was added to a mixture of the above-mentioned oil (353.3 g) and triethyl 1,1,2-ethanetricarboxylate (195.7 g), and the mixture was stirred at 100°C for 20 hours. After cooling, the reaction mixture was diluted with toluene (3 ℓ), and washed with 10% hydrochloric acid, water, a saturated aqueous solution of sodium bicarbonate and saturated brine in order. The organic layer was dried over magnesium sulfate and concentrated under reduced pressure to give 254.0 g of the object compound as an oil.
NMR(CDCl₃)δ :1.16(3H, t, J=7.1Hz), 1.26(3H, t, J=7.1Hz), 1.54(3H, d, J=7.1Hz), 2.96(1H, d, J=17.3Hz), 3.08(1H, d, J=17.3Hz), 3.36(1H, d, J=10.5Hz), 3.82(1H,d,J=10.5Hz), 4.06-4.15(2H, m), 4.23(2H, q, J=7.1Hz), 5.48(1H, q, J=7.1), 7.25-7.36(5H, m)

### (2) (R)-3-Carboxy-3-ethoxycarbonyl-1-((R)-α-methylbenzyl)-5-pyrrolidone

3,3-Diethoxycarbonyl-1-((R)-α-methylbenzyl)-5-pyrrolidone (138 g) dissolved in acetone (1.02 ℓ) and swine liver esterase (19.5 ml, Sigma) were added to 0.05 M phosphate buffer (pH 8.0, 18.6 ℓ), and the mixture was stirred at room temperature for 15 hours. Acetone was distilled away under reduced pressure and the resulting aqueous layer was washed with ethyl acetate. Conc. hydrochloric acid was added to the aqueous layer to adjust its pH to 2. The aqueous layer was extracted with chloroform. The chloroform layer was dried over magnesium sulfate, and concentrated under reduced pressure. The obtained crude product was recrystallized from heated ethyl acetate (2 ℓ) to give 82.872 g of the object compound as needle crystals.
[α] _{D} = +62.6 ° (c=0.86, chloroform)
NMR(CDCl₃)δ : 1.26(3H, t, J=6.0Hz), 1.54(3H, d, J=9.0Hz), 3.03(1H, d, J=18.0Hz), 3.13(1H, d, J=18.0Hz), 3.41(1H, d, J=12.0Hz), 3.85(1H, d, J=12.0Hz), 4.24(2H, q, J=6.0Hz), 5.46(1H, q, J=7.0Hz), 5.84(1H, br.s), 7.23-7.34(5H, m)

### (3) (S)-3-Benzyloxycarbonylamino-3-ethoxycarbonyl-1-((R)-α-methylbenzyl)-5-pyrrolidone

(R)-3-Carboxy-3-ethoxycarbonyl-1-((R)-α-methylbenzyl)-5-pyrrolidone (792.3 g) was suspended in toluene (1.3 ℓ) and triethylamine (362 ml) was added. Then, diphenylphosphoryl azide (543.9 ml) was dropwise added under ice-cooling. After the dropwise addition, the mixture was stirred at 50°C for 1 hour and then stirred at 80°C for 1 hour. Benzyl alcohol (268.4 ml) was added and the mixture was stirred at 110°C for 1.5 hours. After cooling, the reaction mixture was extracted with toluene, and toluene layer was washed with saturated brine. The toluene layer was dried over magnesium sulfate and concentrated under reduced pressure to give 1328.84 g of the object compound as an oil, which was subjected to the next reaction without purification.
NMR(CDCl₃)δ : 1.25(3H, t, J=7.3Hz), 1.54(3H, d, J=7.1Hz), 2.75(1H, d, J=17.3Hz), 2.92(1H, d, J=17.3Hz), 2.25(1H, d, J=10.2Hz), 3.85(1H, d, J=10.2Hz), 4.21(2H, q, J=7.3Hz), 4.99(1H, d, J=12.3Hz), 5.03(1H, d, J=12.3Hz), 5.44(1H, br.s), 5.51(1H, q, J=7.1Hz), 7.22-7.37(10H, m)

### (4) (S)-3-Amino-3-ethoxycarbonyl-1-((R)-α-methylbenzyl)-5-pyrrolidone

10% Palladium-carbon catalyst (108 g, 10 w/w%) was suspended in ethanol (3 ℓ) and (S)-3-benzyloxycarbonylamino-3-ethoxycarbonyl-1-((R)-α-methylbenzyl)-5-pyrrolidone (1328.84 g) dissolved in ethanol (2.24 ℓ) was added. The mixture was stirred at room temperature for 5 hours in a hydrogen stream. The insoluble matter was filtered off, and the filtrate was concentrated to give 890.6 g of the object compound as a pale-yellow-red oil, which was subjected to the next reaction without purification.
NMR(CDCl₃)δ :1.28(3H, t, J=7.1Hz), 1.52(3H, d, J=7.1Hz), 1.66(2H, br.s), 2.39(1H, d, J=16.7Hz), 2.83(1H, d, J=10.1Hz), 3.00(1H, d, J=16.7Hz), 3.67(1H, d, J=10.1Hz), 4.20(2H, q, J=7.1Hz), 5.52(1H, q, J=7.1Hz), 7.24-7.3(5H, m)

### (5) (S)-3-Benzylamino-3-ethoxycarbonyl-1-((R)-α-methylbenzyl)-5-pyrrolidone

Benzyl bromide (370 ml) was added to a mixture of (S)-3-amino-3-ethoxycarbonyl-1-((R)-α-methylbenzyl)-5-pyrrolidone (890.6 g), acetonitrile (1.3 ℓ) and potassium carbonate (1076 g), and the mixture was refluxed for 1 hour. After cooling, the insoluble matter was filtered off, and the filtrate was concentrated under reduced pressure to give 1067.78 g of the object compound as an oil, which was subjected to the next reaction without purification.
NMR(CDCl₃)δ :1.31(3H, t, J=7.1Hz), 1.55(3H, d, J=7.2Hz), 2.56(1H, d, J=16.7Hz), 2.96(1H, d, J=16.7Hz), 3.05(1H, d, J=10.3Hz), 3.45(1H, d, J=12.2Hz), 3.52(1H, d, J=12.2Hz), 3.71(1H, d, J=10.3 Hz), 4.25(2H, q, J=7.1Hz), 5.54(1H, q, J=7.2Hz), 7.17-7.38(10H, m)

### (6) (S)-3-(N-Benzoyl-N-benzyl)amino-3-ethoxycarbonyl-1-((R)-α-methylbenzyl)-5-pyrrolidone

Benzoyl chloride (302 ml) was dropwise added to a mixture of (S)-3-benzylamino-3-ethoxycarbonyl-1-((R)-α-methylbenzyl)-5-pyrrolidone (1067.8 g), pyridine (1.3 ℓ) and 4-dimethylaminopyridine (63.7 g) under ice-cooling, and the mixture was stirred at 80°C for 2 hours. After cooling, pyridine was distilled away under reduced pressure, and ice water was added to the residue. The mixture was extracted with ethyl acetate. The organic layer was washed with 1N hydrochloric acid, a saturated aqueous solution of sodium bicarbonate and saturated brine in order, dried over magnesium sulfate and concentrated under reduced pressure to give 1242.2 g of the amorphous object compound, which was subjected to the next reaction without purification.
NMR(CDCl₃)δ :1.34(3H, t, J=9.0Hz), 1.60(3H, d, J=9.0Hz), 2.74(1H, d, J=18.0Hz), 3.97(1H, d, J=18.0Hz), 3.24(1H, d, J=12.0Hz), 4.31(2H, q, J=9.0Hz), 4.45(1H, d, J=18.0Hz), 4.46(1H, d, J=18.0Hz), 4.64(1H, d, J=18.0Hz), 5.53(1H, q, J=9.0Hz), 7.13-7.39(15H, m)

### (7) (S)-3-(N,N-Dibenzyl)amino-3-hydroxymethyl-1-((R)-α-methylbenzyl)pyrrolidine

Lithium aluminum hydride (192.0 g) was suspended in dry tetrahydrofuran (2.4 ℓ) in an argon stream and (S)-3-(N-benzoyl-N-benzyl)amino-3-ethoxycarbonyl-1-((R)-α-methylbenzyl)-5-pyrrolidone dissolved in dry tetrahydrofuran (3 ℓ) was dropwise added at -10 to 15°C. After refluxing for 1 hour, the reaction mixture was cooled to -10°C and diethyl ether (3 ℓ) was added. Then, water (192 ml), a 15% aqueous solution of sodium hydroxide (192 ml) and water (576 ml) were sequentially added, and the mixture was stirred at room temperature for 0.5 hour. The insoluble matter was filtered off and the filtrate was dried over magnesium sulfate and concentrated under reduced pressure to give 1065.0 g of the object compound as a pale-yellow oil, which was subjected to the next reaction without purification.
NMR(CDCl₃)δ :1.30(3H, d, J=6.0Hz), 1.91-2.12(2H, m), 2.43-2.51(2H, m), 2.51(1H, s), 2.85-2.92(1H, m), 3.15(1H, q, J=6.0Hz), 3.61(1H, d, J=12.0Hz), 3.65(1H, d, J=15.0Hz), 3.81(1H, d, J=15.0Hz), 3.84(1H, d, J=12.0Hz), 7.10-7.32(15H, m)

### (8) (S)-3-(N,N-Dibenzyl)amino-3-fluoromethyl-1-((R)-α-methylbenzyl)pyrrolidine

Diethylaminosulfur trifluoride (358.8.ml) was dissolved in dichloromethane (7.42 ℓ) and (S)-3-(N,N-dibenzyl)amino-3-hydroxymethyl-1-((R)-α-methylbenzyl)pyrrolidine (1065.0 g) dissolved in dichloromethane (0.74 ℓ) was dropwise added at -20°C in an argon stream. After the dropwise addition, the mixture was stirred at -10°C to 0°C for 2.5 hours, and a 15% aqueous solution of sodium hydroxide (1.335 ℓ) was added at 0°C. The mixture was stirred at room temperature for 0.5 hour. The reaction mixture was washed with saturated brine, dried over calcium chloride and concentrated under reduced pressure to give 112.1 g of the object compound as an oil, which was subjected to the next reaction without purification.
NMR(CDCl₃)δ :1.29(3H, d, J=6.0Hz), 1.76-2.13(2H, m), 2.16-2.23(1H, m), 2.51-2.79(5H, m), 3.16(1H, q, J=6.0Hz), 3.61(1H, d, J=12.0Hz), 3.67(1H, d, J=12.0Hz), 7.19-7.33(15H, m)

### (9) (S)-3-Amino-3-fluoromethylpyrrolidine

7.5% Palladium-carbon (264 g, 30 w/w%) was suspended in ethanol (7 ℓ), and (S)-3-(N,N-dibenzyl)amino-3-fluoromethyl-1-((R)-α-methylbenzyl)pyrrolidine (880 g) dissolved in ethanol (4 ℓ) and hydrazine hydrate (318 ml) were added. The mixture was stirred at room temperature for 0.5 hour and refluxed for 2 hours. After cooling, the insoluble matter was filtered off and the filtrate was concentrated. The residue was diluted with dichloromethane (2 ℓ), dried over magnesium sulfate and concentrated under reduced pressure. The concentrated residue was distilled under reduced pressure to give 124 g of the object compound as a colorless oil, boiling point 83-87°C (7.2-7.5 mmHg).
[α] _{D} = -2.7° (c=1.13, ethanol)
NMR(CDCl₃)δ :1.58(3H, br.s), 1.74-1.91(1H, m), 1.93-2.35(1H, m), 2.81(1H, dd, J=12.9Hz, 30.1Hz), 2.89-3.03(3H, m), 3.15-3.25(2H, m)

### Preparative Example 21

### Optical resolution of 3-fluoromethyl-3-methylaminopyrrolidine

(1) 1-Benzyl-3-(N-benzyl-N-methyl)amino-3-fluoromethylpyrrolidine (3.559 g) obtained in Preparative Example 12, 10% palladium-carbon (2.28 g) and hydrazine hydrate (1.712 g) were treated in the same manner as in Preparative Example 32 to give a debenzylated crude product as a pale-yellow oil (1.162 g).
(2) A mixture of 3-fluoromethyl-3-methylaminopyrrolidine (300 mg), (+)-2,3-dibenzoyltartaric acid (0.854 g) and ethanol was refluxed for 1 hour and left standing at room temperature overnight. The precipitated crystals were collected by filtration and recrystallized 4 times from ethanol to give 0.05 g of (+)-2,3-dibenzoyltartrate of (S)-3-fluoromethyl-3-methylaminopyrrolidine. The optical purity of the obtained product was determined by treating the product with benzoyl chloride in the presence of triethylamine by a conventional method to give a dibenzoyl derivative and subjecting the derivative to liquid chromatography (column: Chiral pack AD, mobile phase : n-hexane/isopropanol = 7/3), which was found to be 91.8% ee.

### Preparative Example 22

### Optical resolution of 3-amino-3-fluoromethylpyrrolidine

A mixture of 3-amino-3-fluoromethylpyrrolidine (12.9 g), (+)-2,3-dibenzoyltartaric acid (41.1 g), water (110 ml) and ethanol (790 ml) was refluxed for 2 hours and left standing at room temperature overnight. The precipitated crystals were collected by filtration and recrystallized 3 times from water - ethanol to give 3.32 g of (+)-2,3-dibenzoyltartrate of (S)-3-amino-3-fluoromethylpyrrolidine. The optical purity of the obtained product was determined in the same manner as in Preparative Example 21, which was found to be 95.9% ee.

### Preparative Example 23

### (S)-1-Benzyl-3-(N-benzoyl-N-benzyl)amino-3-ethoxycarbonyl pyrrolidine and (R)-1-benzyl-3-(N-benzoyl-N-benzyl)amino-3-ethoxycarbonylpyrrolidine

1-Benzyl-3-(N-benzoyl-N-benzyl)amino-3-ethoxycarbonylpyrrolidine (40 g) was separated by liquid chromatography using Chiral pack AD (solvent: hexane/isopropanol = 90/10) to give an earlier fraction (R)-1-benzyl-3-(N-benzoyl-N-benzyl)amino-3-ethoxycarbonylpyrrolidine as colorless crystals (13.0 g), melting point 96.8-97.8°C.
[α] _{D} = -82.4° (c=0.216, chloroform)
Also, a later fraction (S)-1-benzyl-3-(N-benzoyl-N-benzyl)amino-3-ethoxycarbonylpyrrolidine was obtained as colorless crystals (13.0 g), melting point 94.1-95.6°C.
[α] _{D} = +82.1 ° (c=0.98, chloroform)

### Preparative Example 24

### (S)-1-Benzyl-3-(N-benzoyl-N-methyl)amino-3-ethoxycarbonylpyrrolidine and (R)-1-benzyl-3-(N-benzoyl-N-methyl)amino-3-ethoxycarbonylpyrrolidine

1-Benzyl-3-(N-benzoyl-N-methyl)amino-3-ethoxycarbonylpyrrolidine (30 g) was separated by liquid chromatography using Chiral pack AD (solvent: hexane/isopropanol = 90/10) to give an earlier fraction (R)-1-benzyl-3-(N-benzoyl-N-methyl)amino-3-ethoxycarbonylpyrrolidine as a pale-yellow oil (10.4 g).
[α] _{D} = -20.56° (c=1.3, chloroform)
Also, a later fraction (S)-1-benzyl-3-(N-benzoyl-N-methyl)amino-3-ethoxycarbonylpyrrolidine was obtained as a pale-yellow oil (13.8 g).
[α] _{D} = +21.34° (c=2, chloroform)

### Preparative Example 25

### 7-(4-Amino-4-hydroxymethylpiperidin-1-yl)-1-cyclopropyl-6-fluoro-1,4-dihydro-8-methoxy-4-oxo-3-quinolinecarboxylic acid hydrochloride

(1) 1-Benzyl-4-benzylamino-4-hydroxymethylpiperidine (2.0 g) obtained in Preparative Example 1 (3) was dissolved in ethanol (50 ml), and 20% palladium hydroxide-carbon (600 mg) and ammonium formate (2.0 g) were added. The mixture was refluxed under heating for 2 hours. The insoluble matter was filtered off and the filtrate was concentrated to give a deprotected crude amine.
(2) The crude amine and 1-cyclopropyl-6,7-difluoro-1,4-dihydro-8-methoxy-4-oxo-3-quinolinecarboxylic acid-BF₂ chelate (805 mg) obtained by a known method were dissolved in a mixed solvent of acetonitrile - dimethylformamide (1:1, 20 ml), and the mixture was stirred at room temperature for 16 hours. The solvent was distilled away. The residue was dissolved in a mixed solvent of chloroform - methanol (1:1, 100 ml) and triethylamine (2 ml) was added. The mixture was refluxed under heating for 5 hours. The solvent was distilled away and the residue was dissolved in 2N hydrochloric acid. The insoluble matter was filtered off and an aqueous ammonia was added for neutralization. The mixture was concentrated to give 300 mg of the object compound as a pale-yellow solid, melting point 230°C; melting point of the corresponding free base: 217-218°C.

### Preparative Example 26

### 7-(3-Amino-3-hydroxymethylpyrrolidin-1-yl)-1-cyclopropyl-6-fluoro-1,4-dihydro-8-methoxy-4-oxo-3-quinolinecarboxylic acid

1-Benzyl-3-benzylamino-3-hydroxymethylpyrrolidine (2.0 g) obtained in Preparative Example 2 (3) was dissolved in acetic acid (20 ml) and 20% palladium hydroxide-carbon (500 mg) was added. The mixture was stirred at 60°C overnight under a hydrogen atmosphere. The catalyst was filtered off and acetic acid was distilled away to give crude amine residue. The crude amine residue obtained was dissolved in dimethylformamide (20 ml) and 1-cyclopropyl-6,7-difluoro-1,4-dihydro-8-methoxy-4-oxo-3-quinolinecarboxylic acid-BF₂ chelate (771 mg) was added. The mixture was stirred at room temperature overnight. Dimethylformamide was distilled away and methanol (50 ml) and triethylamine (5 ml) were added to the residue. The mixture was refluxed under heating for 4 hours. The solvent was distilled away and the residue was purified by column chromatography using octadecylsilane to give 550 mg of the object compound as a pale-yellow solid, melting point 226-230°C.

### Preparative Example 27

### 7-(3-Amino-3-hydroxymethylazetidin-1-yl)-1-cyclopropyl-6-fluoro-1,4-dihydro-8-methoxy-4-oxo-3-quinolinecarboxylic acid

3-Amino-3-hydroxymethyl-1-diphenylmethylazetidine (1.5 g) obtained in Preparative Example 3 (4) was dissolved in acetic acid (60 ml), and palladium black (0.45 g) was added. The mixture was stirred at 60°C for 2 hours under a hydrogen atmosphere. The catalyst was filtered off and acetic acid was distilled away. The obtained residue was partitioned with water - chloroform to give crude amine (1.1 g). The crude amine (555 mg) and 1-cyclopropyl-6,7-difluoro-1,4-dihydro-8-methoxy-4-oxo-3-quinolinecarboxylic acid-BF₂ chelate (621 mg) were dissolved in a mixed solvent of acetonitrile - dimethylformamide (1:1, 20 ml), and the mixture was stirred at room temperature for 12 hours. The solvent was distilled away and the residue was dissolved in ethanol (30 ml). Triethylamine (5 ml) was added and the mixture was refluxed under heating overnight. The insoluble matter was filtered off and the solvent was distilled away. Ethyl acetate was added to allow precipitation of crystals. The crystals were washed with methanol to give 470 mg of the object compound, melting point 235°C.

### Preparative Example 28

### 1-Cyclopropyl-6-fluoro-1,4-dihydro-7-(4-hydroxymethyl-4-methylaminopiperidin-1-yl)-8-methoxy-4-oxo-3-quinolinecarboxylic acid

1-Benzyl-4-hydroxymethyl-4-(N-benzyl-N-methyl)aminopiperidine (2.6 g) obtained in Preparative Example 4 (3) was treated in the same manner as in Preparative Example 25 to give 50 mg of the object compound (50 mg) as crystals, melting point 240°C.

### Preparative Example 29

### 7-(4-Amino-4-methoxymethylpiperidin-1-yl)-1-cyclopropyl-6-fluoro-1,4-dihydro-8-methoxy-4-oxo-3-quinolinecarboxylic acid

1-Benzyl-4-benzylamino-4-methoxymethylpiperidine (1.3 g) obtained in Preparative Example 5 (6) was treated in the same manner as in Preparative Example 26 to give 240 mg of the object compound as a pale-yellow solid, melting point 181-189°C.

### Preparative Example 30

### 1-Cyclopropyl-6-fluoro-1,4-dihydro-8-methoxy-7-(4-methoxymethyl-4-methylaminopiperidin-1-yl)-4-oxo-3-quinolinecarboxylic acid

1-Benzyl-4-methoxymethyl-4-(N-methyl-N-benzyl)aminopiperidine (1.3 g) obtained in Preparative Example 6 (2) was treated in the same manner as in Preparative Example 26 to give 85 mg of the object compound as a pale-yellow solid, melting point 189-192°C.

### Preparative Example 31

### 7-[4-Amino-4-(2-hydroxyethyl)piperidin-1-yl]-1-cyclopropyl-6-fluoro-1,4-dihydro-8-methoxy-4-oxo-3-quinolinecarboxylic acid

1-Benzyl-4-benzylamino-4-(2-hydroxyethyl)piperidine (1.23 g) obtained in Preparative Example 7 (3) was treated in the same manner as in Preparative Example 25 to give 124 mg of the object compound as crystals, melting point 218°C.

### Preparative Example 32

### 7-(4-Amino-4-fluoromethylpiperidin-1-yl)-1-cyclopropyl-6-fluoro-1,4-dihydro-8-methoxy-4-oxo-3-quinolinecarboxylic acid

1-Benzyl-4-dibenzylamino-4-fluoromethylpiperidine (2.0 g) obtained in Preparative Example 8 (5) was dissolved in ethanol (40 ml) and hydrazine hydrate (1.28 g) and 10% palladium-carbon (1.49 g) were added. The mixture was refluxed under heating for 3 hours. The catalyst was filtered off and the solvent was distilled away. The residue was dissolved in chloroform and dried over magnesium sulfate. The solvent was distilled away to give crude amine (472 mg). The obtained crude amine (472 mg) was treated in the same manner as in Preparative Example 25 (2) to give 400 mg of the object compound as a pale-yellow solid, melting point 191°C.

### Preparative Example 33

### 1-Cyclopropyl-6-fluoro-7-(4-fluoromethyl-4-methylaminopiperidin-1-yl)-1,4-dihydro-8-methoxy-4-oxo-3-quinolinecarboxylic acid

1-Benzyl-4-fluoromethyl-4-(N-methyl-N-benzyl)aminopiperidine (360 mg) obtained in Preparative Example 9 was treated in the same manner as in Preparative Example 25 to give 90 mg of the object compound as a pale-yellow solid, melting point 147-150°C.

### Preparative Example 34

### 1-Cyclopropyl-6-fluoro-1,4-dihydro-7-(3-methylamino-3-fluoromethylpiperidin-1-yl)-8-methoxy-4-oxo-3-quinolinecarboxylic acid

(1) 1-Benzyl-3-(N-benzyl-N-methyl)amino-3-fluoromethylpiperidine (7.2 g) obtained in Preparative Example 13 (5) was dissolved in isopropyl alcohol (140 ml) and 10% palladium-carbon catalyst (3 g) and hydrazine hydrate (4 ml) were added at room temperature. The mixture was refluxed under heating for 30 minutes. After cooling, the mixture was filtered through Celite, and isopropyl alcohol was distilled away to give 3 g of 3-fluoromethyl-3-methylaminopiperidine as an oil.
(2) The obtained oil (3 g), 1-cyclopropyl-6,7-difluoro-1,4-dihydro-8-methoxy-4-oxo-3-quinolinecarboxylic acid-BF₂ chelate (3.5 g), triethylamine (1 g) and acetonitrile (35 ml) were stirred at room temperature for 8 hours and concentrated under reduced pressure to remove the solvent. Methanol (100 ml) and triethylamine (5 ml) were added and the mixture was refluxed for 4 hours. The reaction mixture was concentrated and recrystallized from ethanol to give 3.6 g of the object compound, melting point 193-195°C.

### Preparative Example 35

### 1-Cyclopropyl-7-(4-cyclopropylamino-4-fluoromethylpiperidin-1-yl)-6-fluoro-1,4-dihydro-8-methoxy-4-oxo-3-quinolinecarboxylic acid

(1) 1-Benzyl-4-(N-benzyl-N-cyclopropyl)amino-4-fluoromethylpiperidine (4.0 g) obtained in Preparative Example 14 (5) was dissolved in isopropyl alcohol (40 ml) and 10% palladium-carbon catalyst (4 g), and hydrazine hydrate (3.8 g) were added at room temperature. The mixture was refluxed under heating for 6 hours. After cooling, the mixture was filtered through Celite and isopropyl alcohol was distilled away to give 1.54 g of 4-cyclopropylamino-4-fluoromethylpiperidine as an oil.
(2) The obtained oil (1.54 g), 1-cyclopropyl-6,7-difluoro-1,4-dihydro-8-methoxy-4-oxo-3-quinolinecarboxylic acid-BF₂ chelate (2.56 g), triethylamine (0.9 ml) and acetonitrile (35 ml) were stirred at room temperature for 8 hours and concentrated under reduced pressure to remove the solvent. Methanol (20 ml), water (20 ml) and triethylamine (1 ml) were added and the mixture was refluxed for 5 hours. The reaction mixture was concentrated and crystallized from ethanol. The crystals were collected by filtration and recrystallized from ethanol to give the object compound, melting point 177-179°C.

### Preparative Example 36

### 7-(3-Amino-3-fluoromethylpiperidin-1-yl)-1-cyclopropyl-6-fluoro-1,4-dihydro-8-methoxy-4-oxo-3-quinolinecarboxylic acid

1-Benzyl-3-dibenzylamino-3-fluoromethylpiperidine obtained in Preparative Example 17 (5) was treated in the same manner as in Preparative Example 25 (1). The obtained 3-amino-3-fluoromethylpiperidine (0.38 g), 1-cyclopropyl-6,7-difluoro-1,4-dihydro-8-methoxy-4-oxo-3-quinolinecarboxylic acid-BF₂ chelate (0.39 g) and dimethyl sulfoxide (10 ml) were stirred at room temperature for 8 hours and concentrated under reduced pressure to remove the solvent. Methanol (30 ml), chloroform (30 ml) and triethylamine (5 ml) were added and the mixture was refluxed for 4 hours. The reaction mixture was concentrated and crystallized from ethanol. The crystals were collected by filtration and purified by silica gel chromatography to give the object compound.

### Preparative Example 37

### 1-Cyclopropyl-7-(3-dimethylamino-3-fluoromethylpiperidin-1-yl)-6-fluoro-1,4-dihydro-8-methoxy-4-oxo-3-quinolinecarboxylic acid

1-Cyclopropyl-6-fluoro-1,4-dihydro-7-(3-methylamino-3-fluoromethylpiperidin-1-yl)-8-methoxy-4-oxo-3-quinolinecarboxylic acid (1 g) obtained in Preparative Example 34, 37% formalin (0.58 g) and formic acid (10 ml) were stirred at 100°C for 4 hours. After cooling, the excess formic acid and formalin were distilled away under reduced pressure. The residue was dissolved in water and its pH was adjusted to about 7 with 1N sodium hydroxide. The precipitated crystals were collected by filtration and recrystallized from ethanol to give the object compound, melting point 152-154°C.

### Preparative Example 38

### 7-[3-Amino-3-(2-fluoroethyl)pyrrolidin-1-yl]-1-cyclopropyl-6-fluoro-1,4-dihydro-8-methoxy-4-oxo-3-quinolinecarboxylic acid

A mixture of 3-amino-3-(2-fluoroethyl)pyrrolidine (0.38 g) obtained in Preparative Example 18 (6), 1-cyclopropyl-6,7-difluoro-1,4-dihydro-8-methoxy-4-oxo-3-quinolinecarboxylic acid-BF₂ chelate (0.39 g) and dimethyl sulfoxide (10 ml) was stirred at room temperature for 8 hours and concentrated under reduced pressure to remove the solvent. Methanol (30 ml), chloroform (30 ml) and triethylamine (5 ml) were added and the mixture was refluxed for 4 hours. The reaction mixture was concentrated and crystallized from ethanol. The crystals were collected by filtration and purified by silica gel chromatography to give the object compound.

### Preparative Example 39

### 7-(3-Amino-3-chloromethylpyrrolidin-1-yl)-1-cyclopropyl-6-fluoro-1,4-dihydro-8-methoxy-4-oxo-3-quinolinecarboxylic acid

1-Dibenzyl-3-dibenzylamino-3-chloromethylpyrrolidine (4.0 g) obtained in Preparative Example 15 was dissolved in ethanol (400 ml) and 60% perchloric acid (20 ml) and 20% palladium-carbon catalyst (2.0 g) were added. The mixture was stirred in a hydrogen atmosphere for 2 hours. The mixture was passed through Celite and ethanol was distilled away. The residue was dissolved in a 1N aqueous solution of sodium hydroxide and partitioned with chloroform. The organic layer was dried over sodium sulfate and the solvent was distilled away to give 982 mg of 3-amino-3-chloromethylpyrrolidine as a brown oil.

The obtained 3-amino-3-chloromethylpyrrolidine (982 mg), 1-cyclopropyl-6,7-difluoro-1,4-dihydro-8-methoxy-4-oxo-3-quinolinecarboxylic acid-BF₂ chelate (1.25 g) and triethylamine (0.91 ml) were dissolved in acetonitrile (12.5 ml), and the mixture was stirred at room temperature overnight. Ethanol (12.5 ml) was added and the precipitated crystals were collected by filtration, dried under reduced pressure to give 1.3 g of 7-(3-amino-3-chloromethylpyrrolidin-1-yl)-1-cyclopropyl-6-fluoro-1,4-dihydro-8-methoxy-4-oxo-3-quinolinecarboxylic acid-BF₂ chelate as a yellow powder.

The obtained 7-(3-amino-3-chloromethylpyrrolidin-1-yl)-1-cyclopropyl-6-fluoro-1,4-dihydro-8-methoxy-4-oxo-3-quinolinecarboxylic acid-BF₂ chelate (1.1 g) and triethylamine (1.3 ml) were dissolved in a mixed solvent of chloroform (99 ml) and methanol (11 ml), and the mixture was refluxed for 12 hours. The solvent was distilled away under reduced pressure and the residual solid was washed with ethanol, and recrystallized from methanol and saturated aqueous ammonia to give 454 mg of the pale-yellow, amorphous object compound, melting point 177 °C (decomposition).

### Preparative Example 40

### 7-[3-Chloromethyl-3-(N-methyl)aminopyrrolidin-1-yl]-1-cyclopropyl-6-fluoro-1,4-dihydro-8-methoxy-4-oxo-3-quinolinecarboxylic acid

1-Benzyl-3-chloromethyl-3-(N-methyl)benzylaminopyrrolidine (1.5 g) obtained in Preparative Example 16 was dissolved in ethanol (150 ml), and 60% perchloric acid (1.5 ml) and 20% palladium-carbon catalyst (750 mg) were added. The mixture was stirred in a hydrogen atmosphere for 2 hours. The mixture was passed through Celite, and ethanol was distilled away. The residue was dissolved in a 1N aqueous solution of sodium hydroxide and partitioned with chloroform. The organic layer was dried over sodium sulfate and the solvent was distilled away to give 657 mg of 3-chloromethyl-3-methylaminopyrrolidine as a brown oil.

The obtained 3-chloromethyl-3-methylaminopyrrolidine (657 mg), 1-cyclopropyl-6,7-difluoro-1,4-dihydro-8-methoxy-4-oxo-3-quinolinecarboxylic acid-BF₂ chelate (750 mg) and triethylamine (0.55 ml) were dissolved in acetonitrile (7.5 ml), and the mixture was stirred at room temperature overnight. Ethanol (7.5 ml) was added and the precipitated crystals were collected by filtration, dried under reduced pressure to give 511 mg of 7-(3-chloromethyl-3-methylaminopyrrolidin-1-yl)-1-cyclopropyl-6-fluoro-1,4-dihydro-8-methoxy-4-oxo-3-quinolinecarboxylic acid-BF₂ chelate as a yellow powder.

The obtained 7-(3-chloromethyl-3-methylaminopyrrolidin-1-yl)-1-cyclopropyl-6-fluoro-1,4-dihydro-8-methoxy-4-oxo-3-quinolinecarboxylic acid-BF₂ chelate (511 mg) and triethylamine (0.6 ml) were dissolved in a mixed solvent of chloroform (46 ml) and methanol (5.1 ml), and the mixture was refluxed for 12 hours. The solvent was distilled away under reduced pressure and the residual solid was washed with ethanol and recrystallized from methanol and saturated aqueous ammonia to give 75 mg of the pale-yellow, amorphous object compound, melting point 157.2°C.

### Preparative Example 41

### (S)-7-(3-Amino-3-fluoromethyl-1-pyrrolidin-1-yl)-1-ethyl-6-fluoro-1,4-dihydro-8-methoxy-4-oxo-3-quinolinecarboxylic acid

Triethylamine (1.91 ml) and (S)-3-amino-3-fluoromethylpyrrolidine (1.5 g) were added to a suspension of 1-ethyl-6,7-difluoro-1,4-dihydro-8-methoxy-4-oxo-3-quinolinecarboxylic acid difluoroborane complex (3.63 g) in acetonitrile (25 ml) and the mixture was stirred at room temperature overnight. The reaction mixture was concentrated and methanol (30 ml), water (30 ml) and triethylamine (11.7 ml) were added. The mixture was refluxed for 9 hours. The reaction mixture was concentrated and 2N hydrochloric acid (60 ml) was added. The insoluble matter was filtered off and the aqueous layer was washed 3 times with ethyl acetate. A 6N aqueous solution of sodium hydroxide (30 ml) was added to the aqueous layer to make the solution alkaline and washed 3 times with dichloromethane. The aqueous layer was neutralized with acetic acid, and the precipitate was collected by filtration, washed with water and dried. The crystals were recrystallized from ethanol - aqueous ammonia and recrystallized from ethanol - water to give 1.5 g of the object compound, melting point 122.2-123.8°C.
[α] _{D} = +173.8° (c=0.13, 0.1N hydrochloric acid )

### Example 1

### 7-(3-Amino-3-fluoromethylpyrrolidin-1-yl)-1-cyclopropyl-6-fluoro-1,4-dihydro-8-methoxy-4-oxo-3-quinolinecarboxylic acid

3-Amino-3-fluoromethylpyrrolidine (2.634 g) obtained in Preparative Example 11, 1-cyclopropyl-6,7-difluoro-1,4-dihydro-8-methoxy-4-oxo-3-quinolinecarboxylic acid-BF₂ chelate (5.87 g) and triethylamine (3.58 ml) were dissolved in acetonitrile (117 ml), and the mixture was stirred at room temperature for 20 hours. The precipitated crystals were collected by filtration to give 5.75 g of 7-(3-amino-3-fluoromethyl-1-pyrrolidinyl)-1-cyclopropyl-6-fluoro-1,4-dihydro-8-methoxy-4-oxo-3-quinolinecarboxylic acid-BF₂ chelate as a yellow powder. The yellow powder (5.75 g) was suspended in a mixed solvent of methanol (57.5 ml) and water (3.6 ml), and triethylamine (4.6 ml) was added. The mixture was refluxed under heating overnight. The precipitated crystals were collected by filtration and recrystallized from aqueous ammonia and ethanol to give 3.45 g of the object compound as a pale-yellow powder, melting point 210-211°C.

| Elemental analysis C₁₉H₂₁F₂N₃O₄ | | | |
|---|---|---|---|
| Calculated | C; 58.01, | H; 5.38, | N; 10.68 |
| Found | C; 57.74, | H; 5.12, | N; 10.48 |

### Example 2-1

### 1-Cyclopropyl-6-fluoro-7-(3-fluoromethyl-3-methylaminopyrrolidin-1-yl)-1,4-dihydro-8-methoxy-4-oxo-3-quinolinecarboxylic acid-HCl-1/2 H₂O

1-Benzyl-3-(N-benzyl-N-methyl)amino-3-fluoromethylpyrrolidine (3.559 g) obtained in Preparative Example 12, 10% palladium-carbon (2.28 g) and hydrazine hydrate (1.712 g) were treated in the same manner as in Preparative Example 32 to give 1.162 g of debenzylated crude product as a pale-yellow oil. Said crude product (1.162 g), 1-cyclopropyl-6,7-difluoro-1,4-dihydro-8-methoxy-4-oxo-3-quinolinecarboxylic acid-BF₂ chelate (2.487 g), triethylamine (1 ml) and acetonitrile (36 ml) were mixed and the mixture was stirred at room temeperature for 22 hours. The precipitated crystals were collected by filtration and dried under reduced pressure to give 3.068 g of 1-cyclopropyl-7-(3-fluoromethyl-3-methylamino-1-pyrrolidinyl)-6-fluoro-1,4-dihydro-8-methoxy-4-oxo-3-quinolinecarboxylic acid-BF₂ chelate as a yellow powder. The yellow powder (3.068 g) was suspended in a mixed solvent of methanol (30 ml) and water (10 ml), and triethylamine (41 ml) was added. The mixture was refluxed for 9 hours. The solvent was distilled away under reduced pressure and the residual solid was washed with ethanol, collected by filtration and dried under reduced pressure to give 2.701 g of a pale-yellow solid. This solid was suspended in isopropyl alcohol (42 ml), and conc. hydrochloric acid (0.75 ml) diluted with water (5 ml) was added. The mixture was stirred for 0.5 hour. After cooling, the insoluble matter was filtered off and the filtrate was concentrated. The obtained yellow solid was recrystallized from a mixed solvent of methanol - ether to give 2.855 g of the object compound as a pale-yellow powder, melting point 218.0-220.0 °C.

| Elemental analysis C₂₀H₂₃F₂N₃O₄·HCl·1/2H₂O | | | |
|---|---|---|---|
| Calculated | C; 53.09, | H; 5.56, | N; 9.28 |
| Found | C; 53.39, | H; 5.21, | N; 9.05 |

### Example 2-2

### 1-Cyclopropyl-6-fluoro-7-(3-fluoromethyl-3-methylaminopyrrolidin-1-yl)-1,4-dihydro-8-methoxy-4-oxo-3-quinolinecarboxylic acid

1-Cyclopropyl-6-fluoro-7-(3-fluoromethyl-3-methylaminopyrrolidin-1-yl)-1,4-dihydro-8-methoxy-4-oxo-3-quinolinecarboxylic acid-HCl-1/2 H₂O (2.684 g) obtained in Example 2-1 was dissolved in water (23 ml), and sodium hydroxide (0.559 g) dissolved in water (7 ml) was added. The mixture was stirred for 0.5 hour. The insoluble matter was filtered off and acetic acid (0.450 g) was added to the filtrate with stirring at room temperature. The mixture was further stirred for 1 hour. The precipitated crystals were collected by filtration, washed with ethanol and ether in order, and dried under reduced pressure at 80°C for 14 hours to give 2.372 g of the object compound as a colorless powder, melting point 196.2-194.8°C.

| Elemental analysis C₂₀H₂₃F₂N₃O₄ | | | |
|---|---|---|---|
| Calculated | C; 58.96, | H; 5.69, | N; 10.31 |
| Found | C; 58.95, | H; 5.60, | N; 10.30 |

### Example 3

### (1) (S)-1-Benzyl-3-(N-benzoyl-N-methyl)amino-3-hydroxymethylpyrrolidine

(S)-1-Benzyl-3-(N-benzoyl-N-methyl)amino-3-ethoxycarbonylpyrrolidine (12.91 g) obtained in Preparative Example 24 was treated in the same manner as in Preparative Example 20 (7) to give 9.98 g of the object compound as a pale-yellow oil.
[α] _{D} = +49.65° (c=2, chloroform)

### (2) (S)-1-Benzyl-3-(N-benzyl-N-methyl)amino-3-fluoromethylpyrrolidine

(S)-1-Benzyl-3-(N-benzyl-N-methyl)amino-3-hydroxymethylpyrrolidine (9.11 g) was treated in the same manner as in Preparative Example 20 (8) to give 7.95 g of the object compound as a pale-yellow oil.
[α] _{D} = +38.7° (c=0.1, chloroform)

### (3) (S)-3-Fluoromethyl-3-methylaminopyrrolidine

(S)-1-Benzyl-3-(N-benzyl-N-methyl)amino-3-fluoromethylpyrrolidine (6.43 g) was treated in the same manner as in Preparative Example 20 (9) to give 2.10 g of the object compound as a colorless oil, boiling point 80-120°C (10 mmHg).
[α] _{D} = +7.20° (c=0.24, chloroform)

### (4) (S)-1-Cyclopropyl-6-fluoro-7-(3-fluoromethyl-3-methylaminopyrrolidin-1-yl)-1,4-dihydro-8-methoxy-3-quinolinecarboxylic acid

(S)-(+)-3-Fluoromethyl-3-methylaminopyrrolidine (2.07 g) and triethylamine (1.59 g) were dissolved in acetonitrile, and 1-cyclopropyl-6,7-difluoro-1,4-dihydro-8-methoxy-3-quinoline carboxylic acid difluoroborane complex (4.14 g) was added in a nitrogen stream. The mixture was stirred at room temperature for 19 hours. The solvent was distilled away under reduced pressure and methanol (100 ml), dichloromethane (100 ml) and triethylamine (9.76 g) were added to the residue. The mixture was refluxed for 30 hours. The solvent was distilled away and ethanol was added to the residue to allow crystallization. The obtained crude crystals were recrystallized from ethanol - ammonia to give 697 mg of the object compound, melting point 164.1-168.1°C.
[α] _{D} = +237.7° (c=0.05, 1N hydrochloric acid)

### Example 4

### (1) (R)-1-Benzyl-3-(N-benzyl-N-methyl)amino-3-hydroxymethylpyrrolidine

(R)-1-Benzyl-3-(N-benzoyl-N-methyl)amino-3-ethoxycarbonylpyrrolidine (9.33 g) obtained in Preparative Example 24 was treated in the same manner as in Preparative Example 20 (7) to give 8.0 g of the object compound as a pale-yellow oil.
[α] _{D} = -34.0° (c=0.95, ethanol)

### (2) (R)-1-Benzyl-3-(N-benzyl-N-methyl)amino-3-fluoromethylpyrrolidine

(R)-1-Benzyl-3-(N-benzyl-N-methyl)amino-3-hydroxymethylpyrrolidine (7.80 g) was treated in the same manner as Preparative Example 20 (8) to give 6.10 g of the object compound as a pale-yellow oil.
[α] _{D} = -29.7° (c=0.10, ethanol)

### (3) (R)-1-Cyclopropyl-6-fluoro-7-(3-fluoromethyl-3-methylaminopyrrolidin-1-yl)-1,4-dihydro-8-methoxy-3-quinolinecarboxylic acid

(R)-1-Benzyl-3-(N-benzyl-N-methyl)amino-3-fluoromethylpyrrolidine (5.79 g) was treated in the same manner as in Preparative Example 20 (9) to give 3.038 g of crude (R)-3-fluoromethyl-3-methylaminopyrrolidine as a pale-yellow oil.

The above-mentioned crude pale-yellow oil (3.038 g) and 1-cyclopropyl-6,7-difluoro-1,4-dihydro-8-methoxy-3-quinolinecarboxylic acid difluoroborane complex (4.88 g) were treated in the same manner as in Example 17 to give 4.782 g of the object compound.
[α] _{D} = -257.4° (c=0.115, 0.1N hydrochloric acid)

### Example 5

### (1) (R)-1-Benzyl-3-(N,N-dibenzyl)amino-3-hydroxymethylpyrrolidine

(R)-1-Benzyl-3-(N-benzoyl-N-benzyl)amino-3-ethoxycarbonylpyrrolidine (13.2 g) obtained in Preparative Example 23 was treated in the same manner as in Preparative Example 20 (7) to give 11.09 g of the object compound as a pale-yellow oil.
[α] _{D} = -27.3° (c=0.230, chloroform)

### (2) (R)-1-Benzyl-3-(N,N-dibenzyl)amino-3-fluoromethylpyrrolidine

(R)-1-Benzyl-3-(N,N-dibenzyl)amino-3-hydroxymethylpyrrolidine (10.8 g) was treated in the same manner as in Preparative Example 20 (8) to give 9.60 g of the object compound as a pale-yellow oil.
[α] _{D} = -23.9° (c=0.245, chloroform)

### (3) (R)-3-Amino-3-fluoromethylpyrrolidine

(R)-1-Benzyl-3-(N,N-dibenzyl)amino-3-fluoromethylpyrrolidine (8.0 g) was treated in the same manner as in Preparative Example 20 (9) to give 1.31 g of the object compound as a colorless oil, boiling point 100-120°C (10 mmHg, Kugelrohr).

### (4) (R)-7-(3-amino-3-fluoromethylpyrrolidin-1-yl)-1-cyclopropyl-6-fluoro-1,4-dihydro-8-methoxy-3-quinolinecarboxylic acid

(R)-3-Amino-3-fluoromethylpyrrolidine (1.31 g) and 1-cyclopropyl-6,7-difluoro-1,4-dihydro-8-methoxy-3-quinolinecarboxylic acid difluoroborane complex (3.17 g) were treated in the same manner as in Example 3 (4) to give 2.66 g of the object compound, melting point 176-177°C (decomposition).
[α] _{D} = -225° (c=0.210, 0.1N hydrochloric acid)

### Example 6

### (1) (S)-1-Benzyl-3-(N,N-dibenzyl)amino-3-hydroxymethylpyrrolidine

(S)-3-(N-Benzoyl-N-benzyl)amino-1-benzyl-3-ethoxycarbonylpyrrolidine (13.0 g) obtained in Preparative Example 23 was treated in the same manner as in Preparative Example 20 (7) to give 10.5 g of the object compound as a pale-yellow oil.
[α] _{D} = +29.2° (c=0.190, chloroform)

### (2) (S)-3-(N,N-Dibenzyl)amino-1-benzyl-3-fluoromethylpyrrolidine

(S)-3-(N,N-Dibenzyl)amino-1-benzyl-3-hydroxymethylpyrrolidine (10.5 g) was treated in the same manner as in Preparative Example 20 (8) to give 9.0 g of the object compound as a pale-yellow oil.
[α] _{D} = +22.4° (c=0.33, chloroform)

### (3) (S)-3-Amino-3-fluoromethylpyrrolidine

(S)-3-(N,N-Dibenzyl)amino-1-benzyl-3-fluoromethylpyrrolidine (8.6 g) was treated in the same manner as in Preparative Example 20 (9) to give 2.5 g of the object compound as a pale-yellow oil, boiling point 100-120°C (10 mmHg, Kugelrohr).

### (4) (S)-7-(3-Amino-3-fluoromethylpyrrolidin-1-yl)-1-cyclopropyl-6-fluoro-1,4-dihydro-8-methoxy-3-quinolinecarboxylic acid

(S)-3-Amino-3-fluoromethylpyrrolidine (2.5 g) and 1-cyclopropyl-6,7-difluoro-1,4-dihydro-8-methoxy-3-quinolinecarboxylic acid difluoroborane complex (6.0 g) were treated in the same manner as in Example 3 (4) to give 3.00 g of the object compound, melting point 185-188°C (decomposition).
[α] _{D} = -211.1° (c=0.017, 0.1N hydrochloric acid)

### Comparative Example 1

### (1) 7-(4-Amino-4-(t-butoxycarbonylaminomethyl)piperidin-1-yl)-1-cyclopropyl-6-fluoro-1,4-dihydro-8-methoxy-4-oxo-3-quinolinecarboxylic acid-BF₂ chelate

1-Benzyl-4-benzylamino-4-(t-butoxycarbonylaminomethyl)piperidine (2.2 g) obtained in Preparative Example 19 (2) was dissolved in acetic acid (50 ml) and 20% palladium hydroxide-carbon (700 mg) was added. The mixture was stirred at 60°C under a hydrogen atmosphere. Five hours later, the catalyst was filtered off and the filtrate was concentrated to give crude amine (1.2 g). The crude amine (1.2 g) and 1-cyclopropyl-6,7-difluoro-1,4-dihydro-8-methoxy-4-oxo-3-quinolinecarboxylic acid-BF₂ chelate (858 mg) were dissolved in acetonitrile (50 ml), and the mixture was stirred at room temperature overnight. The solvent was distilled away and the residue was purified by silica gel column chromatography to give 430 mg of the object compound as crystals.

### (2) 7-(4-Amino-4-(t-butoxycarbonylaminomethyl)piperidin-1-yl)-1-cyclopropyl-6-fluoro-1,4-dihydro-8-methoxy-4-oxo-3-quinolinecarboxylic acid

7-(4-Amino-4-(t-butoxycarbonylaminomethyl)piperidin-1-yl)-1-cyclopropyl-6-fluoro-1,4-dihydro-8-methoxy-4-oxo-3-quinolinecarboxylic acid·BF₂ chelate (430 mg) obtained in Comparative Example 1 (1) was dissolved in methanol (50 ml) and triethylamine (0.5 ml) was added. The mixture was refluxed under heating for 5 hours. The solvent was distilled away and water - chloroform was added to the residue for partition. The organic layer was dried over magnesium sulfate. The solvent was distilled away and the residue was crystallized from acetone - methanol to give 295 mg of the object compound as colorless crystals.

### (3) 7-(4-Amino-4-aminomethylpiperidin-1-yl)-1-cyclopropyl-6-fluoro-1,4-dihydro-8-methoxy-4-oxo-3-quinolinecarboxylic acid

Trifluoroacetic acid (1.5 ml) was dropwise added to 7-(4-amino-4-(t-butoxycarbonylaminomethyl)piperidin-1-yl)-1-cyclopropyl-6-fluoro-1,4-dihydro-8-methoxy-4-oxo-3-quinolinecarboxylic acid (290 mg) obtained in Comparative Example 1 (2) under ice-cooling, and the mixture was heated to room temperature. Five minutes later, aqueous ammonia was added to make the solution basic. After the solvent was distilled away, the residue was dissolved in methanol, and desalted by active carbon column chromatography to give 76 mg of the object compound as colorless crystals, melting point 145°C.

### Preparative Example 42

### (1) Monoethyl 1-benzyl-5-oxopyrrolidine-3,3-dicarboxylate

Diethyl 1-benzyl-5-oxopyrrolidine-3,3-dicarboxylate (61.1 g) was dissolved in ethanol (60 ml), and a solution of 85% potassium hydroxide (12.6 g) and ethanol (60 ml) was added. The mixture was allowed to stand at room temperature overnight. Ethanol was distilled away under reduced pressure and water was added. The mixture was washed with ethyl acetate. The aqueous layer was adjusted to pH 1 with hydrochloric acid and extracted with ethyl acetate. The ethyl acetate layer was washed with water, dried and concentrated to give 49.5 g of the object compound as white crystals.

### (2) Ethyl 1-benzyl-3-(benzylcarbamoyl)-5-oxopyrrolidine-3-carboxylate

1-Hydroxybenztriazole (2.78 g) and dicyclohexylcarbodiimide (42.5 g) were added to a mixture of monoethyl 1-benzyl-5-oxopyrrolidine-3,3-dicarboxylate (60.0 g), benzylamine (23.2 g) and tetrahydrofuran (500 ml), and the mixture was stirred at room temperature overnight. The insoluble matter was removed by filtration and the filtrate was concentrated. Ethyl acetate (400 ml) was added and the mixture was sequentially washed with an aqueous solution of potassium carbonate, water, dilute hydrochloric acid and saturated brine, dried, and concentrated to give 78 g of the object compound as white crystals, melting point 63-65°C.

### (3) 1-Benzyl-3-(benzylaminomethyl)-3-hydroxymethylpyrrolidine

A solution of ethyl 1-benzyl-3-(benzylcarbamoyl)-5-oxopyrrolidine-3-carboxylate (9.90 g) in tetrahydrofuran (20 ml) was dropwise added to a mixture of lithium aluminum hydride (2.96 g) and tetrahydrofuran (50 ml) over 20 minutes. The mixture was refluxed for 8 hours, and water (3ml), a 15% aqueous solution (3 ml) of sodium hydroxide and water (9 ml) were added under ice-cooling. The mixture was allowed to stand overnight. The insoluble matter was removed by filtration and the filtrate was concentrated. Ethyl acetate was added, and the mixture was washed with saturated brine, dried, and concentrated. The concentrate was purified by silica gel column chromatography to give 6.03 g of the object compound as an oil.
NMR(CDCl₃ ) δ : 1.6-1.9(m, 2H), 2.2-2.8(m, 6H), 3.56(s, 2H), 3.62(s, 2H), 3.74(s, 2H), 7.26(s, 10H)

### (4) 3-Aminomethyl-3-hydroxymethylpyrrolidine

1-Benzyl-3-(benzylaminomethyl)-3-hydroxymethylpyrrolidine (2.0 g) was dissolved in ethanol (20 ml), and 10% palladium-carbon (1 g) was added. The mixture was hydrogenated under atmospheric pressure. After the completion of the reaction, the catalyst was removed, and the mixture was concentrated to give the object compound as an oil.
NMR(CDCl₃ ) δ : 1.3-1.8(m, 2H), 2.6(bs, 6H), 2.8-3.0(m, 4H), 3.62(dd, 2H)

### Preparative Example 43

### (1) Ethyl 1-benzyl-3-(dibenzylcarbamoyl)-5-oxopyrrolidine-3-carboxylate

A solution of ethyl 1-benzyl-3-(benzylcarbamoyl)-5-oxopyrrolidine-3-carboxylate obtained in Preparative Example 42 (2) (20 g) in dimethylformamide (20 ml) was added to a mixture of 60% sodium hydride (2.5 g) and dimethylformamide (60 ml) under ice-cooling. After the mixture was stirred at room temperature for 1.5 hours, benzyl bromide (9.7 g) was added under ice-cooling and the mixture was stirred for 1 hour. The reaction mixture was poured into ice water and extracted with ethyl acetate. The extract was washed with water, dried, concentrated and purified by silica gel column chromatography to give 16.3 g of the object compound as white crystals, melting point 112-115°C.

### (2) 1-Benzyl-3-(dibenzylaminomethyl)-3-hydroxymethylpyrrolidine

Ethyl 1-benzyl-3-(dibenzylcarbamoyl)-5-oxopyrrolidine-3-carboxylate (16.2 g) was treated in the same manner as in Preparative Example 42 (3) to give 11.5 g of the object compound as white crystals, melting point 65-67°C.

### (3) 1-Benzyl-3-(dibenzylaminomethyl)-3-mesyloxymethylpyrrolidine

Mesyl chloride (2.7 ml) was added to a solution of 1-benzyl-3-(dibenzylaminomethyl)-3-hydroxymethylpyrrolidine (7.88 g) and triethylamine (5.5 ml) in chloroform (80 ml) under ice-cooling, and the mixture was stirred for 30 minutes under ice-cooling. The reaction mixture was sequentially washed with an aqueous solution of sodium hydrogencarbonate and water, dried, concentrated and purified by silica gel column chromatography to give 6.97 g of the object compound as an oil.
NMR(CDCl₃ ) δ :1.5-1.9(m, 2H), 2.29(dd, 2H), 2.67(s, 2H), 2.80(s, 3H), 3.45(d, 2H), 3.60(s, 4H), 4.15(s, 2H), 7.23(s, 5H), 7.30(s, 10H)

### (4) 1-Benzyl-3-(dibenzylaminomethyl)-3-fluoromethylpyrrolidine

A solution of 1-benzyl-3-(dibenzylaminomethyl)-3-mesyloxymethylpyrrolidine (6.90 g) in acetonitrile (70 ml) was added to 1N tetrabutylammonium fluoride in tetrahydrofuran (72 ml), and the mixture was stirred at 40°C for 16 hours. After concentration, the concentrate was poured into water (200 ml), and the mixture was made alkaline with aqueous ammonia and extracted with ether. The ether layer was washed with water, dried, concentrated and purified by silica gel column chromatography to give 4.37 g of the object compound as an oil.
NMR(CDCl₃ ) δ : 1.35-1.6(m, 2H), 2.05-2.6(m, 4H), 2.64(s, 2H), 3.42(s, 2H), 3.55(s, 4H), 4.34(dd, J=48, 3Hz, 2H), 7.22(s, 5H), 7.27(s, 10H)

### (5) 3-Aminomethyl-3-fluoromethylpyrrolidine

1-Benzyl-3-(dibenzylaminomethyl)-3-fluoromethylpyrrolidine (4.55 g) was dissolved in ethanol (40 ml), and 10% palladium-carbon (1 g) and hydrazine monohydrate (2.15 g) were added. The mixture was refluxed for 1 hour. After the completion of the reaction, the catalyst was removed, and the mixture was concentrated to give the object compound as an oil.
NMR(CDCl₃ ) δ : 1.61(td, J=7, 2Hz, 2H), 2.68(s, 3H), 2.75-2.85(m, 4H), 3.00(t, J=7Hz, 2H), 4.38(d, J=48Hz, 2H)

### Preparative Example 44

### (1) Ethyl 1-benzyl-3-(N-benzyl-N-methylcarbamoyl)-5-oxopyrrolidine-3-carboxylate

Monoethyl 1-benzyl-5-oxopyrrolidine-3,3-dicarboxylate (29.1 g) obtained in Preparative Example 42 (1) and N-benzyl-N-methylamine (12.1 g) were treated in the same manner as in Preparative Example 42 (2) to give 31 g of the object compound as an oil.
NMR(CDCl₃ ) δ : 1.16(t, J=7Hz, 3H), 2.69(s, 3H), 3.16(s, 2H), 3.59(d, J=10Hz, 1H), 4.0-4.8(m, 7H), 7.26(s, 5H), 7.29(s, 5H)

### (2) 1-Benzyl-3-(N-benzyl-N-methylaminomethyl)-3-hydroxymethylpyrrolidine

Ethyl 1-benzyl-3-(N-benzyl-N-methylcarbamoyl)-5-oxopyrrolidine-3-carboxylate (19.3 g) was treated in the same manner as in Preparative Example 42 (3) to give 11.6 g of the object compound as an oil.
NMR(CDCl₃ ) δ : 1.4-1.9(m, 2H), 2.0-2.9(m, 9H), 3.50(s, 2H), 3.56(ABq, 2H), 3.58(s, 2H), 7.26(s, 10H)

### (3) 3-Hydroxymethyl-3-methylaminomethylpyrrolidine

1-Benzyl-3-(N-benzyl-N-methylaminomethyl)-3-hydroxymethylpyrrolidine (6.80 g) was treated in the same manner as in Preparative Example 43 (5) to give the object compound as an oil.
NMR(CDCl₃ ) δ : 1.3-1.8(m, 2H), 2.44(s, 3H), 2.5-3.0(m, 6H), 3.25(bs, 3H), 3.59(s, 2H)

### Preparative Example 45

### (1) 1-Benzyl-3-(N-benzyl-N-methylaminomethyl)-3-fluoromethylpyrrolidine

A solution of mesyl chloride (3.15 ml) in methylene chloride (11 ml) was dropwise added to a solution of 1-benzyl-3-(N-benzyl-N-methylaminomethyl)-3-hydroxymethylpyrrolidine (11.0 g) obtained in Preparative Example 44 (2) and triethylamine (7.1 ml) in methylene chloride (44 ml) at not more than -20°C. After the completion of the reaction, the reaction mixture was poured into a mixture of ether (200 ml) and water (100 ml), and the ether layer was washed with water, dried and concentrated. The residue was dissolved in acetonitrile (50 ml), and the mixture was added to 1N tetrabutylammonium fluoride in tetrahydrofuran (100 ml). The mixture was stirred at 50°C for 4 hours. After concentration, the concentrate was poured into water (300 ml), and the mixture was made alkaline with aqueous ammonia and extracted with ether. The ether layer was washed with water, dried, concentrated and purified by silica gel column chromatography to give 7.90 g of the object compound as an oil.
NMR(CDCl₃ ) δ : 1.63(td, J=7, 2Hz, 2H), 2.21(s, 3H), 2.3-2.7(m, 6H), 3.56(s, 2H), 3.58(s, 2H), 4.41(ddd, J=48, 11, 8Hz, 2H), 7.27(s, 10H)

### (2) 3-Fluoromethyl-3-methylaminomethylpyrrolidine

1-Benzyl-3-(N-benzyl-N-methylaminomethyl)-3-fluoromethylpyrrolidine (7.90 g) was treated in the same manner as in Preparative Example 43 (5) to give the object compound as an oil.
NMR(CDCl₃ ) δ : 1.62(td, J=7, 2Hz, 2H), 2.35(s, 2H), 2.46(s, 3H), 2.64(s, 2H), 2.8-2.9(m, 2H), 2.97(t, J=7Hz, 2H), 4.36(d, J=48Hz)

### Preparative Example 46

### (1) 1-Benzyl-3,3-bis(hydroxymethyl)pyrrolidine

Diethyl 1-benzyl-5-oxopyrrolidine-3,3-dicarboxylate (3.19 g) was treated in the same manner as in Preparative Example 42 (3) to give 2.57 g of the object compound as an oil.
NMR(CDCl₃) δ : 1.63(t, 2H), 2.48(s, 2H), 2.59(t, 2H), 3.57(s, 8H), 7.27(s, 5H)

### (2) 3,3-Bis(hydroxymethyl)pyrrolidine

1-Benzyl-3,3-bis(hydroxymethyl)pyrrolidine (2.32 g) was treated in the same manner as in Preparative Example 42 (4) to give the object compound as an oil.
NMR(D₂O ) δ : 1.93(t, J=8Hz, 2H), 3.21(s, 2H), 3.39(t, J=8Hz), 3.61(s, 4H)

### Preparative Example 47

### (1) Diethyl 1-benzyl-6-oxopiperidine-3,3-dicarboxylate

A mixture of triethyl 1,1,3-propanetricarboxylate (89.7 g), 1,3,5-tribenzylhexahydro-1,3,5-triazine (102.6 g) and trifluoroacetic acid (6.5 g) was stirred at 100°C for 26 hours. Toluene was added, and the resulting mixture was sequentially washed with 10% hydrochloric acid, a saturated aqueous solution of sodium hydrogencarbonate and water, dried, concentrated, and purified by silica gel column chromatography to give 75 g of the object compound as an oil.
NMR(CDCl₃ ) δ :1.20(t,6H), 2.12-2.68(m,4H), 3.67(s,2H), 3.90-4.30(m,4H), 4.60(s,2H), 7.28(s,5H)

### (2) Monoethyl 1-benzyl-6-oxopiperidine-3,3-dicarboxylate

Diethyl 1-benzyl-6-oxopiperidine-3,3-dicarboxylate (75 g) was treated in the same manner as in Preparative Example 42 (1) to give 57 g of the object compound as white crystals, melting point 125-127°C.

### (3) Ethyl 1-benzyl-3-(N-benzyl-N-methylcarbamoyl)-6-oxopiperidine-3-carboxylate

Monoethyl 1-benzyl-6-oxopiperidine-3,3-dicarboxylate (27.4 g) was treated in the same manner as in Preparative Example 44 (1) to give 23.8 g of the object compound as an oil.
NMR(CDCl₃ ) δ :1.10(t,3H), 2.08-2.62(m,4H), 2.72(s,3H), 3.30-4.92(m,8H), 6.92-7.42(m,10H)

### (4) 1-Benzyl-3-(N-benzyl-N-methylaminomethyl)-3-hydroxymethylpiperidine

Ethyl 1-benzyl-3-(N-benzyl-N-methylcarbamoyl)-6-oxopiperidine-3-carboxylate (23.8 g) was treated in the same manner as in Preparative Example 42 (3) to give 13.8 g of the object compound as an oil.
NMR(CDCl₃ ) δ :1.00-1.76(m,4H), 2.20(s,3H), 2.21-2.88(m,6H), 3.27-3.76(m,6H), 7.00-7.40(m,10H)

### (5) 1-Benzyl-3-(N-benzyl-N-methylaminomethyl)-3-fluoromethylpiperidine

1-Benzyl-3-(N-benzyl-N-methylaminomethyl)-3-hydroxymethylpiperidine (10 g) was treated in the same manner as in Preparative Example 45 (1) to give 5.2 g of the object compound as an oil.
NMR(CDCl₃ ) δ :1.08-1.74(m,4H), 2.20(s,3H), 2.21-2.42(m,4H), 2.52(s,2H), 3.40(d,2H), 3.56(s,2H), 4.50(dd,2H), 7.24(s, 10H)

### (6) 3-Methylaminomethyl-3-fluoromethylpiperidine

1-Benzyl-3-(N-benzyl-N-methylaminomethyl)-3-fluoromethylpiperidine (0.9 g) was treated in the same manner as in Preparative Example 43 (5) to give the object compound as an oil, which was used in the following step without purification.

### Preparative Example 48

### (1) Ethyl 1-benzyl-3-(N-benzylcarbamoyl)-6-oxopiperidine-3-carboxylate

Monoethyl 1-benzyl-6-oxopiperidine-3,3-dicarboxylate (29 g) obtained in Preparative Example 47 (2) was treated in the same manner as in Preparative Example 42 (2) to give 31 g of the object compound as an oil.
NMR(CDCl₃) δ :1.12(t,3H), 2.08-2.64(m,4H), 3.75(s,2H), 4.08(q,2H), 4.35(d,2H), 4.59(s,2H), 6.39(s,1H), 6.92-7.44(m,10H)

### (2) Ethyl 1-benzyl-3-(N,N-dibenzylcarbamoyl)-6-oxopiperidine-3-carboxylate

Ethyl 1-benzyl-3-(N-benzylcarbamoyl)-6-oxopiperidine-3-carboxylate (15 g) was treated in the same manner as in Preparative Example 43 (1) to give 13.7 g of the object compound as an oil.
NMR(CDCl₃) δ :1.04(t,3H), 2.04-2.68(m,4H), 3.32-3.99(m,4H), 4.14-5.10(m,6H), 6.80-7.48(m,15H)

### (3) 1-Benzyl-3-dibenzylaminomethyl-3-hydroxymethylpiperidine

Ethyl 1-benzyl-3-(N,N-dibenzylcarbamoyl)-6-oxopiperidine-3-carboxylate (13.6 g) was treated in the same manner as in Preparative Example 42 (3) to give 8.5 g of the object compound as an oil.
NMR(CDCl₃) δ :1.00-1.72(m,4H), 2.06-2.80(m,6H), 3.10-3.77(m,8H), 7.00-7.36(m,15H)

### (4) 1-Benzyl-3-dibenzylaminomethyl-3-fluoromethylpiperidine

1-Benzyl-3-dibenzylaminomethyl-3-hydroxymethylpiperidine (8.4 g) was treated in the same manner as in Preparative Example 45 (1) to give 3.9 g of the object compound as an oil.
NMR(CDCl₃ ) δ :0.96-1.68(m,4H), 1.70-2.46(m,4H), 2.58(d,2H), 3.24(d,2H), 3.58(s,4H), 3.90-4.80(m,2H), 7.00-7.38(m,15H)

### (5) 3-Aminomethyl-3-fluoromethylpiperidine

1-Benzyl-3-dibenzylaminomethyl-3-fluoromethylpiperidine (2.9 g) was treated in the same manner as in Preparative Example 43 (5) to give 1.3 g of the object compound as an oil, which was used in the following step without purification.

### Preparative Example 49

### (1) Ethyl 1-benzyl-4-cyanopiperidine-4-carboxylate

A solution of diethyl azodicarboxylate (72.1 g) and tetrahydrofuran (120 ml) was dropwise added to a mixture of triphenylphosphine (108 g) and tetrahydrofuran (1.2 l) at -30°C over 40 minutes, and the mixture was further stirred for 1.5 hours. A solution of N-benzyldiethanolamine (27 g) and ethyl cyanoacetate (15.6 g) in tetrahydrofuran (120 ml) was dropwise added at -30°C over 30 minutes, and the mixture was further stirred for 1 hour. The reaction mixture was allowed to stand at -25°C overnight. The resulting mixture was stirred for 9 hours under ice-cooling, and was allowed to stand for 3 days under ice-cooling. The solvent was distilled away, and toluene (1 ℓ) and water (0.5 ℓ) were added. The mixture was made acidic with hydrochloric acid (23 ml), and the aqueous layer was separated. The aqueous layer was made alkaline with potassium carbonate (27 g), and extracted with chloroform and dried. The solvent was distilled away, and the residue was purified by silica gel column chromatography to give 18 g of the object compound as pale-yellow crystals, melting point 53-55°C.

### (2) 1-Benzyl-4-cyano-4-hydroxymethylpiperidine

Ethyl 1-benzyl-4-cyanopiperidine-4-carboxylate (2.72 g) was dissolved in methanol (20 ml) and sodium borohydride (0.567 g) was added under ice-cooling. The mixture was stirred for 2.5 hours. The solvent was removed, and water was added. The mixture was extracted with chloroform and the organic layer was dried. The solvent was distilled away to give 2.22 g of the object compound as white crystals, melting point 108-110°C.

### (3) 4-Aminomethyl-1-benzyl-4-hydroxymethylpiperidine

Lithium aluminum hydride (2.06 g) was suspended in tetrahydrofuran (50 ml), and a solution of 1-benzyl-4-cyano-4-hydroxymethylpiperidine (5 g) in tetrahydrofuran (50 ml) was dropwise added under ice-cooling. The mixture was stirred for 3 hours under ice-cooling. Water (2.1 ml), 15% aqueous solution of sodium hydroxide (2.1 ml) and water (6.3 ml) were added in order under ice-cooling, and the resulting mixture was allowed to stand overnight. The insoluble matter was removed and the solvent was distilled away. The residue was extracted with chloroform, dried, and the solvent was distilled away to give the object compound as white crystals, melting point 75-77°C.

### (4) 4-Aminomethyl-4-hydroxymethylpiperidine

4-Aminomethyl-1-benzyl-4-hydroxymethylpiperidine (1.0 g) was treated in the same manner as in Preparative Example 42 (4) to quantitatively give the object compound as an oil.
NMR(CDCl₃ +D₂ O) δ :1.3-1.5(m, 4H), 2.7-2.9(m, 4H), 2.8(s, 2H), 3.6(s, 2H)

### Preparative Example 50

### (1) 4-Benzoylaminomethyl-1-benzyl-4-hydroxymethylpiperidine

4-Aminomethyl-1-benzyl-4-hydroxymethylpiperidine (2.66 g) and potassium carbonate (1.88 g) were dissolved in tetrahydrofuran (25 ml) and water (25 ml) and a solution of benzoyl chloride (1.91 g) in tetrahydrofuran (20 ml) were dropwise added under ice-cooling. The mixture was stirred for 1 hour. The reaction mixture was extracted with ethyl acetate, washed with saturated brine and dried. The solvent was distilled away to quantitatively give the object compound as white crystals, melting point 158-160°C.

### (2) 4-(N-benzoyl-N-benzylaminomethyl)-1-benzyl-4-hydroxymethylpiperidine

60% Sodium hydride (0.46 g) was suspended in N,N-dimethylformamide (40 ml), and a solution of 4-benzoylaminomethyl-1-benzyl-4-hydroxymethylpiperidine (4.29 g) in N,N-dimethylformamide (40 ml) was dropwise added at room temperature. The mixture was stirred for 2 hours. Benzyl bromide (1.36 ml) was added under ice-cooling, and the mixture was stirred at room temperature overnight. The reaction mixture was poured into ice water and extracted with ethyl acetate. The organic layer was washed with water and dried, and the solvent was distilled away to quantitatively give the object compound as a yellow oil.
NMR(CDCl₃ ) δ :1.5-1.7(m, 4H), 2.3-2.5(m, 4H), 3.5-3.6(m, 7H), 4.5(s, 2H), 7.1-7.5(m, 15H)

### (3) 1-Benzyl-4-dibenzylaminomethyl-4-hydroxymethylpiperidine

Lithium aluminum hydrate (1.19 g) was suspended in tetrahydrofuran (30 ml), and a solution of 4-(N-benzoyl-N-benzylaminomethyl)-1-benzyl-4-hydroxymethylpiperidine (6.7 g) in tetrahydrofuran (30 ml) was dropwise added. The mixture was stirred at room temperature for 30 minutes and refluxed for 7 hours. Then, water (1.2 ml), a 15% aqueous solution of sodium hydroxide (1.2 ml) and water (3.6 ml) were added under ice-cooling. The mixture was allowed to stand overnight. The insoluble matter was removed, and the resulting mixture was concentrated. Ethyl acetate was added and the resulting mixture was washed with saturated brine, dried, concentrated and purified by silica gel column chromatography to give 2.3 g of the object compound as an oil.
NMR(CDCl₃) δ : 1.4-1.6(m, 4H), 2.2-2.4(m, 4H), 2.6(s, 2H), 3.35(s,2H), 3.45(s, 2H), 3.75(s, 2H), 7.3(s, 15H)

### Preparative Example 51

### (1) 1-Benzyl-3-(N,N-dibenzylcarbamoyl)-5-oxopyrrolidine-3-carboxylic acid

Monoethyl 1-benzyl-3-(N,N-dibenzylcarbamoyl)-5-oxopyrrolidine-3-carboxylate (132 g) obtained in Preparative Example 43 (1) was dissolved in methanol (900 ml), and a solution of sodium hydroxide (44.9 g) and water (450 ml) was added. The mixture was stirred at 40°C for 4 hours and methanol was distilled away under reduced pressure. The residue was washed with toluene and hydrochloric acid was added to make the mixture acidic. The resulting mixture was extracted with ethyl acetate, and the organic layer was washed with saturated brine, dried and concentrated to quantitatively give the object compound, melting point 123-125°C.

### (2) 1-Benzyl-3-dibenzylaminomethyl-3-hydroxymethylpyrrolidine

1-Benzyl-3-(N,N-dibenzylcarbamoyl)-5-oxopyrrolidine-3-carboxylic acid (124 g) was dissolved in tetrahydrofuran (1.1 ℓ) and sodium borohydride (79.7 g) was added over 15 minutes under ice-cooling. A solution of sulfuric acid (56 ml) in tetrahydrofuran (560 ml) was added over 1 hour under ice-cooling, and the mixture was refluxed for 3 hours. 3N Hydrochloric acid (500 ml) was added under ice-cooling and the mixture was refluxed for 2.5 hours. After concentration, water (1 ℓ) was added and the resulting mixture was made alkaline with an aqueous solution of sodium hydroxide. The resulting mixture was extracted with ethyl acetate and washed with saturated brine. After drying, the organic layer was concentrated and purified by silica gel column chromatography to give 90 g of the object compound as white crystals, melting point 65-67°C.

### Preparative Example 52

### Diethyl 1-((S)-α-methylbenzyl)-5-oxopyrrolidine-3,3-dicarboxylate

L-(-)-α-Methylbenzylamine (121 g) was dissolved in toluene (121 ml) and 37% formaldehyde (89.3 g) was dropwise added at room temperature. The mixture was stirred at 40°C for 1 hour. Then, sodium hydroxide (1 g) and water (3 ml) were added, and the mixture was stirred for 1 hour. The reaction mixture was washed with water, and the organic layer was dried over anhydrous magnesium sulfate and concentrated under reduced pressure. Triethyl 1,1,2-ethanetricarboxylate (82 g) and trifluoroacetic acid (7.6 g) were added to the obtained oil, and the mixture was stirred at 100°C for 24 hours. After cooling, the mixture was diluted with ethyl acetate (500 ml) and sequentially washed with 10% hydrochloric acid, water and a saturated aqueous solution of sodium hydrogencarbonate. The resulting mixture was dried over anhydrous magnesium sulfate, concentrated under reduced pressure and purified by silica gel column chromatography to give the object compound.
NMR(CDCl₃) δ : 1.17(t,3H), 1.28(t,3H), 1.56(d,3H), 3.03(dd,2H), 3.60(dd,2H), 4.12(q,2H), 4.24(q,2H), 5.48(q,2H), 7.10-7.40(m,5H),
[α] _{D} = - 28.0° (c = 1%, methanol)

### Preparative Example 53

### (RS)-3-Ethoxycarbonyl-1-((S)-α-methylbenzyl)-5-oxopyrrolidine-3-carboxylic acid

85% Potassium hydroxide (13.9 g) was dissolved in ethanol (900 ml) and diethyl 1-(S)-α-methylbenzyl)-5-oxopyrrolidine-3,3-dicarboxylate (70 g) was added. The mixture was stirred at room temperature for 4 hours. The solution was concentrated under reduced pressure, and water (500 ml) was added. The mixture was washed with toluene (300 ml), and 10% hydrochloric acid was added to the aqueous layer to make the solution acidic. The aqueous layer was extracted three times with ethyl acetate (300 ml). The extract was washed with water and saturated brine, dried over anhydrous magnesium sulfate and concentrated under reduced pressure to give the object compound, melting point 128-135°C.

### Preparative Example 54

### Ethyl (RS)-3-(N,N-dibenzylcarbamoyl)-1-((S)-α-methylbenzyl)-5-oxo-pyrrolidine-3-carboxylate

(RS)-3-Ethoxycarbonyl-1-((S)-α-methylbenzyl)-5-oxopyrrolidine-3-carboxylic acid (30.5 g) was dissolved in 1,2-dichloroethane (92 ml), and thionyl chloride (11 ml) and one drop of N,N-dimethylformamide were added. The mixture was refluxed for 2 hours and the reaction mixture was concentrated under reduced pressure. The obtained oil was dissolved in toluene (39 ml) and the mixture was dropwise added to a solution of dibenzylamine (23.7 g) and triethylamine (12.2 g) in toluene (130 ml) under ice-cooling. The mixture was stirred at room temperature for 3 hours. The reaction mixture was washed with water, a sodium hydrogencarbonate-10% aqueous hydrochloric acid solution and water, and dried over anhydrous magnesium sulfate to give the object compound.

### Preparative Example 55

### (S)-N,N-Dibenzyl-3-hydroxymethyl-1-((S)-α-methylbenzyl)-5-oxopyrrolidine-3-carboxamide and (R)-N,N-dibenzyl-3-hydroxymethyl-1-((S)-α-methylbenzyl)-5-oxopyrrolidine-3-carboxamide

Ethyl (RS)-3-(N,N-dibenzylcarbamoyl)-1-((S)-α-methylbenzyl)-5-oxopyrrolidine-3-carboxylate (46 g) was dissolved in methanol (700 ml), and sodium borohydride (50 g) was portionwise added under water-cooling. The mixture was allowed to stand overnight at room temperature, and the reaction mixture was concentrated under reduced pressure. Ethyl acetate (200 ml) was added and the mixture was washed with water and dried over anhydrous magnesium sulfate. The solvent was distilled away to give a mixture of diastereomers. The mixture was separated by silica gel column chromatography using a mixed solvent of n-hexane:ethyl acetate (1:1) to give an (S)-isomer, melting point 140-142°C, [α] _{D}=-35.0° (c=1%, methanol), and an (R)-isomer, melting point 164-166°C, [α] _{D}=-86.9° (c=1%, methanol), respectively.

### Preparative Example 56

### (R)-N,N-Dibenzyl-3-fluoromethyl-1-((S)-α-methylbenzyl)-5-oxopyrrolidine-3-carboxamide

(S)-N,N-Dibenzyl-3-hydroxymethyl-1-((S)-α-methylbenzyl)-5-oxopyrrolidine-3-carboxamide (7.61 g) was dissolved in chloroform (80 ml) and hexafluoropropenediethylamine (7.85 g) was added. The mixture was refluxed under heating for 1 hour. After cooling, the reaction mixture was washed with water, dried over anhydrous magnesium sulfate and concentrated under reduced pressure. The obtained oil was purified by silica gel column chromatography using a mixed solvent of n-hexane:ethyl acetate = 2:1 as a developing solvent to give the object compound as an oil, melting point 85-89°C.
[α] _{D} =-31.6° (c=1%, methanol )

### Preparative Example 57

(R)-N,N-Dibenzyl-3-hydroxymethyl-1-((S)-α-methylbenzyl)-5-oxopyrrolidine-3-carboxamide was treated in the same manner as in Preparative Example 56 to give (S)-N,N-dibenzyl-3-fluoromethyl-1-((S)-α-methylbenzyl)-5-oxopyrrolidine-3-carboxamide, melting point 113-115°C.
[α] _{D} =-88.6° (c=1%, methanol)

### Preparative Example 58

### (S)-3-(N,N-Dibenzylaminomethyl)-3-fluoromethyl-1-((S)-α-methylbenzyl)pyrrolidine

Lithium aluminum hydride (1.23 g) was suspended in tetrahydrofuran (20 ml), and a solution of (R)-N,N-dibenzyl-3-fluoromethyl-1-((S)-α-methylbenzyl)-5-oxopyrrolidine-3-carboxamide (7.2 g) in tetrahydrofuran (20 ml) was dropwise added under water-cooling. The mixture was stirred at room temperature for 1 hour and refluxed under stirring for 3 hours. After cooling, a mixed solvent of water (1.23 ml) and tetrahydrofuran (5 ml) and 5 ml of an aqueous solution of sodium hydroxide (0.18 g) were dropwise added, and the mixture was allowed to stand overnight. The solution was filtered through Celite, and the filtrate was concentrated under reduced pressure and purified by silica gel column chromatography using a mixed solvent of n-hexane:ethyl acetate = 2:1 as a developing solvent to give the object compound as an oil.
[α] _{D} = -13.2° (c=1%, methanol)

### Preparative Example 59

### (R)-3-(N,N-Dibenzylaminomethyl)-3-fluoromethyl-1-((S)-α-methylbenzyl)pyrrolidine

An oily object compound was obtained from (R)-N,N-dibenzyl-3-fluoromethyl-1-((S)-α-methylbenzyl)-5-oxopyrrolidine-3-carboxamide in the same manner as in Preparative Example 58.
[α] _{D} =-44.8° (c=1%, methanol)

### Preparative Example 60

### (S)-3-Aminomethyl-3-fluoromethylpyrrolidine

(S)-3-(N,N-Dibenzylaminomethyl)-3-fluoromethyl-1-((S)-α-methylbenzyl)pyrrolidine (3.84 g) was dissolved in ethanol (40 ml) and 10% palladium-carbon (1.54 g) and hydrazine monohydrate (2.08 g) were added. The mixture was refluxed for 2 hours. The reaction mixture was filtered through Celite and the filtrate was concentrated under reduced pressure to give the object compound as an oil.
[α] _{D} = +7.8° (c=1%, methanol)

### Preparative Example 61

### (R)-3-Aminomethyl-3-fluoromethylpyrrolidine

An oily object compound was obtained from (R)-3-(N,N-dibenzylaminomethyl)-3-fluoromethyl-1-((S)-α-methylbenzyl)pyrrolidine in the same manner as in Preparative Example 60.
[α] _{D} =-7.9° (c=1%, methanol)

### Preparative Example 62

### (R)-3-Ethoxycarbonyl-1-((S)-α-methylbenzyl)-5-oxopyrrolidine-3-carboxylic acid

Acetone (340 ml) and a 0.05 M potassium phosphate buffer (pH 8.0, 3100 ml) were added to diethyl 1-((S)-α-methylbenzyl)-5-oxopyrrolidine-3,3-dicarboxylate (23 g) obtained in Preparative Example 52, and an esterase (6.5 ml, Sigma) was added. The mixture was stirred at 30°C for 7 hours. The reaction mixture was made acidic with dilute hydrochloric acid and the resulting mixture was extracted three times with chloroform (200 ml). The extract was concentrated under reduced pressure to give the object compound, melting point 137-139°C (decomposition).
[α] _{D} =-39.1° (c=1%, methanol)

### Preparative Example 63

### Ethyl (R)-3-(N,N-dibenzylcarbamoyl)-1-((S)-α-methylbenzyl)-5-oxopyrrolidine-3-carboxylate

The object compound was obtained in the same manner as in Preparative Example 54 from (R)-3-ethoxycarbonyl-1-((S)-α-methylbenzyl)-5-oxopyrrolidine-3-carboxylic acid obtained in Preparative Example 62.
[α] _{D} =-10.9° (c=1%, methanol)

### Preparative Example 64

### (S)-N,N-Dibenzyl-3-hydroxymethyl-1-((S)-α-methylbenzyl)-5-oxopyrrolidine-3-carboxamide

The object compound was obtained in the same manner as in Preparative Example 55 from ethyl (R)-3-(N,N-dibenzylcarbamoyl)-1-((S)-α-methylbenzyl)-5-oxopyrrolidine-3-carboxylate obtained in Preparative Example 63, melting point 135-137°C.
[α] _{D} =-35.9° (c=1%, methanol)

### Preparative Example 65

### Diethyl 1-((R)-α-methylbenzyl)-5-oxopyrrolidine-3,3-dicarboxylate

The object compound was obtained in the same manner as in Preparative Example 52 from D-(+)-α-methylbenzylamine.
[α] _{D} =+27.6° (c=1%, methanol)

### Preparative Example 66

### (R)-3-Ethoxycarbonyl-1-((R)-α-methylbenzyl)-5-oxopyrrolidine-3-carboxylic acid

The object compound was obtained in the same manner as in Preparative Example 62 from diethyl 1-((R)-α-methylbenzyl)-5-oxopyrrolidine-3,3-dicarboxylate obtained in Preparative Example 65, melting point 163°C (decomposition).
[α] _{D} =+39.7° (c=1%, methanol)

### Preparative Example 67

### (RS)-3-Ethoxycarbonyl-1-((R)-α-methylbenzyl)-5-oxopyrrolidine-3-carboxylic acid

The object compound was obtained in the same manner as in Preparative Example 53 from diethyl 1-(R)-α-methylbenzyl)-5-oxopyrrolidine-3,3-dicarboxylate obtained in Preparative Example 65.

### Preparative Example 68

### Ethyl (RS)-3-(N-benzyl-N-methylcarbamoyl)-1-((R)-α-methylbenzyl)-5-oxopyrrolidine-3-carboxylate

(RS)-3-Ethoxycarbonyl-1-((R)-α-methylbenzyl)-5-oxopyrrolidine-3-carboxylic acid (32.1 g) obtained in Preparative Example 67 was dissolved in chloroform (100 ml), and thionyl chloride (15 ml) and one drop of N,N-dimethylforimamide were added. The mixture was refluxed for 2 hours and the reaction mixture was concentrated under reduced pressure. The obtained oil was dissolved in toluene (39 ml), and the mixture was dropwise added to a solution of N-methylbenzylamine (15.3 g) and triethylamine (12.7 g) in toluene (130 ml) under ice-cooling. The mixture was refluxed for 2 hours. The reaction mixture was washed with water, sodium hydrogencarbonate, 10% aqueous hydrochloric acid and water, and dried over anhydrous magnesium sulfate to give the object compound.
NMR(CDCl₃,100MHz) δ : 7.40-6.72(m,10H), 5.46(q,1H), 4.88-4.19(m,2H), 4.04(q,2H), 3.34-2.82(m,4H), 2.68(s,3H), 1.58 and 1.57(d,3H), 1.20 and 1.06(t,3H)

### Preparative Example 69

### (R)-N-Benzyl-3-hydroxymethyl-N-methyl-1-((R)-α-methylbenzyl)-5-oxopyrrolidine-3-carboxamide and (S)-N-benzyl-3-hydroxymethyl-N-methyl-1-((R)-α-methylbenzyl)-5-oxopyrrolidine-3-carboxamide

Ethyl (RS)-3-(N-benzyl-N-methylcarbamoyl)-1-((R)-α-methylbenzyl)-5-oxopyrrolidine-3-carboxylate (39 g) obtained in Preparative Example 68 was dissolved in methanol (590 ml) and sodium borohydride (40 g) was portionwise added under water-cooling. The mixture was allowed to stand overnight at room temperature and the reaction mixture was concentrated under reduced pressure. Ethyl acetate (170 ml) was added to the concentrate, and the mixture was washed with water and dried over anhydrous magnesium sulfate. The solvent was distilled away to give a mixture of diastereomers. The mixture was separated and purified by silica gel column chromatorgaphy (developing solvent, chloroform : ethyl acetate = 1:1) to give (R)-N-benzyl-3-hydroxymethyl-N-methyl-1-((R)-α-methylbenzyl)-5-oxopyrrolidine-3-carboxamide ( [α] _{D} = +29.0° (c=1%, methanol)) and (S)-N-benzyl-3-hydroxymethyl-N-methyl-1-((R)-α-methylbenzyl)-5-oxopyrrolidine-3-carboxamide
( [α] _{D} = +79.2° (c=1%, methanol)).

### Preparative Example 70

### (S)-N-Benzyl-3-fluoromethyl-N-methyl-1-((R)-α-methylbenzyl)-5-oxopyrrolidine-3-carboxamide

(R)-N-Benzyl-3-hydroxymethyl-N-methyl-1-((R)-α-methylbenzyl)-5-oxopyrrolidine-3-carboxamide (11.5 g) was dissolved in chloroform (120 ml) and hexafluoropropenediethylamine (14.2 g) was added. The mixture was refluxed for 1.5 hours. After cooling, the reaction mixture was washed with water and sodium hydrogencarbonate, dried over anhydrous magnesium sulfate and concentrated under reduced pressure. The obtained oil was purified by silica gel column chromatography (developing solvent, chloroform : ethyl acetate = 1:1) to give the object compound as crystals, melting point 98-104°C.
[α] _{D} = +27.6° (c=1%, methanol)

### Preparative Example 71

### (R)-3-(N-Benzyl-N-methylaminomethyl)-3-fluoromethyl-1-((R)-α-methylbenzyl)pyrrolidine

A solution of (S)-N-benzyl-3-fluoromethyl-N-methyl-1-((R)-α-methylbenzyl)-5-oxopyrrolidine-3-carboxamide (5.37 g) in tetrahydrofuran (60 ml) was dropwise added to a solution of lithium aluminum hydride (1.1 g) in tetrahydrofuran (60 ml) at room temperature, and the mixture was stirred. The mixture was refluxed for 3 hours and cooled with ice. A mixture of tetrahydrofuran (12 ml) and water (1.2 ml) was dropwise added, and a mixture of 15% aqueous solution of sodium hydroxide (1.2 ml) and water (3.6 ml) was added, followed by stirring. The reaction mixture was filtered through Celite, and the filtrate was concentrated under reduced pressure and extracted with ethyl acetate. The extract was washed with a saturated aqueous solution of sodium chloride, dried over anhydrous magnesium sulfate, and concentrated under reduced pressure. The obtained oil was purified by silica gel column chromatography (developing solvent, n-hexane : ethyl acetate = 9:1) to give the object compound as a yellow oil.
[α] _{D} = +33.5 ° (c=1%, methanol)

### Preparative Example 72

### (R)-3-Fluoromethyl-3-methylaminomethylpyrrolidine

(R)-3-(N-Benzyl-N-methylaminomethyl)-3-fluoromethyl-1-((R)-α-methylbenzyl)pyrrolidine (2.05 g) was dissolved in ethanol (55 ml), and 10% palladium-carbon (0.8 g) and hydrazine monohydrate (0.90 g) were added at room temperature. The mixture was refluxed for 40 minutes. The reaction mixture was filtered through Celite, and the filtrate was concentrated under reduced pressure to give the object compound as a yellow oil.
[α] _{D} = -8.13° (c=1%, methanol)

### Preparative Example 73

### Ethyl (R)-3-benzylcarbamoyl-1-((S)-α-methylbenzyl)-5-oxopyrrolidine-3-carboxylate

(R)-3-Ethoxycarbonyl-1-((S)-α-methylbenzyl)-5-oxopyrrolidine-3-carboxylic acid (10 g) was dissolved in tetrahydrofuran (100 ml). Hydroxybenztriazole (0.89 g) was added and the mixture was stirred. Dicyclohexylcarbodiimide (6.76 g) and benzylamine (3.86 g) were added under ice-cooling, and the mixture was stirred at room temperature for 30 minutes. The reaction mixture was allowed to stand for 2 days and filtered through Celite. The filtrate was concentrated under reduced pressure. Ethyl acetate was added, and the mixture was washed with an aqueous solution of potassium carbonate, water, dilute hydrochloric acid and a saturated aqueous solution of sodium chloride, and dried over anhydrous magnesium sulfate. The obtained oil was purified by silica gel column chromatography (developing solvent, n-hexane : ethyl acetate = 4:1) to give the object compound as a pale yellow oil.
NMR(CDCl₃,100MHz) δ : 8.50-8.30(bs,1H), 7.30-7.20(m,10H), 5.30(q,1H), 4.40-4.30(m,2H), 4,10-3.90(m,3H), 3.70(ABq,1H), 3.00(ABq,2H), 1.50(d,3H), 1.05(t,3H)

### Preparative Example 74

### (S)-N-Benzyl-3-hydroxymethyl-1-((S)-α-methylbenzyl)-5-oxopyrrolidine-3-carboxamide

Ethyl (R)-3-benzylcarbamoyl-1-((S)-α-methylbenzyl)-5-oxopyrrolidine-3-carboxylate (12 g) was dissolved in methanol (120 ml) and sodium borohydride (10 g) was added under ice-cooling. The mixture was stirred for 2 hours under water-cooling. The mixture was allowed to stand at room temperature overnight and concentrated under reduced pressure. The residue was extracted with chloroform and dried over anhydrous magnesium sulfate. The obtained oil was purified by silica gel chromatography (developing solvent : chloroform). After crystallization, the resulting product was washed with isopropyl ether to give the object compound as colorless crystals, melting point 122-124°C.
[α] _{D} =-35.0° (c=1%, methanol)

### Preparative Example 75

### (R)-N-Benzyl-3-fluoromethyl-1-((S)-α-methylbenzyl)-5-oxopyrrolidine-3-carboxamide

(S)-N-Benzyl-3-hydroxymethyl-1-((S)-α-methylbenzyl)-5-oxopyrrolidine-3-carboxamide (11.46 g) was dissolved in chloroform (60 ml) and hexafluoropropenediethylamine (14.8 g) was added. The mixture was refluxed for 50 minutes. Ice water (100 ml) was added under water-cooling and the mixture was extracted twice with chloroform. The extract was dried over anhydrous sodium sulfate and concentrated under reduced pressure. The obtained oil was purified by silica gel chromatography (developing solvent : chloroform) to give the object compound as crystals, melting point 114-116°C.

### Preparative Example 76

### (R)-N-Benzyl-3-fluoromethyl-N-methyl-1-((S)-α-methylbenzyl)-5-oxopyrrolidine-3-carboxamide

A solution of (R)-N-benzyl-3-fluoromethyl-1-((S)-α-methylbenzyl)-5-oxopyrrolidine-3-carboxamide (3.72 g) in dimethylformamide (30 ml) was added to a solution of 60% sodium hydride (0.51 g) in dimethylformamide (20 ml) under ice-cooling, and the mixture was stirred at room temperature for 1 hour. Methyl iodide (0.7 ml) was added under ice-cooling, and the mixture was stirred at room temperature for 5.5 hours. Ice water (200 ml) was added to the reaction mixture, and the mixture was extracted twice with ethyl acetate, washed with saturated brine, and dried over anhydrous magnesium sulfate. The obtained oil was purified by silica gel column chromatography (developing solvent : chloroform) to give the object compound as crystals, melting point 95-98°C.
[α] _{D} = -28.8° (c=1%, methanol)

### Preparative Example 77

### (S)-3-(N-Benzyl-N-methylaminomethyl)-3-fluoromethyl-1-((S)-α-methylbenzyl)pyrrolidine

The object compound was obtained as an oil, in the same manner as in Preparative Example 71 from (R)-N-benzyl-3-fluoromethyl-N-methyl-1-((S)-α-methylbenzyl)-5-oxopyrrolidine-3-carboxamide.
[α] _{D} = -35.6° (c=1%, methanol)

### Preparative Example 78

### (S)-3-Fluoromethyl-3-methylaminomethylpyrrolidine

The object compound was obtained as a yellow oil, in the same manner as in Preparative Example 72 from (S)-3-(N-benzyl-N-methylaminomethyl)-3-fluoromethyl-1-((S)-α-methylbenzyl)pyrrolidine.
[α] _{D} = +7.47° (c=1%, methanol)

### Preparative Example 79

### (S)-3-(N,N-Dibenzylaminomethyl)-3-fluoromethyl-1-((S)-α-methylbenzyl)pyrrolidine

(R)-N,N-Dibenzyl-3-fluoromethyl-1-((S)-α-methylbenzyl)-5-oxopyrrolidine-3-carboxamide (14 g) obtained in Preparative Example 56 was dissolved in tetrahydrofuran (40 ml) and the solution was added to a 1N borane-tetrahydrofuran solution (126 ml). The mixture was refluxed for 4 hours. A 3N hydrochloric acid solution (48 ml) was dropwise added to the reaction mixture under ice-cooling, and the mixture was refluxed for 2 hours with stirring. The reaction mixture was made alkaline with an aqueous solution of sodium hydroxide, and extracted with ethyl acetate. The extract was washed with water, dried, concentrated and purified by silica gel column chromatography to give the object compound as an oil.

### Preparative Example 80

(S)-N,N-Dibenzyl-3-fluoromethyl-1-((S)-α-methylbenzyl)-5-oxopyrrolidine-3-carboxamide obtained in Preparative Example 57 was treated in the same manner as in Preparative Example 79 to give (R)-3-(N,N-dibenzylaminomethyl)-3-fluoromethyl-1-((S)-α-methylbenzyl)pyrrolidine.

### Preparative Example 81

### (S)-3-(N,N-dibenzylaminomethyl)-3-fluoromethyl-1-((S)-α-methylbenzyl)pyrrolidine

Sodium borohydride (12.2 g) was added to a solution of (R)-N,N-dibenzyl-3-fluoromethyl-1-((S)-α-methylbenzyl)-5-oxopyrrolidine-3-carboxamide (28.6 g) obtained in Preparative Example 56 in tetrahydrofuran (300 ml), and a solution of conc. sulfuric acid (8.6 ml) in tetrahydrofuran (100 ml) was dropwise added at not more than 5°C over 1 hour. 3N Hydrochloric acid (78 ml) was dropwise added under ice-cooling and the mixture was refluxed for 1 hour with stirring. The reaction mixture was made alkaline by an aqueous solution of sodium hydroxide, and extracted with ethyl acetate, washed with water, dried, concentrated, and purified by silica gel column chromatography to give the object compound as an oil.

### Preparative Example 82

(S)-N,N-Dibenzyl-3-fluoromethyl-1-((S)-α-methylbenzyl)-5-oxopyrrolidine-3-carboxamide obtained in Preparative Example 57 was treated in the same manner as in Preparative Example 81 to give (R)-3-(N,N-dibenzylaminomethyl)-3-fluoromethyl-1-((S)-α-methylbenzyl)pyrrolidine.

### Preparative Example 83

### (1) (S)-3-(N,N-Dibenzylaminomethyl)-1-ethoxycarbonyl-3-fluoromethylpyrrolidine

(S)-3-(N,N-Dibenzylaminomethyl)-3-fluoromethyl-1-((S)-α-methylbenzyl)pyrrolidine (62 g) obtained in Preparative Example 58 was dissolved in toluene (620 ml), and ethyl chloroformate (40 ml) was added. The mixture was stirred at 75-80°C for 5 hours. The insoluble matter was removed, and the reaction mixture was sequentially washed with an aqueous solution of potassium carbonate, water, a 1N aqueous solution of citric acid and water, dried, and concentrated to give the object compound as an oil. NMR(CDCl₃ ) δ : 1.22(t, 3H), 1.5-1.7(m, 2H), 2.62(s, 2H), 2.8-3.4(m,4H), 3.59(ABq,4H), 4.07(q, 2H), 4.30(d,2H), 7.15-7.35(m,10H)

### (2) (R)-3-(N,N-Dibenzylaminomethyl)-3-fluoromethylpyrrolidine

(S)-3-(N,N-Dibenzylaminomethyl)-1-ethoxycarbonyl-3-fluoromethylpyrrolidine (52 g) was dissolved in isopropyl alcohol (500 ml), and sodium hydroxide (27 g) was added. The mixture was refluxed for 40 hours with stirring. After concentration, water (300 ml) was added to the concentrate and the mixture was extracted with ethyl acetate. The organic layer was washed with water, dried and concentrated to give the object compound as an oil.
NMR(CDCl₃) δ : 1.40-1.60(m,2H), 2.23(bs,1H), 2.50-2.90(m,5H), 3.60(s,4H), 4.34(d,2H), 7.15-7.35(m,10H)

### Preparative Example 84

(1) (R)-3-(N,N-Dibenzylaminomethyl)-3-fluoromethyl-1-((S)-α-methylbenzyl)pyrrolidine obtained in Preparative Example 59 was treated in the same manner as in Preparative Example 83 (1) to give (R)-3-(N,N-dibenzylaminomethyl)-1-ethoxycarbonyl-3-fluoromethylpyrrolidine.
   NMR(CDCl₃) δ : 1.21(t,3H), 1.55-1.75(m,2H), 2,61(s,2H), 2.80-3.40(m,4H), 3.58(ABq,4H), 4.06(q,2H), 4.30(d,2H), 7.15-7.35(m,10H)
(2) (R)-3-(N,N-Dibenzylaminomethyl)-1-ethoxycarbonyl-3-fluoromethylpyrrolidine was treated in the same manner as in Preparative Example 83 (2) to give (S)-3-(N,N-dibenzylaminomethyl)-3-fluoromethylpyrrolidine.
   NMR(CDCl₃) δ : 1.40-1.60(m,2H), 2.35(s,1H)), 2.55-2.9(m,5H)), 3.60(s,4H), 4.33(d,2H), 7.15-7.35(m,10H)

### Preparative Example 85

### (1) (R)-3-(N,N-Dibenzylcarbamoyl)-1-((S)-α-methylbenzyl)-5-oxopyrrolidine-3-carboxylic acid

Ethyl (R)-3-(N,N-dibenzylcarbamoyl)-1-((S)-α-methylbenzyl)-5-oxopyrrolidine-3-carboxylate (48.5 g) obtained in Preparative Example 63 was dissolved in methanol (250 ml), and a solution of sodium hydroxide (16 g) in water (125 ml) was added. The mixture was stirred at 40°C for 2 hours. After concentration, the reaction mixture was washed with toluene, made acidic with 6N hydrochloric acid and extracted with chloroform. The extract was washed with water, dried, concentrated, and crystallized from isopropyl ether. The crystals were collected by filtration to give the object compound, melting point 138-139°C.

### (2) (S)-3-N,N-Dibenzyl-3-hydroxymethyl-1-((S)-α-methylbenzyl)-5-oxopyrrolidine-3-carboxamide

N,N-Dimethylformamide (877 mg) was dissolved in dichloromethane (20 ml), and oxalyl chloride (2.93 ml) was dropwise added under ice-cooling. The mixture was stirred at room temperature for 2 hours. After concentration, acetonitrile (10 ml) and tetrahydrofuran (20 ml) were added, and a solution of (R)-3-(N,N-dibenzylcarbamoyl)-1-((S)-α-methylbenzyl)-5-oxopyrrolidine-3-carboxylic acid (4.57 g) in tetrahydrofuran (10 ml) was dropwise added under ice-cooling. The mixture was stirred at said temperature for 1 hour. A solution of sodium borohydride (908 mg) in N,N-dimethylformamide (25 ml) was dropwise added under ice-cooling, and the mixture was stirred at said temperature for 1.5 hours. A 2N hydrochloric acid (20 ml) was added, and the mixture was extracted with ethyl acetate. The extract was washed with water, dried, concentrated and crystallized from isopropyl ether. The crystals were collected by filtration to give the object compound.

### Preparative Example 86

1-Benzyl-3-(N,N-dibenzylcarbamoyl)-5-oxopyrrolidine-3-carboxylic acid obtained in Preparative Example 51 (1) was treated in the same manner as in Preparative Example 85 (2) to give 1-benzyl-3-N,N-dibenzyl-3-hydroxymethyl-5-oxopyrrolidine-3-carboxamide, melting point 127-129°C.

The following compounds can be obtained by any of Preparative Examples mentioned above.
○1-Benzyl-3-dimethylaminomethyl-3-hydroxymethylpyrrolidine
NMR(CDCl₃ ) δ : 1.2-1.8(m, 2H), 2.1-2.4(m, 2H), 2.2(s, 6H), 2.5(s, 2H), 2.6-2.9(m, 2H), 3.5-3.6(m, 4H), 5.3(bs, 1H), 7.2-7.3(m, 5H)
○1-Benzyl-3-dimethylaminomethyl-3-fluoromethylpyrrolidine
NMR(CDCl₃ ) δ : 1.5-1.7(m, 2H), 2.1-2.7(m, 6H), 2.25(s, 6H), 3.55(s, 2H), 4.1(d,1H), 4.55(d, 1H), 7.2-7.3(m, 5H)
○1-Benzyl-3-cyclopropylaminomethyl-3-hydroxymethylpyrrolidine
NMR(CDCl₃ ) δ : 0.3-0.5(m,4H), 1.3-1.8(m,2H), 1.9-2.1(m,1H), 2.2-2.8(m,4H), 2.9(s,2H), 3.6(s,4H), 7.1-7.3(m,5H)
○Ethyl 1-benzyl-3-(dimethylcarbamoyl)-5-oxopyrrolidine-3-carboxylate
melting point 95-97°C
○Ethyl 1-benzyl-3-(N-benzyl-N-ethylcarbamoyl)-5-oxopyrrolidine-3-carboxylate
NMR(CDCl₃ ) δ : 1.16(t, 3H), 1.26(t, 3H), 3.12(s, 2H), 3.55(d, 1H), 3.9-4.8(m, 9H), 7.2-7.3(m, 10H)
○Ethyl 1-benzyl-3-(cyclopropylcarbamoyl)-5-oxopyrrolidine-3-carboxylate
NMR(CDCl₃) δ : 0.3-0.5(d, 2H), 0.6-0.8(m, 2H), 1.2(t, 3H), 2.5-2.8(m, 1H), 3.05(d, 2H), 3.65(d, 2H), 4.15(q, 2H), 4.4(d, 2H), 6.4(bs, 1H), 7.1-7.3(m, 5H)
○Ethyl 1-benzyl-3-(N-benzyl-N-cyclopropylcarbamoyl)-5-oxopyrrolidine-3-carboxylate
NMR(CDCl₃ ) δ : 0.6-0.8(d, 4H), 1.2(t, 3H), 2.2-2.6(m, 1H), 3.15(s, 2H), 3.6(d, 1H), 3.9-4.2(m, 3H), 4.3-4.7(m 4H), 7.0-7.3(m, 10H)

### Preparative Example 87

### 7-(3-Aminomethyl-3-hydroxymethylpyrrolidin-1-yl)-1-cyclopropyl-6-fluoro-1,4-dihydro-8-methoxy-4-oxo-3-quinolinecarboxylic acid

3-Aminomethyl-3-hydroxymethylpyrrolidine (0.78 g) obtained in Preparative Example 42 (4) and 1-cyclopropyl-6,7-difluoro-1,4-dihydro-8-methoxy-4-oxo-3-quinolinecarboxylic acid-BF₂ chelate (0.82 g) were dissolved in dimethyl sulfoxide (10 ml), and the mixture was stirred at room temperature overnight. The solvent was distilled away under reduced pressure, and methanol (15 ml) and triethylamine (1 ml) were added. The mixture was refluxed for 2 hours. The solvent was distilled away under reduced pressure and ethanol was added to allow crystallization. The crystals were recrystallized from ethanol - ammonia to give 0.57 g of the object compound, melting point 198-200°C.

### Preparative Example 88

### 7-(3,3-Bis(hydroxymethyl)pyrrolidin-1-yl)-1-cyclopropyl-6-fluoro-1,4-dihydro-8-methoxy-4-oxo-3-quinolinecarboxylic acid

3,3-Bis(hydroxymethyl)pyrrolidine (0.61 g) obtained in Preparative Example 46 (2) and 1-cyclopropyl-6,7-difluoro-1,4-dihydro-8-methoxy-4-oxo-3-quinolinecarboxylic acid-BF₂ chelate (0.62 g) were treated in the same manner as in Preparative Example 87 to give 0.44 g of the object compound, melting point 255-257°C.

### Example 7

### 7-(3-Aminomethyl-3-fluoromethylpyrrolidin-1-yl)-1-cyclopropyl-6-fluoro-1,4-dihydro-8-methoxy-4-oxo-3-quinolinecarboxylic acid

3-Aminomethyl-3-fluoromethylpyrrolidine (0.660 g) obtained in Preparative Example 43 (5) and 1-cyclopropyl-6,7-difluoro-1,4-dihydro-8-methoxy-4-oxo-3-quinolinecarboxylic acid-BF₂ chelate (0.69 g) were dissolved in acetonitrile (5 ml), and the mixture was stirred at room temperature overnight. The solvent was distilled away under reduced pressure and ethanol was added to allow crystallization. Methanol (80 ml) and triethylamine (5 ml) were added to the obtained crystals. The mixture was refluxed for 2 hours. The solvent was distilled away under reduced pressure and ethanol was added to allow crystallization. The crystals were recrystallized from ethanol to give 0.38 g of the object compound, melting point 192-194°C.

### Example 8

### Methyl 7-(3-aminomethyl-3-fluoromethylpyrrolidin-1-yl)-1-cyclopropyl-6-fluoro-1,4-dihydro-8-methoxy-4-oxo-3-quinolinecarboxylate

7-(3-Aminomethyl-3-fluoromethylpyrrolidin-1-yl)-1-cyclopropyl-6-fluoro-1,4-dihydro-8-methoxy-4-oxo-3-quinolinecarboxylic acid (8.9 g) obtained in Example 7 was suspended in methanol (100 ml) and thionyl chloride (3.2 ml) was dropwise added with stirring under ice-cooling. After the dropwise addition, the mixture was refluxed overnight. Methanol and excess thionyl chloride were distilled away under reduced pressure. The residue was dissolved in water and the solution was made alkaline with potassium carbonate. The mixture was extracted twice with chloroform, dried and concentrated to dryness to give 9.8 g of the object compound, melting point 143-145°C.

### Example 9

### (1) Methyl (S)-(+)-7-(3-aminomethyl-3-fluoromethylpyrrolidin-1-yl)-1-cyclopropyl-6-fluoro-1,4-dihydro-8-methoxy-4-oxo-3-quinolinecarboxylate

A solution of (R)-O-methylmandelic acid (2.2 g) in methanol (8 ml) was added to a solution of the compound (8.1 g) obtained in Example 8 in methanol (32 ml), and the mixture was left standing at room temperature for 3 days. The precipitated crystals were collected by filtration and recrystallized 4 times from methanol. The obtained crystals were suspended in water, and the suspension was made alkaline with potassium carbonate and extracted with chloroform. The extract was washed with water, dried and concentrated to give 1.1 g of the object compound, melting point 150-152°C, optical purity not less than 95% ee (HPLC).

### (2) (S)-(+)-7-(3-Aminomethyl-3-fluoromethylpyrrolidin-1-yl)-1-cyclopropyl-6-fluoro-1,4-dihydro-8-methoxy-4-oxo-3-quinolinecarboxylic acid

An optically active ester (1.1 g) obtained in (1) was dissolved in methanol (10 ml), and an aqueous solution (2 ml) of potassium hydroxide (0.22 g) was added. The mixture was stirred at room temperature overnight. The solvent was distilled away under reduced pressure. The residue was dissolved in water and its pH was adjusted to 7 with acetic acid. The mixture was extracted with chloroform, dried and concentrated. The residue was recrystallized from ethanol to give 0.42 g of the object compound as white crystals, melting point 186-188°C.
[α] _{D} = +40.1° (1.50% methanol - chloroform), optical purity not less than 95% ee (HPLC)

### Example 10

### (1) Methyl (R)-(-)-7-(3-aminomethyl-3-fluoromethylpyrrolidin-1-yl)-1-cyclopropyl-6-fluoro-1,4-dihydro-8-methoxy-4-oxo-3-quinolinecarboxylate

The ester recovered from the mother liquor obtained in Example 9 (1) was dissolved in methanol (28 ml), and a solution of (S)-O-methylmandelic acid (1.9 g) in methanol (7 ml) was added. The mixture was left standing at room temperature overnight. The precipitated crystals were collected by filtration and recrystallized 3 times from methanol. The obtained crystals were suspended in water, and the suspension was made alkaline with potassium carbonate. The mixture was extracted with chloroform. The extract was washed with water, dried and concentrated to give 1.1 g of the object compound, melting point 151-152°C, optical purity not less than 95% ee (HPLC).

### (2) (R)-(-)-7-(3-Aminomethyl-3-fluoromethylpyrrolidin-1-yl)-1-cyclopropyl-6-fluoro-1,4-dihydro-8-methoxy-4-oxo-3-quinolinecarboxylic acid

The optically active ester (1.1 g) obtained in (1) was reacted in the same manner as in Example 9 (2) to give 0.55 g of the object compound as white crystals, melting point 187-189°C.
[α] _{D} = -39.4° (1.50% methanol - chloroform), optical purity not less than 95% ee (HPLC)

### Example 11

### (S)-(+)-7-(3-Aminomethyl-3-fluoromethylpyrrolidin-1-yl)-1-cyclopropyl-6-fluoro-1,4-dihydro-8-methoxy-4-oxo-3-quinolinecarboxylic acid

(S)-3-Aminomethyl-3-fluoromethylpyrrolidine (0.88 g) obtained in Preparative Example 60, triethylamine (0.45 g) and 1-cyclopropyl-6,7-difluoro-1,4-dihydro-8-methoxy-4-oxo-3-quinolinecarboxylic acid-BF₂ chelate (1.52 g) were treated in the same manner as in Example 7 and recrystallized from chloroform - ethanol to give the object compound, melting point 188-190°C.
[α] _{D} = +24.9° (c=1%, acetic acid)

### Example 12

### (R)-(-)-7-(3-Aminomethyl-3-fluoromethylpyrrolidin-1-yl)-1-cyclopropyl-6-fluoro-1,4-dihydro-8-methoxy-4-oxo-3-quinolinecarboxylic acid

(R)-3-Aminomethyl-3-fluoromethylpyrrolidine (0.88 g) obtained in Preparative Example 61, triethylamine (0.45 g) and 1-cyclopropyl-6,7-difluoro-1,4-dihydro-8-methoxy-4-oxo-3-quinolinecarboxylic acid-BF₂ chelate (1.52 g) were treated in the same manner as in Example 7 and recrystallized from chloroform - ethanol to give the object compound, melting point 189-191°C.
[α] _{D} = -25.3° (c=1%, acetic acid)

### Example 13

### 1-Cyclopropyl-6-fluoro-7-(3-fluoromethyl-3-methylaminomethylpyrrolidin-1-yl)-1,4-dihydro-8-methoxy-4-oxo-3-quinolinecarboxylic acid

3-Fluoromethyl-3-methylaminomethylpyrrolidine (1.46 g) obtained in Preparative Example 45 (2) and 1-cyclopropyl-6,7-difluoro-1,4-dihydro-8-methoxy-4-oxo-3-quinolinecarboxylic acid-BF₂ chelate (1.37 g) were treated in the same manner as in Example 7 and recrystallized from ethanol - ammonia to give 0.51 g of the object compound, melting point 208-210°C.

### Preparative Example 89

### 1-Cyclopropyl-6-fluoro-7-(3-fluoromethyl-3-methylaminomethylpiperidin-1-yl)-1,4-dihydro-8-methoxy-4-oxo-3-quinolinecarboxylic acid

3-Fluoromethyl-3-methylaminomethylpiperidine obtained in Preparative Example 47 (6) and 1-cyclopropyl-6,7-difluoro-1,4-dihydro-8-methoxy-4-oxo-3-quinolinecarboxylic acid-BF₂ chelate (0.44 g) were treated in the same manner as in Example 7 and recrystallized from chloroform - ethanol to give 0.20 g of the object compound, melting point 135-138°C.

### Preparative Example 90

### 7-(3-Aminomethyl-3-fluoromethylpiperidin-1-yl)-1-cyclopropyl-6-fluoro-1,4-dihydro-8-methoxy-4-oxo-3-quinolinecarboxylic acid

3-Aminomethyl-3-fluoromethylpiperidine obtained in Preparative Example 48 (5) and 1-cyclopropyl-6,7-difluoro-1,4-dihydro-8-methoxy-4-oxo-3-quinolinecarboxylic acid-BF₂ chelate (1.70 g) were treated in the same manner as in Example 7 and purified by silica gel column chromatography to give 1.20 g of the pale-yellow, amorphous object compound.
NMR(DMSO-d₆) δ : 0.60-1.30(m, 4H), 1.32-1.88(m, 4H), 2.86(s, 2H), 3.04-3.34(m, 4H), 3.68(s, 3H), 3.96-4.26(m, 1H), 4.56(d, 2H), 7.66(d, 1H), 8.64(s, 1H)

### Example 14

### (R)-(-)-1-Cyclopropyl-6-fluoro-7-(3-fluoromethyl-3-methylaminomethylpyrrolidin-1-yl)-1,4-dihydro-8-methoxy-4-oxo-3-quinolinecarboxylic acid

(R)-3-Fluoromethyl-3-methylaminomethylpyrrolidine (0.97 g) obtained in Preparative Example 72 was dissolved in acetonitrile (26 ml), and triethylamine (0.62 ml) and 1-cyclopropyl-6,7-difluoro-1,4-dihydro-8-methoxy-4-oxo-3-quinolinecarboxylic acid-BF₂ chelate (1.52 g) were added. The mixture was treated in the same manner as in Example 7 and recrystallized from ethanol - aqueous ammonia to give the object compound as colorless crystals, melting point 156-157°C.
[α] _{D} = -51.9° (c=1%, acetic acid)

### Example 15

### (S)-(+)-1-Cyclopropyl-6-fluoro-7-(3-fluoromethyl-3-methylaminomethylpyrrolidin-1-yl)-1,4-dihydro-8-methoxy-4-oxo-3-quinolinecarboxylic acid

(S)-3-Fluoromethyl-3-methylaminomethylpyrrolidine obtained in Preparative Example 78 was treated in the same manner as in Example 13 to give the object compound as colorless crystals, melting point 150-152°C.
[α] _{D} = +59.3° (c=1%, chloroform:methanol=1:1)

### Example 16

### (1) (S)-1-Cyclopropyl-7-(3-(N,N-dibenzylaminomethyl)-3-fluoromethylpyrrolidin-1-yl)-6-fluoro-1,4-dihydro-8-methoxy-4-oxo-3-quinolinecarboxylic acid

A mixture of (R)-3-(N,N-dibenzylaminomethyl)-3-fluoromethylpyrrolidine (23 g) obtained in Preparative Example 83 (2), 1-cyclopropyl-6,7-difluoro-1,4-dihydro-8-methoxy-4-oxo-3-quinolinecarboxylic acid-BF₂ chelate (21 g), triethylamine (6.5 g) and acetonitrile (200 ml) was stirred for 14 hours. After concentration, ethanol was added and the obtained crystals were collected by filtration. Methanol (200 ml), chloroform (100 ml) and triethylamine (50 ml) were added to the crystals and the mixture was refluxed for 4 hours. After concentration, ethanol was added and the obtained crystals were collected by filtration to give the object compound.

### (2) (S)-7-(3-Aminomethyl-3-fluoromethylpyrrolidin-1-yl)-1-cyclopropyl-6-fluoro-1,4-dihydro-8-methoxy-4-oxo-3-quinolinecarboxylic acid

(S)-1-Cyclopropyl-7-(3-(N, N-dibenzylaminomethyl)-3-fluoromethylpyrrolidin-1-yl)-6-fluoro-1,4-dihydro-8-methoxy-4-oxo-3-quinolinecarboxylic acid (19.58 g) was dissolved in ethanol (300 ml), and 10% palladium-carbon (3.9 g) and hydrazine hydrate (5.0 g) were added. The mixture was refluxed with stirring for 4 hours. The precipitated crystals were dissolved in a 2N aqueous solution of sodium hydroxide. The mixture was filtrated to remove the catalyst. After concentration, water (100 ml) was added and the pH of the solution was adjusted to 7 with dilute hydrochloric acid. The precipitated crystals were collected by filtration to give the object compound, melting point 188-190°C.

### Example 17

### (S)-7-(3-Aminomethyl-3-fluoromethylpyrrolidin-1-yl)-1-cyclopropyl-6-fluoro-1,4-dihydro-8-methoxy-4-oxo-3-quinolinecarboxylic acid

(S)-1-Cyclopropyl-7-(3-(N, N-dibenzylaminomethyl)-3-fluoromethylpyrrolidin-1-yl)-6-fluoro-1,4-dihydro-8-methoxy-4-oxo-3-quinolinecarboxylic acid (7.51 g) obtained in Example 16 (1) was suspended in ethanol (40 ml) and methanol (40 ml). 6N Hydrochloric acid (4.3 ml) and 10% palladium-carbon (1.5 g) were added and hydrogen was blown thereto at 40°C. After the completion of the reaction, the catalyst was removed by filtration and the filtrate was concentrated. Acetone (170 ml) was added and the precipitated hydrochloride of the object compound was collected by filtration as crystals, melting point 213-217°C.

The obtained hydrochloride was dissolved in water (100 ml) and the pH of the solution was adjusted to 7 with a 4N aqueous solution of sodium hydroxide. The precipitated crystals were collected by filtration to give the object compound, melting point 196-198°C.

### Example 18

### (1) (S)-1-Cyclopropyl-7-(3-(N,N-dibenzylaminomethyl)-3-fluoromethylpyrrolidin-1-yl)-6,8-difluoro-1,4-dihydro-4-oxo-3-quinolinecarboxylic acid

1-Cyclopropyl-6,7,8-trifluoro-1,4-dihydro-4-oxo-3-quinolinecarboxylic acid (5 g), (R)-3-N,N-dibenzylaminomethyl-3-fluoromethylpyrrolidine (6.6 g) and triethylamine (2.0 g) were dissolved in acetonitrile (75 ml), and the mixture was stirred for 7.5 hours. After cooling, the precipitated crystals were collected by filtration to give the object compound as pale-yellow crystals, melting point 185-186°C.
[α] _{D} = +4.3° (c=1%, chloroform)

### (2) (S)-1-Cyclopropyl-7-(3-(N,N-dibenzylaminomethyl)-3-fluoromethylpyrrolidin-1-yl)-6-fluoro-1,4-dihydro-8-methoxy-4-oxo-3-quinolinecarboxylic acid

Dimethylacetamide was added to (S)-1-cyclopropyl-7-(3-(N,N-dibenzylaminomethyl)-3-fluoromethylpyrrolidin-1-yl)-6,8-difluoro-1,4-dihydro-4-oxo-3-quinolinecarboxylic acid (3 g) and sodium methoxide (1.4 g), and the mixture was stirred at 100°C for 2 hours. After cooling, water was added and the pH of the solution was adjusted to 6.0 with acetic acid. The mixture was extracted with ethyl acetate. The extract was washed with water, dried, concentrated and crystallized from isopropyl ether - ethanol. The crystals were collected by filtration. The crystals were recrystallized from ethanol to give the object compound, melting point 132-133°C.
[α] _{D} = +42.0° (c=1%, chloroform)

### Example 19

### (1) Ethyl (S)-4-(3-(N,N-dibenzylaminomethyl)-3-fluoromethylpyrrolidin-1-yl)-2,3,5-trifluorobenzoylacetate

Ethyl 2,3,4,5-tetrafluorobenzoylacetate (13.2 g), (R)-3-(N,N-dibenzylaminomethyl)-3-fluoromethylpyrrolidine (18.2 g) and triethylamine (5.6 g) were dissolved in dimethyl sulfoxide (54 ml), and the mixture was stirred at 100°C for 5 hours. After cooling, water was added and the mixture was extracted with ethyl acetate. The extract was washed with water, dried and concentrated. The obtained oil was purified by silica gel column chromatography (developing solvent; chloroform:methanol=50:1) to give the object compound as a yellow oil.
[α] _{D} = -10.5° (c=1%, chloroform)
NMR(CDCl₃) δ : 1.36(t,3H), 1.70(t,3H), 2.65(s,2H), 3.55(m,4H), 3.60(s.4H)), 3.86(d,2H), 4.00-4.38(m,3H), 4.63(s,1H), 7.25(m,11H)

### (2) Ethyl (S)-2-(4-(3-(N,N-dibenzylaminomethyl)-3-fluoromethylpyrrolidin-1-yl)-2,3,5-trifluorobenzoyl)-3-cyclopropylaminoacrylate

Ethyl (S)-4-(3-(N,N-dibenzylaminomethyl)-3-fluoromethylpyrrolidin-1-yl)-2,3,5-trifluorobenzoylacetate (5.2 g) and dimethylformamide dimethyl acetal (2.5 ml) were dissolved in dry toluene (30 ml), and the mixture was refluxed for 8 hours. After concentration, the residue was dissolved in dry ethanol (15 ml), and cyclopropylamine (0.53 g) was added. The mixture was stirred at room temperature for 4.5 hours and concentrated. The obtained oil was purified by silica gel column chromatography (developing solvent; chloroform:methanol=100:1) to give the object compound as a pale-yellow oil.
[α] _{D} = +15.9° (c=1%, chloroform)
NMR(CDCl₃) δ : 0.83(m,4H), 1.00-1.13(t,3H), 1.66(m,2H), 2.69(s,1H), 2.93(m,1H), 3.20-3.55(m,4H), 3.60(s,4H), 4.08(m,2H), 4.35(dd,1H), 4.45(dd,1H), 6.78-7.05(m,1H), 7.26(m,10H), 7.95-8.15(d,1H)

### (3) Ethyl (S)-1-cyclopropyl-7-(3-(N,N-dibenzylaminomethyl)-3-fluoromethylpyrrolidin-1-yl)-6,8-difluoro-1,4-dihydro-4-oxo-3-quinolinecarboxylate

Ethyl (S)-2-(4-(3-(N,N-dibenzylaminomethyl)-3-fluoromethylpyrrolidin-1-yl)-2,3,5-trifluorobenzoyl)-3-cyclopropylaminoacrylate (0.3 g) was dissolved in dry dimethylformamide (1.5 ml), and potassium carbonate (0.07 g) was added. The mixture was stirred at 50°C for 7.5 hours. After cooling, water was added and the mixture was extracted with ethyl acetate. The extract was washed with water, dried and concentrated. The obtained oil was purified by silica gel column chromatography (developing solvent; chloroform:methanol=50:1) to give the object compound as a pale-yellow oil.
[α] _{D} = +1.93° (c=1%, chloroform)
NMR(CDCl₃) δ : 1.05(bs,2H), 1.16(d,2H), 1.40(t,3H), 1.73(m,2H), 2.70(s,1H), 3.26-3.58(m,4H), 3.63(q,4H), 3.82(m,1H), 4.37(q,3H), 4.50(q,1H), 7.10-7.36(m,11H), 7.80(d,1H), 8.50(s,1H)

### (4) (S)-1-Cyclopropyl-7-(3-(N,N-dibenzylaminomethyl)-3-fluoromethylpyrrolidin-1-yl)-6,8-difluoro-1,4-dihydro-4-oxo-3-quinolinecarboxylic acid

Ethyl (S)-1-cyclopropyl-7-(3-(N,N-dibenzylaminomethyl)-3-fluoromethylpyrrolidin-1-yl)-6,8-difluoro-1,4-dihydro-4-oxo-3-quinolinecarboxylate (7.5 g) was dissolved in ethanol (100 ml), and sodium hydroxide (0.62 g) dissolved in water (10 ml) was added. The mixture was stirred at 50°C for 2 hours. After concentration, water was added and the pH of the solution was adjusted to 6.5 with dil. hydrochloric acid. The precipitated crystals were collected by filtration and washed with heated ethanol. The obtained crystals were recrystallized from N,N-dimethylformamide to give the object compound as pale-yellow crystals, melting point 184-186°C.
[α] _{D} = +4.8° (c=1%, chloroform)
The compound obtained is treated in the same manner as in Example 18 (2) to convert same to an 8-methoxy compound.

### Example 20

### (1) (S)-4-(3-(N,N-Dibenzylaminomethyl)-3-fluoromethylpyrrolidin-1-yl)-2,3,5-trifluorobenzoic acid

2,3,4,5-Tetrafluorobenzoylbenzoic acid (10 g), (R)-3-(N,N-dibenzylaminomethyl)-3-fluoromethylpyrrolidine (19.3 g) and triethylamine (5.8 g) were dissolved in dimethyl sulfoxide (25 ml), and the mixture was stirred with heating at 110°C for 6 hours. After cooling, water was added and the mixture was extracted with ethyl acetate. The extract was washed with water, dried and concentrated. The obtained oil was purified by silica gel column chromatography (developing solvent; chloroform: methanol=30:1) to give crystals. The crystals were recrystallized from aqueous ethanol to give the object compound as white powdery crystals, melting point 140-145°C.
[α] _{D} = -10.9° (c=1%, chloroform)

### (2) (S)-4-(3-(N,N-Dibenzylaminomethyl)-3-fluoromethylpyrrolidin-1-yl)-2,3,5-trifluorobenzoyl chloride hydrochloride

(S)-4-(3-(N,N-Dibenzylaminomethyl)-3-fluoromethylpyrrolidin-1-yl)-2,3,5-trifluorobenzoic acid (12.6 g) was dissolved in dry 1,2-dichloroethane (63 ml), and two drops of dry dimethylformamide were added. Thionyl chloride (2.3 ml) was added at room temperature and the mixture was refluxed for 2 hours. The reaction mixture was concentrated to give the object compound as pale-gray crystals, melting point 170-173°C (decomposition).

### (3) Ethyl (S)-1-cyclopropyl-7-(3-(N,N-dibenzylaminomethyl)-3-fluoromethylpyrrolidin-1-yl)-6,8-difluoro-1,4-dihydro-4-oxo-3-quinolinecarboxylate

(S)-4-(3-(N,N-Dibenzylaminomethyl)-3-fluoromethylpyrrolidin-1-yl)-2,3,5-trifluorobenzoyl chloride hydrochloride (0.97 g) was dissolved in acetonitrile (8 ml). Ethyl N,N-dimethylaminoacrylate (0.27 g) and triethylamine (0.4 g) were dissolved in acetonitrile (1 ml) and dropwise added thereto with stirring at room temperature. The mixture was stirred at room temperature for 1 hour and at 50°C for 4 hours. The reaction mixture was cooled to room temperature and cyclopropylamine (0.11 g) was added. The mixture was stirred at room temperature for 3 hours and the reaction mixture was concentrated. Water was added to the residue and the mixture was extracted with ethyl acetate. The extract was washed with water, dried and concentrated. Dry dimethylformamide (3 ml) and potassium carbonate (0.26 g) were added to the residue, and the mixture was stirred at 60°C for 8 hours. Water was added and the mixture was extracted with ethyl acetate. The extract was washed with water, dried and concentrated. The obtained oil was purified by silica gel column chromatography (developing solvent; chloroform:methanol=30:1) to give the object compound as a pale-yellow oil.

The obtained compound is treated in the same manner as in Example 19 (4) and Example 18 (2) to convert same to an 8-methoxy compound.

By the method of the above-mentioned Preparative Examples and Examples, the following compounds can be obtained.
○1-Cyclopropyl-7-(3-dimethylaminomethyl-3-fluoromethylpyrrolidin-1-yl)-6-fluoro-1,4-dihydro-8-methoxy-4-oxo-3-quinolinecarboxylic acid, melting point 178-180°C
○1-Cyclopropyl-6-fluoro-1,4-dihydro-7-(3-hydroxymethyl-3-methylaminomethylpiperidin-1-yl)-8-methoxy-4-oxo-3-quinolinecarboxylic acid, melting point 166-169°C

### Action and Effect of the Invention

The compounds (I) wherein n is 0, optical isomers thereof, salts thereof and hydrates thereof of the present invention show enforced and a wide range of *in vitro* and *in vivo* antibacterial effects against Gram-positive bacteria, while retaining a strong antibacterial effect against Gram-negative bacteria that the conventional quinolonecarboxylic acid antibacterial agents have. In addition, the compounds of the present invention show significant antibacterial effect on MRSA which is difficult to suppress with the currently-available drugs. In particular, they show superior antibacterial effects against quinolone resistant *Staphylococcus aureus*, for which there has never conventionally existed an effective drug, as well as against anaerobic bacteria, *Mycoplasma* and acid-fast bacteria. Therefore, the compounds of the present invention are expected to be new antibacterial agents showing superior clinical effects. The compounds of the present invention show less therapeutically unfavorable effects such as emergence of micronucleus and toxicity to the central nervous system, even in the form of racemates, as compared with the conventional antibacterial agents. When resolved into optically active compounds, moreover, they further reduced the above-mentioned therapeutically unfavorable effects, which cannot be known from the literatures showing only known plane structures. Of the optically active compounds, (S) compounds show remarkable effects as described.

The compound (I) of the present invention wherein n is 1 retains a strong antibacterial effect against Gram-negative bacteria, as mentioned above, and additionally shows enforced and a wide range of *in vitro* and *in vivo* antibacterial effects against Gram-positive bacteria. In addition, the problematic side-effects on the central nervous system were scarcely found and the compound was low toxic. Accordingly, clinically superior utility as an antibacterial agent is expected. In particular, the compound showed a markedly enforced antibacterial effect against Gram-negative bacteria such as *Staphylococcus aureus*, *Staphylococcus epidermidis*, *Streptococcus pneumoniae* and *Enterococcus*, while retaining an antibacterial effect against Gram-negative bacteria, which was equal to or superior to that of the conventional quinolonecarboxylic acid antibacterial agents. In addition, the compound showed a strong antibacterial effect against MRSA, quinolone resistant MRSA, quinolone resistant *Staphylococcus epidermidis* and quinolone resistant *Enterococcus*, as well as against anaerobic bacteria, *Chlamydia*, *Mycoplasma* and acid-fast bacteria. Since the strong *in vitro* antibacterial effect against such wide range of bacteria has been proven to be consistent with the therapeutic effect in mice, the compound is expected to show extremely superior clinical effect as a therapeutic agent for various infectious diseases caused by these pathogenic bacteria. In the absence of a suitable treatment drug for multiple drug resistant MRSA which poses very serious clinical problems as a causative bacterium of nosocomial infections, or the infectious diseases caused by, from among the causative bacteria of complicated urinary tract infection, *Staphylococcus epidermidis* or *Enterococcus* having resistance to both oral cephem preparations and quinolone preparations, the clinical utility of the compound of the present invention is considered extremely high. As discussed earlier, the compounds of the present invention have antibacterial effects against an extremely broad range of pathogenic bacteria including resistant bacteria. Therefore, the compounds of the present invention are expected to scarcely cause superinfection even when administered as a therapeutic agent for bacterial infections on an extended term basis to patients who acquired immune deficiency as a result of administrations of anticancer drugs and other medicaments.

## Claims

1. A 8-methoxy-quinolonecarboxylic acid derivative of the formula 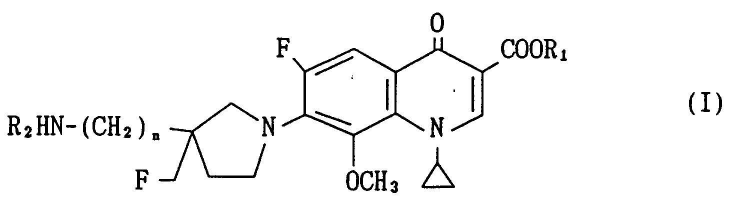 wherein R₁ is a hydrogen atom, a lower alkyl, a phenylalkyl or an ester residue hydrolyzable in the living body, R₂ is a hydrogen atom or methyl and n is an integer of 0 or 1,
an optical isomer thereof, a pharmaceutically acceptable salt thereof or a hydrate thereof.

2. The 8-methoxy-quinolonecarboxylic acid derivative of Claim 1, wherein R₁ is a hydrogen atom and n is 0,
an optical isomer thereof, a pharmaceutically acceptable salt thereof or a hydrate thereof.

3. The 8-methoxy-quinolonecarboxylic acid derivative of Claim 1, wherein R₁ is a hydrogen atom and n is 1,
an optical isomer thereof, a pharmaceutically acceptable salt thereof or a hydrate thereof.

4. The 8-methoxy-quinolonecarboxylic acid derivative of Claim 1, which is a member selected from the group consisting of 7-(3-amino-3-fluoromethylpyrrolidin-1-yl)-1-cyclopropyl-6-fluoro-1,4-dihydro-8-methoxy-4-oxo-3-quinolinecarboxylic acid, 7-(3-aminomethyl-3-fluoromethylpyrrolidin-1-yl)-1-cyclopropyl-6-fluoro- 1,4-dihydro-8-methoxy-4-oxo-3-quinolinecarboxylic acid, 1-cyclopropyl-6-fluoro-7-(3-fluoromethyl-3-methylaminopyrrolidin-1-yl)-1,4-dihydro-8-methoxy-4-oxo-3-quinolinecarboxylic acid and 1-cyclopropyl-6-fluoro-7-(3-fluoromethyl-3-methylaminomethylpyrrolidin-1-yl)-1,4-dihydro-8-methoxy-4-oxo-3-quinolinecarboxylic acid,
an optical isomer thereof, a pharmaceutically acceptable salt thereof or a hydrate thereof.

5. The 8-methoxy-quinolonecarboxylic acid derivative of any one of Claims 1, 2 and 4, which is a member selected from the group consisting of
7-(3-amino-3-fluoromethylpyrrolidin-1-yl)-1-cyclopropyl-6-fluoro-1,4-dihydro-8-methoxy-4-oxo-3-quinolinecarboxylic acid, (R)-7-(3-amino-3-fluoromethylpyrrolidin-1-yl)-1-cyclopropyl-6-fluoro-1,4-dihydro-8-methoxy-4-oxo-3-quinolinecarboxylic acid and (S)-7-(3-amino-3-fluoromethylpyrrolidin-1-yl)-1-cyclopropyl-6-fluoro-1,4-dihydro-8-methoxy-4-oxo-3-quinolinecarboxylic acid,
a pharmaceutically acceptable salt thereof or a hydrate thereof.

6. (S)-7-(3-Amino-3-fluoromethylpyrrolidin-1-yl)-1-cyclopropyl-6-fluoro-1,4-dihydro-8-methoxy-4-oxo-3-quinolinecarboxylic acid or a pharmaceutically acceptable salt thereof.

7. The 8-methoxy-quinolonecarboxylic acid derivative of any one of Claims 1, 3 and 4, which is a member selected from the group consisting of
7-(3-aminomethyl-3-fluoromethylpyrrolidin-1-yl)-1-cyclopropyl-6-fluoro-1,4-dihydro-8-methoxy-4-oxo-3-quinolinecarboxylic acid, (R)-7-(3-aminomethyl-3-fluoromethylpyrrolidin-1-yl)-1-cyclopropyl-6-fluoro-1,4-dihydro-8-methoxy-4-oxo-3-quinolinecarboxylic acid and (S)-7-(3-aminomethyl-3-fluoromethylpyrrolidin-1-yl)-1-cyclopropyl-6-fluoro-1,4-dihydro-8-methoxy-4-oxo-3-quinolinecarboxylic acid, an optical isomer thereof, a pharmaceutically acceptable salt thereof or a hydrate thereof.

8. (S)-7-(3-Aminomethyl-3-fluoromethylpyrrolidin-1-yl)-1-cyclopropyl-6-fluoro-1,4-dihydro-8-methoxy-4-oxo-3-quinolinecarboxylic acid or a pharmaceutically acceptable salt thereof.

9. A pharmaceutical composition comprising the 8-methoxy-quinolonecarboxylic acid derivative of any one of Claims 1 to 8, an optical isomer thereof, a pharmaceutically acceptable salt thereof or a hydrate thereof, and a pharmaceutically acceptable carrier.
